(19) Europäisches Patentamt
European Patent Office
Office européen des brevets

(11) EP 4 653 435 A1

(12) EUROPEAN PATENT APPLICATION
published in accordance with Art. 153(4) EPC

(43) Date of publication:
**26.11.2025 Bulletin 2025/48**

(21) Application number: **24744149.6**

(22) Date of filing: **11.01.2024**

(51) International Patent Classification (IPC):
***C07D 471/04*** (2006.01) ***C07D 491/044*** (2006.01)
***C07D 401/12*** (2006.01) ***C07D 239/95*** (2006.01)
***C07D 213/75*** (2006.01) ***A61P 35/00*** (2006.01)
***A61K 31/437*** (2006.01) ***A61K 31/4427*** (2006.01)

(52) Cooperative Patent Classification (CPC):
**A61K 31/437; A61K 31/4427; A61K 31/444; A61K 31/4545; A61K 31/496; A61K 31/506; A61K 31/5377; A61P 35/00; A61P 35/02; C07D 213/75; C07D 239/95; C07D 401/12; C07D 471/04; C07D 491/044; C07D 519/00**

(86) International application number:
**PCT/CN2024/071738**

(87) International publication number:
**WO 2024/152973 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.01.2023 CN 202310060341**
**15.03.2023 CN 202310249725**
**14.04.2023 CN 202310397839**
**01.06.2023 CN 202310642654**
**22.08.2023 CN 202311058640**
**08.12.2023 CN 202311684268**

(71) Applicant: **Suzhou Puhe Biopharma Co., Ltd.**
**Suzhou, Jiangsu 215124 (CN)**

(72) Inventors:
- **LIU, Bin**
  **Suzhou, Jiangsu 215124 (CN)**
- **GAO, Feng**
  **Suzhou, Jiangsu 215124 (CN)**
- **GUO, Yongqi**
  **Suzhou, Jiangsu 215124 (CN)**
- **WU, Yongyong**
  **Suzhou, Jiangsu 215124 (CN)**
- **JING, Liandong**
  **Suzhou, Jiangsu 215124 (CN)**
- **LI, Zhizhong**
  **Suzhou, Jiangsu 215124 (CN)**
- **WU, Zhuo**
  **Suzhou, Jiangsu 215124 (CN)**

(74) Representative: **BIP Patents**
**c/o Bayer Intellectual Property GmbH**
**Alfred-Nobel-Straße 50**
**40789 Monheim am Rhein (DE)**

# (54) PRMT5-MTA INHIBITOR

(57) Provided in the present invention is a compound as a PRMT5-MTA inhibitor, which is a compound as represented by formula (I) or a pharmaceutically acceptable salt, an isotopic variant, a tautomer or a stereoisomer thereof. Further provided in the present invention are a pharmaceutical composition containing the compound and the use thereof in the treatment of cancers.

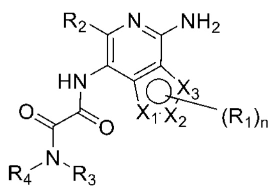

(I)

EP 4 653 435 A1

## Description

## TECHNICAL FIELD

The present invention belongs to the field of medicine, and in particular relates to PRMT5-MTA inhibitors.

## BACKGROUND ART

Protein arginine methyltransferases (PRMTs) can methylate histones and non-histones, thereby participating in the regulation of biological processes such as gene transcription, signal transduction, protein stability, cell proliferation, differentiation, apoptosis, and tumor formation (Nat Rev Mol Cell Biol. 2019 Oct; 20(10): 642-657) (Nat Rev Drug Discov. 2021 Jul; 20(7): 509-530). Eleven members of the PRMT family have been discovered thus far, and may be classified into types I, II, and III based on differences in how they catalyze arginine methylation, wherein PRMT5 is a type II member that catalyzes by way of symmetrical dimethylation.

An epigenetic enzyme, PRMT5 is involved in a variety of biological processes, including transcriptional regulation, RNA metabolism, ribosome biogenesis, and cell cycle regulation. PRMT5 protein is overexpressed in a variety of cancer types, including B and T cell lymphoma, metastatic melanoma, neuroblastoma, glioblastoma, ovarian cancer, and breast cancer, and increasing evidence suggests that it plays an important role in tumor occurrence and development (Cell Stress 2020 Aug; 4(8): 199-215) (Cancer Gene Ther. 2022 Mar; 29(3-4): 264-276). On this basis, PRMT5 inhibitors have become a key focus in the research and development of tumor therapy drugs.

Early PRMT5 inhibitors could be divided into two classes: substrate competitive inhibitors, represented by GSK3326595; and SAM competitive inhibitors, represented by JNJ64619178. Both types of drugs are potent inhibitors of PRMT5 and demonstrate powerful anti-tumor activity. However, strong blood toxicity has been observed due to their potent inhibitory activity against PRMT5 in both normal cells and tumor cells, limiting their clinical applications and thus impacting on the outcomes of clinical therapy (Bioorg Med Chem Lett. 2019 Jun 1; 29(11): 1264-1269) (Expert Opin Ther Pat. 2019 Feb; 29(2): 97-114) (Annals of Oncology (2020) 31 (suppl_4): S462-S504. 10.1016/annonc/annonc271) (Annals of Oncology (2019) 30 (suppl_5): v159-v193. 10.1093/annonc/mdz244).

A paper published in *Science* in 2016 revealed that *MTAP* deletion has synthetic lethality with PRMT5 (Science 2016 Mar 11; 351(6278): 1214-8). *MTAP* deletion is prevalent in a variety of solid tumors, including pancreatic cancer and glioma. Because MTAP is an enzyme that degrades intracellular MTA, *MTAP* deletion can lead to intracellular accumulation of MTA, and MTA competes for binding to PRMT5 with the methyl donor SAM of its functional substrate, thereby inhibiting the function of PRMT5. Since MTA accumulates specifically in *MTAP*-deficient tumor cells, by strengthening the binding inhibition of MTA and PRMT5 it is possible to specifically inhibit PRMT5 activity in tumor cells, with only weak inhibitory effects on PRMT5 activity in normal cells, thereby providing a therapeutic safety window that lowers toxicity without compromising anti-tumor efficacy (Nat Rev Drug Discov. 2020 Jan; 19(1): 23-38) (Cell Rep. 2016 Apr 19; 15(3): 574-587). Preclinical validation data have now been obtained for MTA-synergistic PRMT5 inhibitors (J Med Chem. 2022 Feb 10; 65(3): 1749-1766), and their development holds great promise for the treatment of *MTAP*-deficient tumors.

Despite the progress that has been made in PRMT5 research, to date there remains a lack of effective and selective PRMT5-MTA inhibitors.

## SUMMARY OF THE INVENTION

A compound of formula (I), or a pharmaceutically acceptable salt, isotopic variant, tautomer or stereoisomer thereof:

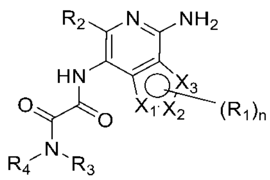

(I)

wherein,

- $X_1$, $X_2$ and $X_3$ are each independently selected from -CH=, -O-, -S-, -N= or -NH-, and at least one of $X_1$, $X_2$ and $X_3$ is -N= or -NH-, the circle indicating aromaticity;
- $R_1$ is selected from $C_{1-6}$ alkyl, deuterated $C_{1-6}$ alkyl, $C_{3-6}$ cycloalkyl or $C_{1-6}$ haloalkyl;
- $R_2$ is selected from H, $C_{1-6}$ alkyl or halogen;

$R_3$ and $R_4$ are independently selected from H, $C_{1\text{-}6}$ alkyl, $C_{3\text{-}6}$ cycloalkyl or 4-10 membered heterocyclyl, wherein the $C_{1\text{-}6}$ alkyl, $C_{3\text{-}6}$ cycloalkyl or 4-10 membered heterocyclyl in $R_3$ or $R_4$ is each optionally substituted by 1 or 2 $R_5$, wherein $R_5$ is selected from hydrogen, deuterium, halogen, $C_{1\text{-}6}$ alkyl, $C_{1\text{-}6}$ alkoxy, $C_{1\text{-}6}$ haloalkyl, CN, $SF_5$, $C_{3\text{-}6}$ cycloalkyl, $-S(O)_2CF_3$, $-OCF_3$, $-SCF_3$, $-SCH_3$ or 5-10 membered heteroaryl; the 5-10 membered heteroaryl is optionally further substituted by $R_6$, wherein $R_6$ is selected from H, CN, halogen, $C_{1\text{-}6}$ alkyl, $C_{1\text{-}6}$ haloalkyl, $SF_5$, $OR_a$ or 5-6 membered heteroaryl, the 5-6 membered heteroaryl being optionally substituted by 1, 2 or 3 $R_z$;

alternatively, $R_3$, $R_4$ and the nitrogen atom to which they are attached together form a 4-10 membered heterocyclic group, the 4-10 membered heterocyclic group comprising 0 or 1 additional heteroatom selected from oxygen, nitrogen or sulfur, and the 4-10 membered heterocyclic group is optionally substituted with 1, 2, 3 or 4 $R_x$;

$R_x$ is selected from $C_{1\text{-}6}$ alkyl, $C_{1\text{-}6}$ haloalkyl, phenyl or 5-10 membered heteroaryl, wherein the phenyl or 5-10 membered heteroaryl is optionally substituted with 1, 2 or 3 $R_y$;

$R_y$ is selected from halogen, $C_{1\text{-}6}$ alkyl, $C_{1\text{-}6}$ haloalkyl, $SF_5$, CN, $OR_a$, $C_{1\text{-}6}$ alkoxy, $-NR_{z1}R_{z2}$, $NR_{z1}R_{z2}$-$C_{1\text{-}6}$ alkyl-, $C_{3\text{-}6}$ cycloalkyl, 4-12 membered heterocyclyl or 5-10 membered heteroaryl, wherein the 4-12 membered heterocyclyl or 5-10 membered heteroaryl is optionally substituted by 1, 2 or 3 $R_z$;

$R_z$ is selected from $C_{1\text{-}6}$ alkyl, halogen, CN, $C_{1\text{-}6}$ haloalkyl or $C_{1\text{-}6}$ alkoxy;

$R_a$ is selected from H, $C_{1\text{-}6}$ alkyl or $C_{1\text{-}6}$ haloalkyl;

$R_{z1}$ and $R_{z2}$ are each independently selected from H or $C_{1\text{-}6}$ alkyl; and

*n* is 0, 1 or 2.

In another aspect, the present invention provides a compound of formula (IIa) or formula (IIb), or a pharmaceutically acceptable salt, isotopic variant, tautomer or stereoisomer thereof:

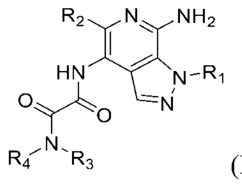
(IIa) or
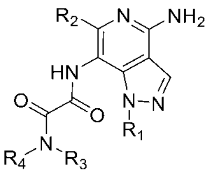
(IIb)

wherein,

$R_1$ is selected from H, $C_{1\text{-}6}$ alkyl, deuterated $C_{1\text{-}6}$ alkyl, $C_{3\text{-}6}$ cycloalkyl or $C_{1\text{-}6}$ haloalkyl;

$R_2$ is selected from H, $C_{1\text{-}6}$ alkyl or halogen;

$R_3$ and $R_4$ are independently selected from H, $C_{1\text{-}6}$ alkyl, $C_{3\text{-}6}$ cycloalkyl or 4-10 membered heterocyclyl, wherein the $C_{1\text{-}6}$ alkyl, $C_{3\text{-}6}$ cycloalkyl or 4-10 membered heterocyclyl in $R_3$ or $R_4$ is each optionally substituted by 1 or 2 $R_5$, wherein $R_5$ is selected from hydrogen, deuterium, halogen, $C_{1\text{-}6}$ alkyl, $C_{1\text{-}6}$ alkoxy, $C_{1\text{-}6}$ haloalkyl, CN, $SF_5$, $C_{3\text{-}6}$ cycloalkyl, $-S(O)_2CF_3$, $-OCF_3$, $-SCF_3$, $-SCH_3$ or 5-10 membered heteroaryl; the 5-10 membered heteroaryl is optionally further substituted by $R_6$, wherein $R_6$ is selected from H, CN, halogen, $C_{1\text{-}6}$ alkyl, $C_{1\text{-}6}$ haloalkyl, $SF_5$, $OR_a$ or 5-6 membered heteroaryl, the 5-6 membered heteroaryl being optionally substituted by 1, 2 or 3 $R_z$;

alternatively, $R_3$, $R_4$ and the nitrogen atom to which they are attached together form a 4-10 membered heterocyclic group, the 4-10 membered heterocyclic group comprising 0 or 1 additional heteroatom selected from oxygen, nitrogen or sulfur, and being optionally substituted with 1, 2, 3 or 4 $R_x$;

$R_x$ is selected from $C_{1\text{-}6}$ alkyl, $C_{1\text{-}6}$ haloalkyl, phenyl or 5-10 membered heteroaryl, wherein the phenyl or 5-10 membered heteroaryl is optionally substituted with 1, 2 or 3 $R_y$;

$R_y$ is selected from halogen, $C_{1\text{-}6}$ alkyl, $C_{1\text{-}6}$ haloalkyl, $SF_5$, CN, $OR_a$, $C_{1\text{-}6}$ alkoxy, $-NR_{z1}R_{z2}$, $NR_{z1}R_{z2}$-$C_{1\text{-}6}$ alkyl, $C_{3\text{-}6}$ cycloalkyl, 4-12 membered heterocyclyl or 5-10 membered heteroaryl, wherein the 4-12 membered heterocyclyl or 5-10 membered heteroaryl is optionally substituted by 1, 2 or 3 $R_z$;

$R_z$ is selected from $C_{1\text{-}6}$ alkyl, halogen, CN, $C_{1\text{-}6}$ haloalkyl or $C_{1\text{-}6}$ alkoxy;

$R_a$ is selected from H, $C_{1\text{-}6}$ alkyl or $C_{1\text{-}6}$ haloalkyl; and

$R_{z1}$ and $R_{z2}$ are each independently selected from H or $C_{1\text{-}6}$ alkyl.

In another aspect, the present invention provides specific compounds as exemplified in the present application.

In another aspect, the present invention provides a pharmaceutical composition comprising a compound of the present invention and a pharmaceutically acceptable excipient, which may also contain other therapeutic agents.

In another aspect, the present invention provides use of the compound of the present invention in the preparation of a medicament for treating and/or preventing a disease mediated by PRMT5 methyltransferase.

In another aspect, the present invention provides a method for treating and/or preventing a PRMT5 methyltransferase-mediated disease in a subject, comprising administering a compound or composition of the present invention

to the subject.

In another aspect, the present invention provides a compound of the present invention or a composition of the present invention for use in treating and/or preventing a disease mediated by PRMT5 methyltransferase.

In a specific embodiment, the diseases treated by the present invention include cancers selected from the group consisting of acoustic neuroma, adenocarcinoma, adrenal cancer, anal cancer, angiosarcoma (e.g., lymphangiosarcoma, lymphangioendothelioma, hemangioma), appendix cancer, benign monoclonal gammopathy, bile duct cancer, bladder cancer, brain cancer (e.g., meningioma, glioma, e.g., astrocytoma, oligodendroglioma, medulloblastoma), bronchial cancer, carcinoid tumor, cervical cancer (e.g., cervical adenocarcinoma), choriocarcinoma, chordoma, craniopharyngioma, colorectal cancer (e.g., colon cancer, rectal cancer, colorectal adenocarcinoma), epithelial cancer, ependymoma, endothelial sarcoma (e.g., Kaposi's sarcoma, multiple idiopathic hemorrhagic sarcoma), endometrial cancer (e.g., uterine cancer, uterine sarcoma), esophageal cancer (e.g., esophageal adenocarcinoma, Barrett's adenocarcinoma), Ewing's sarcoma, eye cancer (e.g., intraocular melanoma, retinoblastoma), hypereosinophilia, gallbladder cancer, stomach cancer (e.g., gastric adenocarcinoma), gastrointestinal stromal tumor (GIST), head and neck cancer (e.g., head and neck squamous cell carcinoma, oral cancer (e.g., oral squamous cell carcinoma, laryngeal cancer (e.g., laryngeal cancer, pharyngeal cancer, nasopharyngeal cancer, oropharyngeal cancer))), hematopoietic system cancer (e.g., leukemia, such as acute lymphoblastic leukemia (ALL) (e.g., B-cell ALL, T-cell ALL), acute myeloid leukemia (AML) (e.g., B-cell AML, T-cell AML), chronic myeloid leukemia (CML) (e.g., B-cell CML, T-cell CML), chronic lymphocytic leukemia (CLL) (e.g., B-cell CLL, T-cell CLL), follicular lymphoma, chronic lymphocytic leukemia/small lymphocytic lymphoma (CLL/SLL), marginal zone B-cell lymphoma (e.g., mucosa-associated lymphoid tissue (MALT) lymphoma, lymph node marginal zone B-cell lymphoma, splenic marginal zone B-cell lymphoma), primary mediastinal B-cell lymphoma, Burkitt's lymphoma, lymphoplasmacytic lymphoma, hairy cell leukemia (HCL), immunoblastic large cell lymphoma, precursor B lymphoblastic lymphoma, and primary central nervous system (CNS) lymphoma; and T-cell non-Hodgkin's lymphoma, e.g., precursor T-lymphoblastic lymphoma/leukemia, peripheral T-cell lymphoma (e.g., cutaneous T-cell lymphoma (e.g., mycosis fungoides, Sezary syndrome), angioimmunoblastic T-cell lymphoma, extranodal natural killer T-cell lymphoma, enteropathy-associated T-cell lymphoma, subcutaneous panniculitis-like T-cell lymphoma; anaplastic large cell lymphoma); a mixture of one or more of the above leukemias/lymphomas; multiple myeloma (MM)), heavy chain disease (e.g., $\alpha$-chain disease, $\gamma$-chain disease, $\mu$-chain disease), hemangioblastoma, inflammatory myofibroblastic tumor, immune cell amyloidosis, renal cancer (e.g., Wilms tumor, renal cell carcinoma), liver cancer (e.g., hepatocellular carcinoma, malignant liver cell carcinoma), lung cancer (e.g., bronchogenic carcinoma, small cell lung cancer (SCLC), non-small cell lung cancer (NSCLC), lung adenocarcinoma, leiomyosarcoma (LMS), mastocytosis (e.g., systemic mastocytosis), myelodysplastic syndrome (MDS), mesothelioma, myeloproliferative disorder (MPD) (e.g., polycythemia vera (PV), essential thrombocythemia (ET), idiopathic extramedullary metaplasia of the bone marrow (AMM), chronic idiopathic myelofibrosis, chronic myeloid leukemia (CML), chronic neutrophilic leukemia (CNL), hypereosinophilic syndrome (HES), neuroblastoma, neurofibromas (e.g., neurofibromatosis type 1 or type 2, schwannomatosis), neuroendocrine cancer (e.g., gastroenteropancreatic neuroendocrine tumor (GEP-NET), carcinoid tumor), osteosarcoma, ovarian cancer (e.g., cystadenocarcinoma, ovarian embryonal carcinoma, ovarian adenocarcinoma), papillary adenocarcinoma, or penile cancer.

Other objects and advantages of the present invention will be apparent to those skilled in the art from the following detailed description, examples and claims.

*Definitions*

*Chemical Definitions*

Definitions of specific functional groups and chemical terms are described in more detail below.

When a numerical range is listed, each value and subrange within the stated range is intended to be included. For example, "$C_{1-6}$ alkyl" includes $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$, and $C_{5-6}$ alkyls.

"$C_{1-6}$ alkyl" refers to a straight or branched saturated hydrocarbon group having 1 to 6 carbon atoms. In some embodiments, $C_{1-4}$ alkyl and $C_{1-2}$ alkyl are preferred. Examples of $C_{1-6}$ alkyls include: methyl ($C_1$), ethyl ($C_2$), n-propyl ($C_3$), isopropyl ($C_3$), n-butyl ($C_4$), tert-butyl ($C_4$), sec-butyl ($C_4$), isobutyl ($C_4$), n-pentyl ($C_5$), 3-pentyl ($C_5$), pentyl ($C_5$), neopentyl ($C_5$), 3-methyl-2-butyl ($C_5$), tert-pentyl ($C_5$) and n-hexyl ($C_6$). The term "$C_{1-6}$ alkyl" further includes heteroalkyls, in which one or more (e.g., 1, 2, 3 or 4) carbon atoms are replaced by heteroatoms (e.g., oxygen, sulfur, nitrogen, boron, silicon, phosphorus). The alkyl group may be optionally substituted by one or more substituents, for example, substituted by 1 to 5 substituents, 1 to 3 substituents or 1 substituent. Common alkyl abbreviations include: Me ($-CH_3$), Et ($-CH_2CH_3$), iPr ($-CH(CH_3)_2$), nPr ($-CH_2CH_2CH_3$), n-Bu ($-CH_2CH_2CH_2CH_3$), or i-Bu ($-CH_2CH(CH_3)_2$).

"$C_{2-6}$ alkenyl" refers to a straight or branched hydrocarbon group having 2 to 6 carbon atoms and at least one carbon-carbon double bond. In some embodiments, $C_{2-4}$ alkenyl is preferred. Examples of $C_{2-6}$ alkenyl include: vinyl ($C_2$), 1-propenyl ($C_3$), 2-propenyl ($C_3$), 1-butenyl ($C_4$), 2-butenyl ($C_4$), butadienyl ($C_4$), pentenyl ($C_5$), pentadienyl ($C_5$), hexenyl

($C_6$), and the like. The term "$C_2$-$_6$ alkenyl" further includes heteroalkenyls, in which one or more (e.g., 1, 2, 3 or 4) carbon atoms are replaced by heteroatoms (e.g., oxygen, sulfur, nitrogen, boron, silicon, phosphorus). The alkenyl group may be optionally substituted by one or more substituents, for example, substituted by 1 to 5 substituents, 1 to 3 substituents or 1 substituent.

"$C_2$-$_6$ alkynyl" refers to a straight or branched hydrocarbon group having 2 to 6 carbon atoms, at least one carbon-carbon triple bond and optionally one or more carbon-carbon double bonds. In some embodiments, $C_2$-$_4$ alkynyl is preferred. Examples of $C_2$-$_6$ alkynyl include, but are not limited to, ethynyl ($C_2$), 1-propynyl ($C_3$), 2-propynyl ($C_3$), 1-butynyl ($C_4$), 2-butynyl ($C_4$), pentynyl ($C_5$), hexynyl ($C_6$), and the like. The term "$C_2$-$_6$ alkynyl" also includes heteroalkynyls, in which one or more (e.g., 1, 2, 3 or 4) carbon atoms are replaced by heteroatoms (e.g., oxygen, sulfur, nitrogen, boron, silicon, phosphorus). Alkynyl groups may be optionally substituted by one or more substituents, for example, by 1 to 5 substituents, 1 to 3 substituents or 1 substituent.

"$C_1$-$_6$ alkylene" refers to a divalent group formed by removing another hydrogen of a $C_1$-$_6$ alkyl, and may be substituted or unsubstituted. In some embodiments, $C_1$-$_4$ alkylene, $C_2$-$_4$ alkylene and $C_1$-$_3$ alkylene are preferred. Unsubstituted alkylene includes, but is not limited to, methylene ($-CH_2-$), ethylene ($-CH_2CH_2-$), propylene ($-CH_2CH_2CH_2-$), butylene ($-CH_2CH_2CH_2CH_2-$), pentylene ($-CH_2CH_2CH_2CH_2CH_2-$), hexylene ($-CH_2CH_2CH_2CH_2CH_2CH_2-$), and the like. Exemplary substituted alkylene groups, for example, alkylene groups substituted with one or more alkyl(methyl) groups, include, but are not limited to, substituted methylene groups ($-CH(CH_3)-$, $-C(CH_3)_2-$), substituted ethylene groups ($-CH(CH_3)CH_2-$, - $CH_2CH(CH_3)-$, $-C(CH_3)_2CH_2-$, $-CH_2C(CH_3)_2-$), substituted propylene groups ($-CH(CH_3)CH_2CH_2-$, - $CH_2CH(CH_3)CH_2-$, $-CH_2CH_2CH(CH_3)-$, $-C(CH_3)_2CH_2CH_2-$, $-CH_2C(CH_3)_2CH_2-$, $-CH_2CH_2C(CH_3)_2-$), and the like.

"$C_2$-$_6$ alkenylene" refers to a divalent group formed by removing another hydrogen of a $C_2$-$_6$ alkenyl group, and may be substituted or unsubstituted. In some embodiments, $C_2$-$_4$ alkenylene is particularly preferred. Exemplary unsubstituted alkenylene groups include, but are not limited to, vinylene ($-CH=CH-$) and propenylene (e.g., $-CH=CHCH_2-$, $-CH_2-CH=CH-$). Exemplary substituted alkenylene groups, for example, alkenylene groups substituted with one or more alkyl(methyl) groups, include, but are not limited to, substituted ethylene groups ($-C(CH_3)=CH-$, $-CH=C(CH_3)-$), substituted propenylene groups ($-C(CH_3)=CHCH_2-$, $-CH=C(CH_3)CH_2-$, $-CH=CHCH(CH_3)-$, $-CH=CHC(CH_3)_2-$, $-CH(CH_3)-CH=CH-$, - $C(CH_3)_2-CH=CH-$, $-CH_2-C(CH_3)=CH-$, $-CH_2-CH=C(CH_3)-$), and the like.

"$C_2$-$_6$ alkynylene" refers to a divalent group formed by removing another hydrogen of a $C_2$-$_6$ alkynyl group, and may be substituted or unsubstituted. In some embodiments, $C_2$-$_4$ alkynylene is particularly preferred. Exemplary alkynylene groups include, but are not limited to, ethynylene (-C=C-), substituted or unsubstituted propynylene ($-C\equiv CCH_2-$), and the like.

"Halo" or "halogen" refers to fluorine (F), chlorine (Cl), bromine (Br) and iodine (I).

Therefore, "$C_1$-$_6$ haloalkyl" refers to an aforedescribed "$C_1$-$_6$ alkyl" that is substituted by one or more halogen groups. In some embodiments, $C_1$-$_4$ haloalkyl is particularly preferred, more preferably $C_1$-$_2$ haloalkyl. Exemplary haloalkyls include, but are not limited to: $-CF_3$, $-CH_2F$, $-CHF_2$, $-CHFCH_2F$, - $CH_2CHF_2$, $-CF_2CF_3$, $-CCl_3$, $-CH_2Cl$, $-CHCl_2$, 2,2,2-trifluoro-1,1-dimethyl-ethyl, and the like. The haloalkyl group can be substituted at any available point of attachment, for example, 1 to 5 substituents, 1 to 3 substituents, or 1 substituent.

"$C_1$-$_6$ alkoxy" refers to an -OR group in which R is a $C_1$-$_6$ alkyl group as defined above. $C_1$-$_4$ alkoxy is preferred.

"$C_1$-$_6$ haloalkoxy" refers to a "$C_1$-$_6$ alkoxy" group which is substituted with one or more halogen groups. In some embodiments, $C_1$-$_4$ haloalkoxyalkyl is particularly preferred, and $C_1$-$_2$ haloalkoxyalkyl is more preferred.

"$C_{3-10}$ cycloalkyl" refers to a non-aromatic cyclic hydrocarbon group having 3 to 10 ring carbon atoms and zero heteroatoms. In some embodiments, $C_{4-10}$ cycloalkyl, $C_5$-$_{10}$ cycloalkyl, $C_4$-$_7$ cycloalkyl, $C_3$-$_7$ cycloalkyl, $C_3$-$_6$ cycloalkyl, $C_3$-$_5$ cycloalkyl and $C_3$-$_4$ cycloalkyl are particularly preferred, more preferably $C_5$-$_6$ cycloalkyl. Cycloalkyl also includes a ring system in which the above cycloalkyl ring is fused with one or more aryl or heteroaryl groups, wherein the point of attachment is on the cycloalkyl ring, and in such a case, the number of carbons continues to represent the number of carbons in the cycloalkyl system. Exemplary cycloalkyl groups include, but are not limited to, cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), etc. The cycloalkyl group may be optionally substituted with one or more substituents, for example, 1 to 5 substituents, 1 to 3 substituents, or 1 substituent.

"3-12 membered heterocyclyl" refers to a group of a 3- to 12-membered non-aromatic ring system having ring carbon atoms and 1 to 5 ring heteroatoms, wherein each heteroatom is independently selected from nitrogen, oxygen, sulfur, boron, phosphorus and silicon. In heterocyclyls containing one or more nitrogen atoms, the point of attachment may be a carbon or nitrogen atom as long as the valence permits. In some embodiments, a 3-10 membered heterocyclyl is preferably a 3-10 membered non-aromatic ring system having ring carbon atoms and 1 to 3 ring heteroatoms; in some embodiments, a 4-10 membered heterocyclyl is preferably a 4-10 membered non-aromatic ring system having ring carbon atoms and 1 to 4 ring heteroatoms; in some embodiments, a 5-10 membered heterocyclyl is preferably a 5-10 membered non-aromatic ring system having ring carbon atoms and 1 to 5 ring heteroatoms; in some embodiments, a 5-8 membered heterocyclyl is preferably a 5-8 membered non-aromatic ring system having ring carbon atoms and 1 to 5 ring heteroatoms;

in some embodiments, a 3-7 membered heterocyclyl is preferably a 3-to 7-membered non-aromatic ring system having ring carbon atoms and 1 to 4 ring heteroatoms; preferably a 3- to 6-membered heterocyclyl, which is a 3- to 6-membered non-aromatic ring system having ring carbon atoms and 1 to 3 ring heteroatoms; preferably a 4- to 7-membered heterocyclyl, which is a 4- to 7-membered non-aromatic ring system having ring carbon atoms and 1 to 3 ring heteroatoms; preferably a 4- to 6-membered heterocyclyl, which is a 4- to 6-membered non-aromatic ring system having ring carbon atoms and 1 to 3 ring heteroatoms; more preferably a 5- to 6-membered heterocyclyl, which is a 5- to 6-membered non-aromatic ring system having ring carbon atoms and 1 to 3 ring heteroatoms; more preferably a 3- to 5-membered heterocyclyl, which is a 3- to 5-membered non-aromatic ring system having ring carbon atoms and 1 to 3 ring heteroatoms. Heterocyclyl also includes a ring system in which the above-mentioned heterocyclyl ring is fused to one or more cycloalkyl groups, wherein the point of attachment is on the cycloalkyl ring, or a ring system in which the above-mentioned heterocyclyl ring is fused to one or more aryl or heteroaryl groups, wherein the point of attachment is on the heterocyclyl ring; and in such a case, the number of ring members continues to represent the number of ring members in the heterocyclyl ring system. Exemplary 3-membered heterocyclyls containing one heteroatom include, but are not limited to, aziridinyl, oxiranyl, and thiiranyl (thiorenyl). Exemplary 4-membered heterocyclyls containing one heteroatom include, but are not limited to, azetidinyl, oxetanyl and thietanyl. Exemplary 5-membered heterocyclyls containing one heteroatom include, but are not limited to, tetrahydrofuranyl, dihydrofuranyl, tetrahydrothienyl, dihydrothienyl, pyrrolidinyl, pyrrolinyl, and pyrrolyl-2,5-dione. Exemplary 5-membered heterocyclic groups containing two heteroatoms include, but are not limited to, dioxolanyl, oxasulfuranyl, disulfuranyl, and oxazolidin-2-one. Exemplary 5-membered heterocyclic groups containing three heteroatoms include, but are not limited to, triazolinyl, oxadiazolinyl, and thiadiazolinyl. Exemplary 6-membered heterocyclic groups containing one heteroatom include, but are not limited to, piperidinyl, tetrahydropyranyl, dihydropyridinyl, and thianyl. Exemplary 6-membered heterocyclic groups containing two heteroatoms include, but are not limited to, piperazinyl, morpholinyl, dithianyl, and dioxanyl. Exemplary 6-membered heterocyclic groups containing three heteroatoms include, but are not limited to, triazinanyl. Exemplary 7-membered heterocyclic groups containing one heteroatom include, but are not limited to, azepanyl, oxepanyl, and thiepanyl. Exemplary 5-membered heterocyclyl groups fused to a $C_6$ aryl ring (also referred to herein as 5,6-bicyclic heterocyclyl groups) include, but are not limited to, dihydroindolinyl, isoindolinyl, dihydrobenzofuranyl, dihydrobenzothienyl, benzoxazolinonyl, and the like. Exemplary 6-membered heterocyclyl groups fused to a $C_6$ aryl ring (also referred to herein as 6,6-bicyclic heterocyclyl groups) include, but are not limited to, tetrahydroquinolinyl, tetrahydroisoquinolinyl, tetrahydrobenzopyranyl, tetrahydropyranopyridinyl, and the like. The heterocyclyl group may be optionally substituted with one or more substituents, for example, substituted with 1 to 5 substituents, 1 to 3 substituents, or 1 substituent.

"$C_{6}$-$_{1O}$ aryl" refers to a monocyclic or polycyclic (e.g., bicyclic) 4*n*+2 aromatic ring system (e.g., having 6 or 10 $\pi$ electrons shared in a cyclic arrangement) having 6-10 ring carbon atoms and zero heteroatoms. In some embodiments, the aryl has six ring carbon atoms ("$C_6$ aryl"; e.g., phenyl). In some embodiments, the aryl has ten ring carbon atoms ("$C_{10}$ aryl"; e.g., naphthyl, e.g., 1-naphthyl and 2-naphthyl). Aryl also includes ring systems in which the above aryl ring is fused to one or more cycloalkyl or heterocyclic groups, and the point of attachment is on the aryl ring, in which case the number of carbon atoms continues to represent the number of carbon atoms in the aryl ring system. The aryl group may be optionally substituted with one or more substituents, e.g., 1 to 5 substituents, 1 to 3 substituents, or 1 substituent.

"5-14 membered heteroaryl" refers to a group of a 5-14 membered monocyclic or bicyclic 4n+2 aromatic ring system (e.g., having 6, 10, or 14 $\pi$ electrons shared in a cyclic arrangement) having ring carbon atoms and 1-4 ring heteroatoms, wherein each heteroatom is independently selected from nitrogen, oxygen, and sulfur. In heteroaryl groups containing one or more nitrogen atoms, the point of attachment may be a carbon or nitrogen atom as long as the valence permits. Heteroaryl bicyclic ring systems may include one or more heteroatoms in one or both rings. Heteroaryl also includes a ring system in which the above-mentioned heteroaryl ring is fused to one or more cycloalkyl or heterocyclic groups, and the point of attachment is on the heteroaryl ring, in which case the number of carbon atoms continues to represent the number of carbon atoms in the heteroaryl ring system. In some embodiments, a 5-10 membered heteroaryl is preferred, which is a 5-10 membered monocyclic or bicyclic 4n+2 aromatic ring system having ring carbon atoms and 1-4 ring heteroatoms. In some embodiments, 5-10 membered heteroaryls are preferred, which are 6-10 membered monocyclic or bicyclic 4n+2 aromatic ring systems with ring carbon atoms and 1-4 ring heteroatoms. In some embodiments, 5-9 membered heteroaryls are preferred, which are 5-9 membered monocyclic or bicyclic 4*n*+2 aromatic ring systems with ring carbon atoms and 1-4 ring heteroatoms. In other embodiments, 5-6 membered heteroaryls are particularly preferred, which are 5-6 membered monocyclic or bicyclic 4*n*+2 aromatic ring systems with ring carbon atoms and 1-4 ring heteroatoms. Exemplary 5-membered heteroaryls containing one heteroatom include, but are not limited to, pyrrolyl, furanyl, and thienyl. Exemplary 5-membered heteroaryls containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (e.g., 1,2,4-oxadiazolyl) and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridinyl. Exemplary 6-membered heteroaryl groups containing two heteroatoms include, but are not limited to, pyridazinyl, pyrimidinyl and

pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azacycloheptatrienyl, oxacycloheptatrienyl and thiacycloheptatrienyl. Exemplary 5,6-bicyclic heteroaryl groups include, but are not limited to, indolyl, isoindolyl, indazolyl, benzotriazolyl, benzothienyl, isobenzothienyl, benzofuranyl, benzisofuranyl, benzimidazolyl, benzoxazolyl, benzisoxazolyl, benzoxadiazolyl, benzothiazolyl, benzisothiazolyl, benzothiadiazolyl, indazinyl, and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl, and quinazolinyl. The heteroaryl group may be optionally substituted with one or more substituents, for example, with 1 to 5 substituents, 1 to 3 substituents, or 1 substituent.

"Cycloalkylene", "heterocyclylene", "arylene" or "heteroarylene" is a divalent group formed by removing another hydrogen from "cycloalkyl", "heterocyclyl", "aryl" or "heteroaryl" as defined above, and may be substituted or unsubstituted. For example, "$C_{5}$-$_{7}$ cycloalkylene" refers to a divalent group formed by removing another hydrogen from $C_{5}$-$_{7}$ cycloalkyl, "5-8 membered heterocyclylene" refers to a divalent group formed by removing another hydrogen from a 5-8 membered heterocyclyl, "$C_{6}$-$_{10}$ arylene" refers to a divalent group formed by removing another hydrogen from a $C_{6}$-$_{10}$ aryl, and "5-6 membered heteroarylene" refers to a divalent group formed by removing another hydrogen from a 5-6 membered heteroaryl.

Alkyl, alkenyl, alkynyl, cycloalkyl, heterocyclyl, aryl and heteroaryl groups and the like as defined herein are optionally substituted groups.

Exemplary substituents on carbon atoms include, but are not limited to, halogen, -CN, $-NO_2$, $-N_3$, - $SO_2H$, $-SO_3H$, -OH, $-OR^{aa}$, $-ON(R^{bb})_2$, $-N(Rbb)_2$, $-N(R^{bb})_3{}^+X^-$, $-N(OR^{cc})R^{bb}$, -SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, $-C(=O)N(R^{bb})_2$, $-OC(=O)N(R^{bb})_2$, - $NR^{bb}C(=O)R^{aa}$ , $-NR^{bb}CO_2R^{aa}$, $-NR^{bb}C(=O)N(R^{bb})_2$, $-C(=NR^{bb})R^{aa}$, $-C(=NR^{bb})OR^{aa}$, $-OC(=NR^{bb})R^{aa}$, - $OC(=NR^{bb})OR^{aa}$, $-C(=NR^{bb})N(R^{bb})_2$, $-OC(= NR^{bb})N(R^{bb})_2$, $-NR^{bb}C(=NR^{bb})N(R^{bb})_2$, $-C(=O)NR^{bb}SO_2R^{aa}$, - $NR^{bb}SO_2R^{aa}$, $-SO_2N(R^{bb})_2$, $-SO_2R^{aa}$, $-SO_2OR^{aa}$, $-OSO_2R^{aa}$, $-S(=O)R^{aa}$ , $-OS(=O)R^{aa}$, $-Si(R^{aa})_3$, $-OSi(R^{aa})_3$, - $C(=S)N(R^{bb})_2$, $-C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, - $SC(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, $-P(=O)_2N(R^{bb})_2$, - $OP(=O)_2N(R^{bb})_2$, $-P(=O)(NR^{bb})_2$, $-OP(=O)(NR^{bb})_2$, $-NR^{bb}P(=O)(OR^{cc})_2$, $-NR^{bb}P(=O)(NR^{bb})_2$, $-P(R^{cc})_2$, - $P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, cycloalkyl, heterocyclyl, aryl and heteroaryl, wherein each alkyl, alkenyl, alkynyl, cycloalkyl, heterocyclyl, aryl and heteroaryl is independently substituted with 0, 1, 2, 3, 4 or 5 $R^{dd}$ groups;

or two geminal hydrogen atoms on a carbon atom are replaced by groups =O =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=NR^{bb}$ or $=NOR^{cc}$;

each of $R^{aa}$ is independently selected from alkyl, haloalkyl, alkenyl, alkynyl, cycloalkyl, heterocyclyl, aryl and heteroaryl, or two $R^{aa}$ groups combine to form a heterocyclyl or heteroaryl ring, wherein each alkyl, alkenyl, alkynyl, cycloalkyl, heterocyclyl, aryl and heteroaryl is independently substituted with 0, 1, 2, 3, 4 or 5 $R^{dd}$ groups;

Each of $R^{bb}$ is independently selected from hydrogen, -OH, $-OR^{aa}$, $-N(R^{cc})_2$, -CN, $-C(=O)R^{aa}$, - $C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, - $SOR^{aa}$, $-C(=S)N(R^{cc})_2$, $-C(=O)SR^{cc}$ , $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, cycloalkyl, heterocyclyl, aryl and heteroaryl, or two $R^{bb}$ groups combine to form a heterocyclyl or heteroaryl ring, wherein each alkyl, alkenyl, alkynyl, cycloalkyl, heterocyclyl, aryl and heteroaryl is independently substituted with 0, 1, 2, 3, 4 or 5 $R^{dd}$ groups;

each of $R^{cc}$ is independently selected from hydrogen, alkyl, haloalkyl, alkenyl, alkynyl, cycloalkyl, heterocyclyl, aryl and heteroaryl, or two $R^{cc}$ groups combine to form a heterocyclyl or heteroaryl ring, wherein each alkyl, alkenyl, alkynyl, cycloalkyl, heterocyclyl, aryl and heteroaryl is independently substituted with 0, 1, 2, 3, 4 or 5 $R^{dd}$ groups;

Each of $R^{dd}$ is independently selected from: halogen, -CN, $-NO_2$, $-N_3$, $-SO_2H$, $-SO_3H$, -OH, $-OR^{ee}$, - $ON(R^{ff})_2$, $-N(R^{ff})_2$, , $-N(R^{ff})_3{}^+X^-$, $-N(OR^{ee})R^{ff}$, -SH, $-SR^{ee}$, $-SSR^{ee}$, $-C(=O)R^{ee}$, $-CO_2H$, $-CO_2R^{ee}$, $-OC(=O)R^{ee}$, $-OCO_2R^{ee}$, $-C(=O)N(R^{ff})_2$, $-OC(=O)N(R^{ff})_2$, $-NR^{ff}C(=O)R^{ee}$, $-NR^{ff}CO_2R^{ee}$, $-NR^{ff}C(=O)N(R^{ff})_2$, - $C(=NR^{ff})OR^{ee}$, $-OC(=NR^{ff})R^{ee}$, $-OC(=NR^{ff})OR^{ee}$, $-C(=NR^{ff})N(R^{ff})_2$, $-OC(=NR^{ff})N(R^{ff})_2$, - $NR^{ff}C(=NR^{ff})N(R^{ff})_2$, $-NR^{ff}SO_2R^{ee}$, $-SO_2N(R^{ff})_2$, $-SO_2R^{ee}$, $-SO_2OR^{ee}$, $-OSO_2R^{ee}$, $-S(=O)R^{ee}$, $-Si(R^{ee})_3$, - $OSi(R^{ee})_3$, $-C(=S)N(R^{ff})_2$, $-C(=O)SR^{ee}$, $-C(=S)SR^{ee}$, $-SC(=S)SR^{ee}$, $-P(=O)_2R^{ee}$, $-P(=O)(R^{ee})_2$, $-OP(=O)(R^{ee})_2$, $-OP(=O)(OR^{ee})_2$, alkyl, haloalkyl, alkenyl, alkynyl, cycloalkyl, heterocyclyl, aryl, heteroaryl, wherein each alkyl, alkenyl, alkynyl, cycloalkyl, heterocyclyl, aryl and heteroaryl is independently substituted with 0, 1, 2, 3, 4 or 5 $R^{gg}$ groups, or two geminal $R^{dd}$ substituents may be combined to form =O or =S;

Each of $R^{ee}$ is independently selected from alkyl, haloalkyl, alkenyl, alkynyl, cycloalkyl, aryl, heterocyclyl, and heteroaryl, wherein each alkyl, alkenyl, alkynyl, cycloalkyl, heterocyclyl, aryl, and heteroaryl is independently substituted with 0, 1, 2, 3, 4, or 5 $R^{gg}$ groups;

Each of $R^{ff}$ is independently selected from hydrogen, alkyl, haloalkyl, alkenyl, alkynyl, cycloalkyl, heterocyclyl, aryl and heteroaryl, or two $R^{ff}$ groups combine to form a heterocyclyl or heteroaryl ring, wherein each alkyl, alkenyl, alkynyl, cycloalkyl, heterocyclyl, aryl and heteroaryl is independently substituted with 0, 1, 2, 3, 4 or 5 $R^{gg}$ groups;

Each of $R^{gg}$ is independently: halogen, -CN, $-NO_2$, $-N_3$, $-SO_2H$, $-SO_3H$, -OH, $-OC_{1-6}$ alkyl, $-ON(C_{1-6}$ alkyl$)_2$, $-N(C_{1-6}$ alkyl$)_2$, $-N(C_{1-6}$ alkyl$)_3^+X^-$, $-NH(C_{1-6}$ alkyl$)_2^+X^-$, $-NH_2(C_{1-6}$ alkyl$)^+X^-$, $-NH_3^+X^-$, $-N(OC_{1-6}$ alkyl$)(C_{1-6}$ alkyl), $-N(OH)(C_{1-6}$ alkyl), -NH(OH), -SH, $-SC_{1-6}$ alkyl, $-SS(C_{1-6}$ alkyl), $-C(=O)(C_{1-6}$ alkyl), $-CO_2H$, $-CO_2(C_{1-6}$ alkyl), $-OC(=O)(C_{1-6}$ alkyl), $-OCO_2(C_{1-6}$ alkyl), $-C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl$)C(=O)(C_{1-6}$ alkyl), $-NHCO_2(C_{1-6}$ alkyl), $-NHC(=O)N(C_{1-6}$ alkyl$)_2$, $-NHC(=O)NH(C_{1-6}$ alkyl), $-NHC(=O)NH_2$, $-C(=NH)O(C_{1-6}$ alkyl), $-OC(=NH)(C_{1-6}$ alkyl), $-OC(=NH)OC_{1-6}$ alkyl, $-C(=NH)N(C_{1-6}$ alkyl$)_2$, $-C(=NH)NH(C_{1-6}$ alkyl), $-C(=NH)NH_2$, $-OC(=NH)N(C_{1-6}$ alkyl$)_2$, $-OC(NH)NH(C_{1-6}$ alkyl), $-OC(NH)NH_2$, $-NHC(NH)N(C_{1-6}$ alkyl$)_2$, $-NHC(=NH)NH_2$, $-NHSO_2(C_{1-6}$ alkyl), $-SO_2N(C_{1-6}$ alkyl$)_2$, $-SO_2NH(C_{1-6}$ alkyl), $-SO_2NH_2$, $-SO_2C_{1-6}$ alkyl, $-SO_2OC_{1-6}$ alkyl, $-OSO_2C_{1-6}$ alkyl, $-SOC_{1-6}$ alkyl, $-Si(C_{1-6}$ alkyl$)_3$, $-OSi(C_{1-6}$ alkyl$)_3$, $-C(=S)N(C_{1-6}$ alkyl$)_2$, $C(=S)NH(C_{1-6}$ alkyl), $C(=S)NH_2$, $-C(=O)S(C_{1-6}$ alkyl), $-C(=S)SC_{1-6}$ alkyl, $-SC(=S)SC_{1-6}$ alkyl, $-P(=O)_2(C_1$-6 alkyl), $-P(=O)(C_{1-6}$ alkyl$)_2$, $-OP(=O)(C_{1-6}$ alkyl$)_2$, $-OP(=O)(OC_{1-6}$alkyl)z, $C_{1-6}$ alkyl, $C_{1-6}$haloalkyl, $C_2$-$C_6$ alkenyl, $C_2$-$C_6$ alkynyl, $C_3$-$C_7$ cycloalkyl, $C_6$-$C_{10}$ aryl, $C_3$-$C_7$ heterocyclyl, $C_5$-$C_{10}$ heteroaryl; or two geminal $R^{gg}$ substituents may be combined to form =O or =S; wherein $X^-$ is a counter ion.

Exemplary substituents on the nitrogen atom include, but are not limited to, hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{bb})R^{aa}$, $-C(=NR^{cc})OR^{aa}$, $-C(=NR^{cc})N(R^{cc})_2$, $-SO_2N(R^{cc})2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, cycloalkyl, heterocyclyl, aryl and heteroaryl, or two $R^{cc}$ groups attached to the nitrogen atom combine to form a heterocyclyl or heteroaryl ring, wherein each alkyl, alkenyl, alkynyl, cycloalkyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as described above.

The term "deuterium (D or 2H)" is a stable isotope of hydrogen that exists at a natural abundance of 0.015 mol%. The term "deuterated" refers to a group or compound in which one or more hydrogen atoms H are replaced by D.

"Deuterated compound" refers to a compound in which one or more hydrogens bonded to carbon are replaced by one or more deuteriums. Similarly, "deuterated" refers to a chemical structure or organic group in which one or more hydrogens bonded to carbon are replaced by one or more deuteriums, for example, "deuterated alkyl", "deuterated cycloalkyl", "deuterated heterocycloalkyl", "deuterated aryl", etc. For example, "deuterated alkyl" refers to an alkyl group defined in the present application, in which at least one hydrogen atom bonded to carbon is replaced by deuterium. In a deuterated alkyl, at least one carbon atom is bonded to one deuterium; one carbon atom can be bonded to multiple deuteriums; and multiple carbon atoms in an alkyl group can also be bonded to deuterium. For example, deuterated methyl includes methyl-d3, in which three hydrogen atoms are replaced by deuterium; it also includes monodeuterated methyl and dideuterated methyl. In some embodiments, the compounds of the present invention include deuterated compounds.

## Other Definitions

The term "pharmaceutically acceptable salt" as used herein refers to those carboxylic acid and amino acid addition salts of the compounds of the present invention which are suitable for use in contact with patient tissues within the scope of sound medical judgment, do not produce undue toxicity, irritation, allergic response, etc., are commensurate with a reasonable benefit/risk ratio, and are effective for their intended use, including (where possible) zwitterionic forms of the compounds of the present invention.

"Subjects" for administration include, but are not limited to, humans (i.e., males or females of any age group, e.g., pediatric subjects (e.g., infants, children, adolescents) or adult subjects (e.g., young adults, middle-aged adults, or older adults)) and/or non-human animals, e.g., mammals, e.g., primates (e.g., cynomolgus monkeys, rhesus monkeys), cattle, pigs, horses, sheep, goats, rodents, cats, and/or dogs. In some embodiments, the subject is a human. In some embodiments, the subject is a non-human animal. The terms "human," "patient," and "subject" are used interchangeably herein.

"Disease," "disorder," and "condition" are used interchangeably herein.

Generally, the "effective amount" of a compound refers to an amount sufficient to cause a target biological response. As will be appreciated by those of ordinary skill in the art, the effective amount of the compounds of the invention may vary depending on factors such as the biological target, the pharmacokinetics of the compound, the disease being treated, the mode of administration, and the age, health, and symptoms of the subject. An effective amount includes a therapeutically effective amount and a prophylactically effective amount.

"Combination" and related terms refer to the simultaneous or sequential administration of the compounds of the invention and other therapeutic agents. For example, the compounds of the invention can be administered simultaneously or sequentially with the other therapeutic agents in separate unit dosage forms, or can be administered simultaneously with the other therapeutic agents in a single unit dosage form.

## DETAILED DESCRIPTION OF THE PREFERRED EMBODIMENTS

As used herein, "compounds of the present invention" refers to compounds of the following formula (I) (including sub-formulae, such as formula (IIa), (IIb), (III), (IV), (III-1), (III-2), (III-3), (IV-1), (IV-2), (V-1), (V-2), (VI-1), (VI-2), (VII-1) or (VII-2) etc.), pharmaceutically acceptable salts, enantiomers, diastereoisomers or isotopic variants thereof, and mixtures thereof.

In one embodiment, the present invention relates to a compound of formula (I), or a pharmaceutically acceptable salt, isotopic variant, tautomer, or stereoisomer thereof:

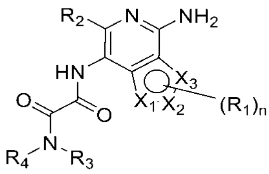

(I)

$X_1$, $X_2$ and $X_3$ are each independently selected from CH, O, S or N, and $X_1$, $X_2$ and $X_3$ contain at least one N atom, the circle indicating aromaticity;

$R_1$ is selected from H, $C_{1-6}$ alkyl, deuterated $C_{1-6}$ alkyl, $C_{3-6}$ cycloalkyl or $C_{1-6}$ haloalkyl;

$R_2$ is selected from H, $C_{1-6}$ alkyl or halogen;

$R_3$ and $R_4$ are independently selected from H, $C_{1-6}$ alkyl, $C_{3-6}$ cycloalkyl or 4-10 membered heterocyclyl, wherein the $C_{1-6}$ alkyl, $C_{3-6}$ cycloalkyl or 4-10 membered heterocyclyl in $R_3$ or $R_4$ is each optionally substituted by 1 or 2 $R_5$, wherein $R_5$ is selected from hydrogen, deuterium, halogen, $C_{1-6}$ alkyl, $C_{1-6}$ alkoxy, $C_{1-6}$ haloalkyl, CN, $SF_5$, $C_{3-6}$ cycloalkyl, $-S(O)_2CF_3$, $-OCF_3$, $-SCF_3$, $-SCH_3$ or 5-10 membered heteroaryl; the 5-10 membered heteroaryl is optionally further substituted by $R_6$, wherein $R_6$ is selected from H, CN, halogen, $C_{1-6}$ alkyl, $C_{1-6}$ haloalkyl, $SF_5$, $OR_a$ or 5-6 membered heteroaryl, the 5-6 membered heteroaryl being optionally substituted by 1, 2 or 3 $R_z$;

alternatively, $R_3$, $R_4$ and the nitrogen atom to which they are attached together form a 4-10 membered heterocyclic group, the 4-10 membered heterocyclic group comprising 0 or 1 additional heteroatom selected from oxygen, nitrogen or sulfur, and being optionally substituted with 1, 2, 3 or 4 $R_x$;

$R_x$ is selected from $C_{1-6}$ alkyl, $C_{1-6}$ haloalkyl, phenyl or 5-10 membered heteroaryl, wherein the phenyl or 5-10 membered heteroaryl is optionally substituted with 1, 2 or 3 $R_y$;

$R_y$ is selected from halogen, $C_{1-6}$ alkyl, $C_{1-6}$ haloalkyl, $SF_5$, CN, $OR_a$, $C_{1-6}$ alkoxy, $-NR_{z1}R_{z2}$, $NR_{z1}R_{z2}$-$C_{1-6}$ alkyl-, $C_{3-6}$ cycloalkyl, 4-12 membered heterocyclyl or 5-10 membered heteroaryl, wherein the 4-12 membered heterocyclyl or 5-10 membered heteroaryl is optionally substituted by 1, 2 or 3 $R_z$;

$R_z$ is selected from $C_{1-6}$ alkyl, halogen, CN, $C_{1-6}$ haloalkyl or $C_{1-6}$ alkoxy;

$R_a$ is selected from H, $C_{1-6}$ alkyl or $C_{1-6}$ haloalkyl;

$R_{z1}$ and $R_{z2}$ are each independently selected from H or $C_{1-6}$ alkyl; and

*n* is 0, 1 or 2.

In some embodiments, $X_1$, $X_2$ and $X_3$ are each independently selected from -CH=, -O-, -S-, -N= or - NH-, and at least one of $X_1$, $X_2$ and $X_3$ is -N= or -NH-.

In some embodiments, $-NR_3R_4$ is

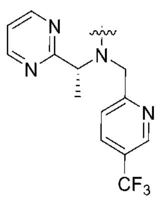, 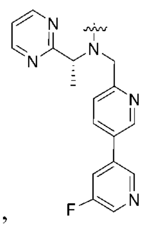, 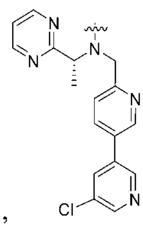, 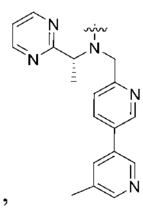, 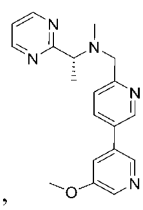

or

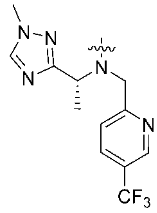

.

In some embodiments, $-NR_3R_4$ is

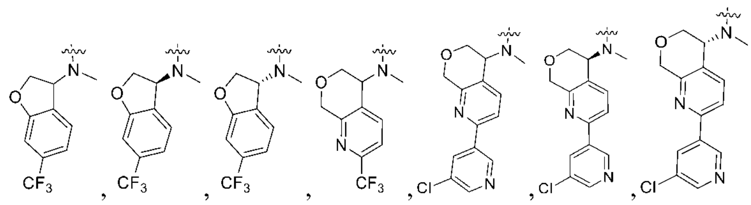

, , , , , , ,

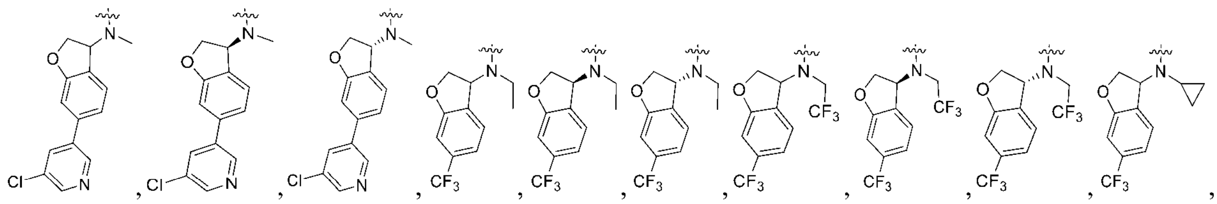

, , , , , , , , , , ,

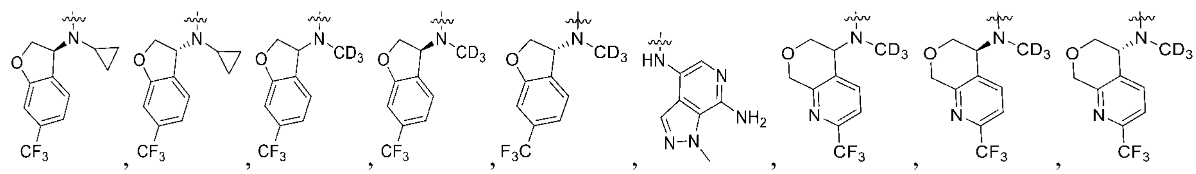

, , , , , , , , ,

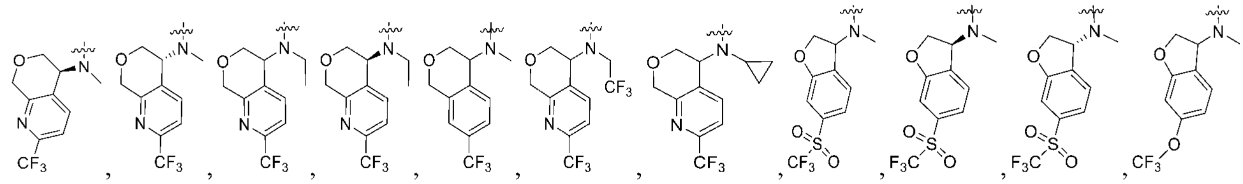

, , , , , , , , , , ,

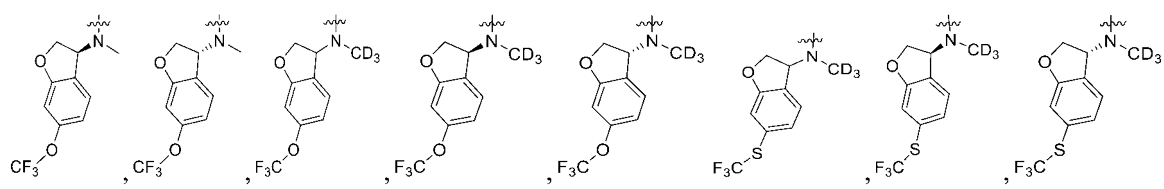

, , , , , , , ,

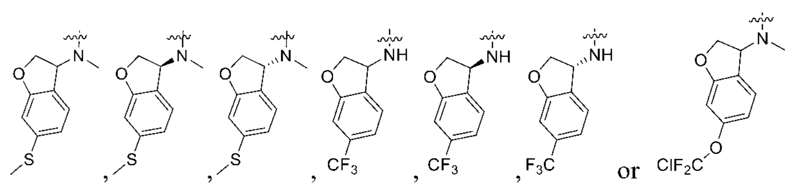

, , , , , or

.

In some embodiments, $-NR_3R_4$ is

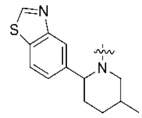, 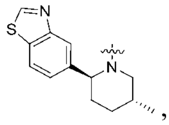, 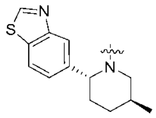, 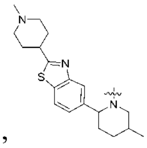, 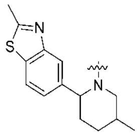,

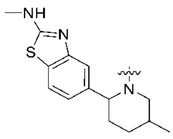, 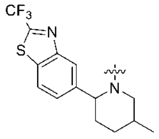, 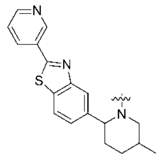, 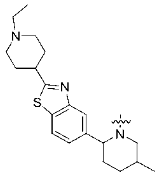, 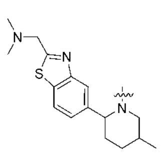, 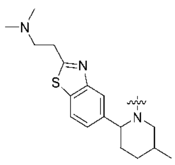, 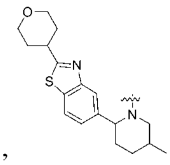,

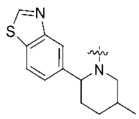, 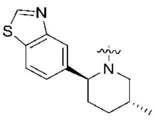, 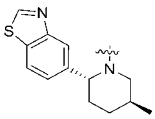, 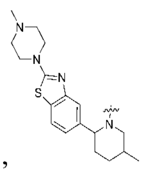, 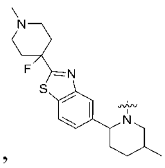 or 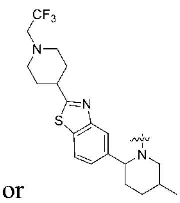.

In some embodiments, $-NR_3R_4$ is

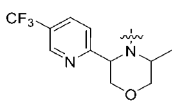, 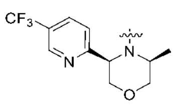, 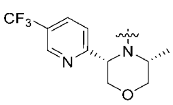, 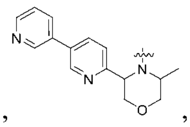,

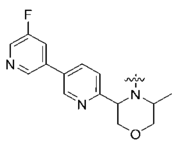, 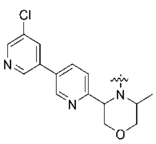, 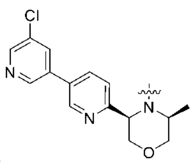, 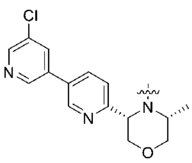, 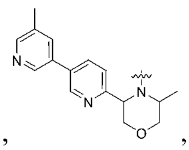, 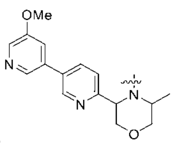

or

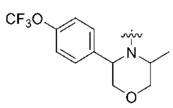.

In another embodiment, the present invention relates to a compound of formula (IIa) or formula (IIb), or a pharmaceutically acceptable salt, isotopic variant, tautomer or stereoisomer thereof:

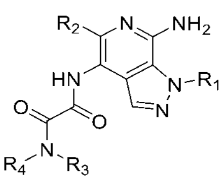 (IIa) or 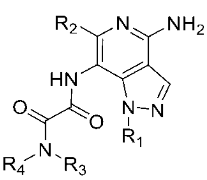 (IIb)

wherein,

$R_1$ is selected from H, $C_{1-6}$ alkyl, deuterated $C_{1-6}$ alkyl, $C_{3-6}$ cycloalkyl or $C_{1-6}$ haloalkyl;
$R_2$ is selected from H, $C_{1-6}$ alkyl or halogen;
$R_3$ and $R_4$ are independently selected from H, $C_{1-6}$ alkyl, $C_{3-6}$ cycloalkyl or 4-10 membered heterocyclyl, wherein the $C_{1-6}$ alkyl, $C_{3-6}$ cycloalkyl or 4-10 membered heterocyclyl in $R_3$ or $R_4$ is each optionally substituted by 1 or 2 $R_5$, wherein $R_5$ is selected from hydrogen, deuterium, halogen, $C_{1-6}$ alkyl, $C_{1-6}$ alkoxy, $C_{1-6}$ haloalkyl, CN, $SF_5$, $C_{3-6}$

cycloalkyl, $-S(O)_2CF_3$, $-OCF_3$, $-SCF_3$, $-SCH_3$ or 5-10 membered heteroaryl; the 5-10 membered heteroaryl is optionally further substituted by $R_6$, wherein $R_6$ is selected from H, CN, halogen, $C_{1-6}$ alkyl, $C_{1-6}$ haloalkyl, $SF_5$, $OR_a$ or 5-6 membered heteroaryl, and the 5-6 membered heteroaryl is optionally substituted by 1, 2 or 3 $R_z$;
alternatively, $R_3$, $R_4$ and the nitrogen atom to which they are attached together form a 4-10 membered heterocyclic group, the 4-10 membered heterocyclic group comprising 0 or 1 additional heteroatom selected from oxygen, nitrogen or sulfur, and being optionally substituted with 1, 2, 3 or 4 $R_x$;
$R_x$ is selected from $C_{1-6}$ alkyl, $C_{1-6}$ haloalkyl, phenyl or 5-10 membered heteroaryl, wherein the phenyl or 5-10 membered heteroaryl is optionally substituted with 1, 2 or 3 $R_y$;
$R_y$ is selected from halogen, $C_{1-6}$ alkyl, $C_{1-6}$ haloalkyl, $SF_5$, CN, $OR_a$, $C_{1-6}$ alkoxy, $-NR_{z1}R_{z2}$, $NR_{z1}R_{z2}$-$C_{1-6}$ alkyl-, $C_{3-6}$ cycloalkyl, 4-12 membered heterocyclyl or 5-10 membered heteroaryl, wherein the 4-12 membered heterocyclyl or 5-10 membered heteroaryl is optionally substituted by 1, 2 or 3 $R_z$;
$R_z$ is selected from $C_{1-6}$ alkyl, halogen, CN, $C_{1-6}$ haloalkyl or $C_{1-6}$ alkoxy;
$R_a$ is selected from H, $C_{1-6}$ alkyl or $C_{1-6}$ haloalkyl; and
$R_{z1}$ and $R_{z2}$ are each independently selected from H or $C_{1-6}$ alkyl.

In another embodiment, the present invention provides a compound of formula (III) or formula (IV), or a pharmaceutically acceptable salt, isotopic variant, tautomer or stereoisomer thereof:

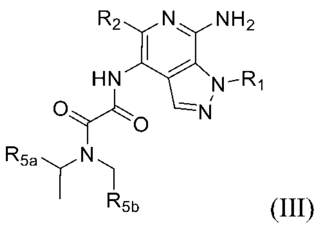
(III) or
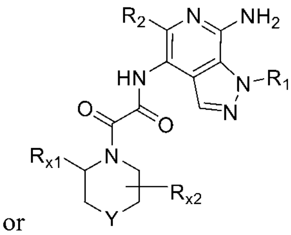
(IV)

$R_1$ is selected from H, $C_{1-6}$ alkyl, deuterated $C_{1-6}$ alkyl, $C_{3-6}$ cycloalkyl or $C_{1-6}$ haloalkyl;
$R_2$ is selected from H, methyl, Cl or F;
$R_{5a}$ or $R_{5b}$ are each independently selected from 5-10 membered heteroaryl; the 5-10 membered heteroaryl is optionally substituted by $R_6$, $R_6$ being selected from H, CN, halogen, $C_{1-6}$ alkyl, $C_{1-6}$ haloalkyl, $SF_5$, $OR_a$ or 5-6 membered heteroaryl; the 5-6 membered heteroaryl is optionally substituted by 1, 2 or 3 $R_z$;
$R_{x1}$ is selected from phenyl or 5-10 membered heteroaryl, wherein the phenyl or 5-10 membered heteroaryl is optionally substituted with 1, 2 or 3 $R_y$;
$R_y$ is selected from halogen, $C_{1-6}$ alkyl, $C_{1-6}$ haloalkyl, $SF_5$, CN, $OR_a$, $C_{1-6}$ alkoxy, $-NR_{z1}R_{z2}$, $NR_{z1}R_{z2}$-$C_{1-6}$Alkyl-, $C_{3-6}$ cycloalkyl, 4-12 membered heterocyclyl or 5-10 membered heteroaryl, wherein the 4-12 membered heterocyclyl or 5-10 membered heteroaryl is optionally substituted by 1, 2 or 3 $R_z$;
$R_z$ is selected from $C_{1-6}$ alkyl, halogen, CN, $C_{1-6}$ haloalkyl or $C_{1-6}$ alkoxy;
$R_a$ is selected from H, $C_{1-6}$ alkyl or $C_{1-6}$ haloalkyl;
$R_{x2}$ is selected from $C_{1-6}$ alkyl or $C_{1-6}$ haloalkyl;
$R_{z1}$ and $R_{z2}$ are each independently selected from H or $C_{1-6}$ alkyl; and
Y is selected from O, S or $CH_2$.

In another embodiment, the present invention provides a compound of formula (VI) or formula (VII), or a pharmaceutically acceptable salt, isotopic variant, tautomer or stereoisomer thereof:

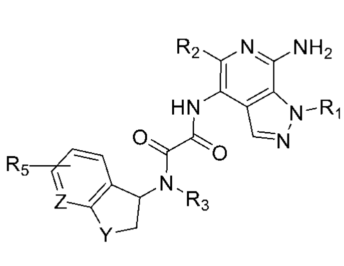
(VI) or
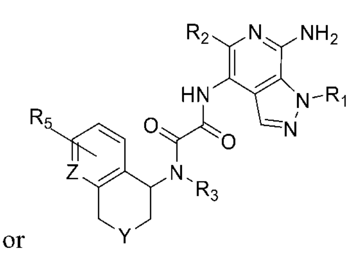
(VII)

$R_1$ is selected from H, $C_{1-6}$ alkyl, deuterated $C_{1-6}$ alkyl, $C_{3-6}$ cycloalkyl or $C_{1-6}$ haloalkyl;
$R_2$ is selected from H, methyl, Cl or F;
$R_3$ is selected from H, $C_{1-6}$ alkyl, deuterated $C_{1-6}$ alkyl, $C_{3-6}$ cycloalkyl or 4-10 membered heterocyclyl; the $C_{1-6}$ alkyl or

4-10 membered heterocyclyl is optionally substituted by 1 or 2 $R_5$;
$R_5$ is selected from hydrogen, deuterium, halogen, $C_{1-6}$ alkyl, $C_{1-6}$ alkoxy, $C_{1-6}$ haloalkyl, $C_{1-6}$ haloalkoxy, CN, $SF_5$, $C_{3-6}$ cycloalkyl, $-S(O)_2CF_3$, $-OCF_3$, $-SCF_3$, $-SCH_3$ or 5-10 membered heteroaryl; the 5-10 membered heteroaryl is optionally further substituted with $R_6$, $R_6$ being selected from H, CN, halogen, $C_{1-6}$ alkyl, $C_{1-6}$ haloalkyl, $SF_5$, $OR_a$ or 5-6 membered heteroaryl; the 5-6 membered heteroaryl is optionally substituted with 1, 2 or 3 $R_z$;
$R_z$ is selected from $C_{1-6}$ alkyl, halogen, CN, $C_{1-6}$ haloalkyl or $C_{1-6}$ alkoxy;
$R_a$ is selected from H, $C_{1-6}$ alkyl or $C_{1-6}$ haloalkyl;
Y is selected from NH, O, S or $CH_2$;
Z is selected from N or CH.

In some embodiments, $R_1$ is $CH_3$ or $CD_3$.

In some embodiments, the compound is

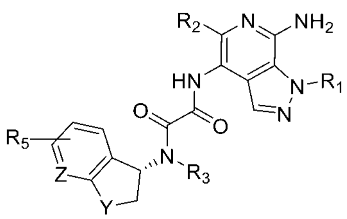 (VIA) or 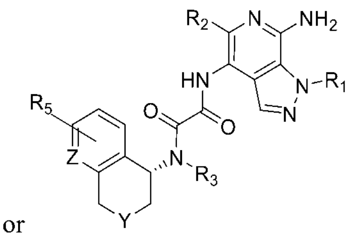 (VIIA).

In some embodiments, the compound is

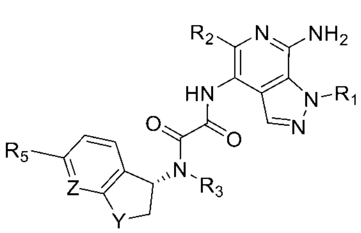 (VIB) or 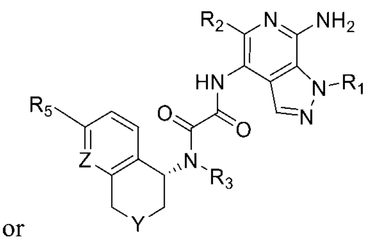 (VIIB).

In some embodiments,

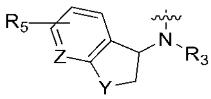 or 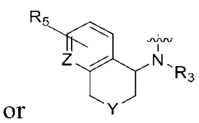

for:

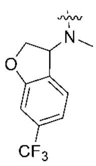, 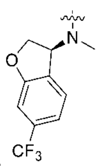, 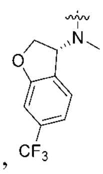, 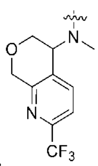, 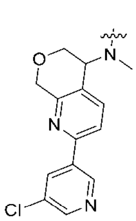, 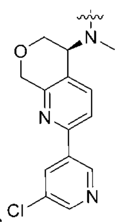, 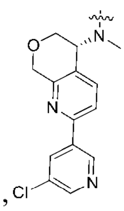, 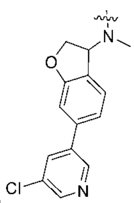, 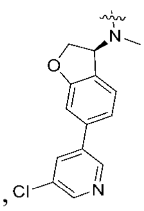,

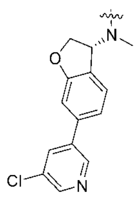, 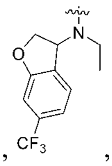, 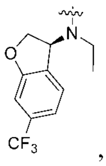, 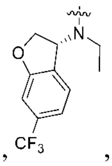, 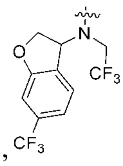, 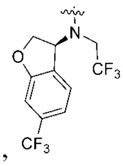, 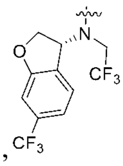, 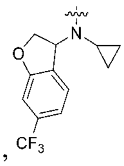, 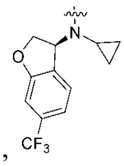, 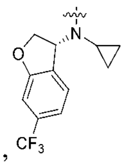,

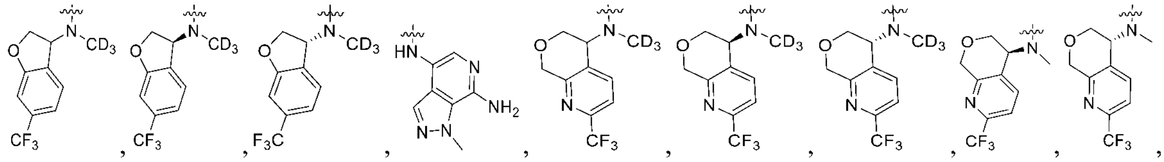

, , , , , , , , ,

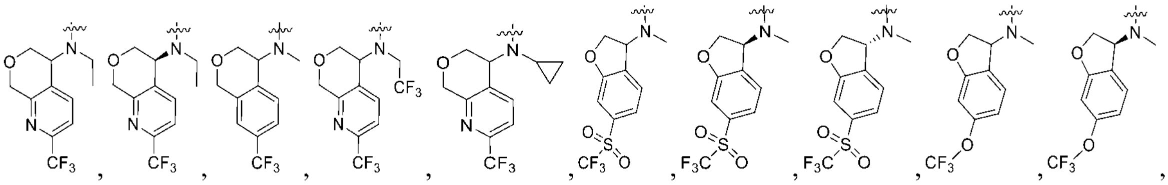

, , , , , , , , , ,

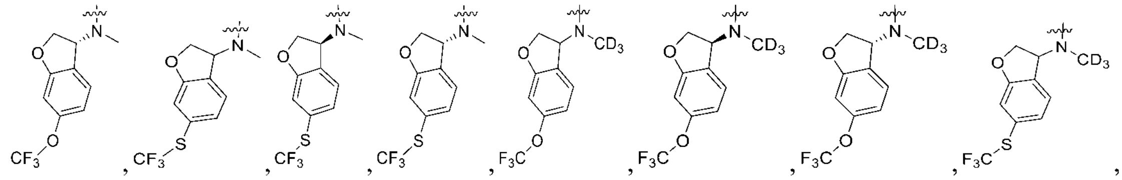

, , , , , , , ,

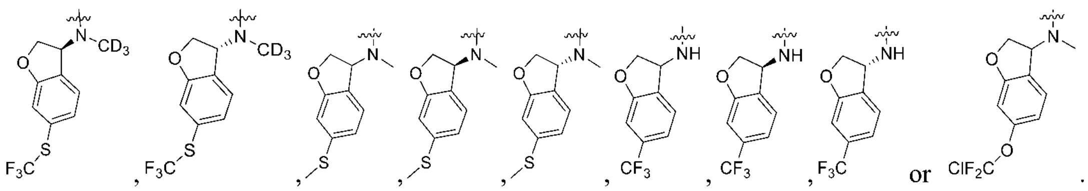

, , , , , , , or .

In another embodiment, the present invention provides a compound of formula (III-1), or a pharmaceutically acceptable salt, isotopic variant, tautomer or stereoisomer thereof:

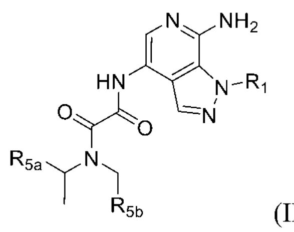

(III-1)

$R_1$ is selected from methyl, $CD_3$, ethyl, or cyclopropyl;

$R_{5a}$ or $R_{5b}$ are each independently selected from 6-membered heteroaryl; the 6-membered heteroaryl is optionally further substituted by $R_6$, $R_6$ being selected from H, CN, halogen, $C_{1-6}$ alkyl, $C_{1-6}$ haloalkyl, $SF_5$, $OR_a$ or 5-6-membered heteroaryl; the 5-6-membered heteroaryl is optionally substituted by 1, 2 or 3 $R_z$;

$R_z$ is selected from $C_{1-6}$ alkyl, halogen, CN, $C_{1-6}$ haloalkyl or $C_{1-6}$ alkoxy;

$R_a$ is selected from H, $C_{1-6}$ alkyl or $C_{1-6}$ haloalkyl.

In another more specific embodiment, the present invention provides a compound of formula (III-2), or a pharmaceutically acceptable salt, isotopic variant, tautomer or stereoisomer thereof:

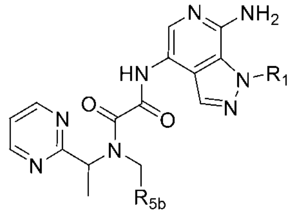

(III-2)

$R_1$ is selected from methyl, $CD_3$, ethyl, or cyclopropyl;

$R_{5b}$ is

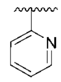,

which is optionally further substituted by $R_6$, $R_6$ being selected from H, CN, halogen, $C_1$-6 alkyl, $C_{1-6}$ haloalkyl, $SF_5$, $OR_a$ or 5-6 membered heteroaryl; the 5-6 membered heteroaryl is optionally substituted by 1, 2 or 3 $R_z$;

$R_z$ is selected from $C_{1-6}$ alkyl, halogen, CN, $C_{1-6}$ haloalkyl or $C_{1-6}$ alkoxy;

$R_a$ is selected from H, $C_{1-6}$ alkyl or $C_{1-6}$ haloalkyl.

In another more specific embodiment, the present invention provides a compound of formula (III-3), or a pharmaceutically acceptable salt, isotopic variant, tautomer or stereoisomer thereof:

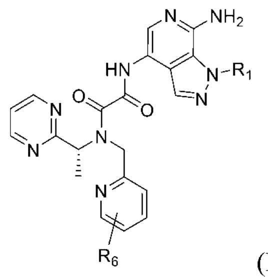

(III-3)

$R_1$ is selected from methyl, $CD_3$, ethyl or cyclopropyl;

$R_6$ is selected from H, CN, halogen, $C_{1-6}$ alkyl, $C_{1-6}$ haloalkyl, $SF_5$, $OR_a$ or 5-6 membered heteroaryl; the 5-6 membered heteroaryl is optionally substituted by 1, 2 or 3 $R_z$;

$R_z$ is selected from $C_{1-6}$ alkyl, halogen, CN, $C_{1-6}$ haloalkyl or $C_{1-6}$ alkoxy;

$R_a$ is selected from H, $C_{1-6}$ alkyl or $C_{1-6}$ haloalkyl.

In another embodiment, the present invention provides a compound of formula (IV-1), or a pharmaceutically acceptable salt, isotopic variant, tautomer or stereoisomer thereof:

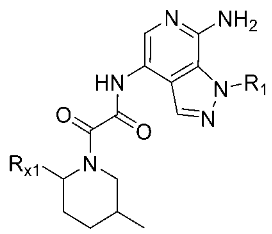

(IV-1),

$R_1$ is selected from $C_{1-6}$ alkyl, deuterated $C_{1-6}$ alkyl, $C_{3-6}$ cycloalkyl or $C_{1-6}$ haloalkyl;

$R_{x1}$ is selected from phenyl or 5-10 membered heteroaryl, wherein the phenyl or 5-10 membered heteroaryl is optionally substituted with 1, 2 or 3 $R_y$;

$R_y$ is selected from halogen, $C_{1-6}$ alkyl, $C_{1-6}$ haloalkyl, $SF_5$, CN, $OR_a$, $C_{1-6}$ alkoxy, $C_{3-6}$ cycloalkyl,

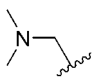, 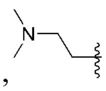,

4-12 membered heterocyclyl or 5-10 membered heteroaryl, wherein the 4-12 membered heterocyclyl or 5-10 membered heteroaryl is optionally substituted with 1, 2 or 3 $R_z$;

$R_z$ is selected from $C_{1-6}$ alkyl, halogen, CN, $C_{1-6}$ haloalkyl or $C_{1-6}$ alkoxy;

$R_a$ is selected from H, $C_{1-6}$ alkyl or $C_{1-6}$ haloalkyl.

In another more specific embodiment, the present invention provides a compound of formula (IV-2), or a

pharmaceutically acceptable salt, isotopic variant, tautomer or stereoisomer thereof:

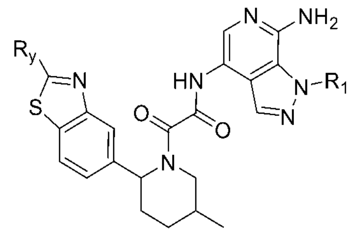

(IV-2),

wherein,

$R_1$ is selected from methyl, $CD_3$, ethyl or cyclopropyl;
$R_y$ is selected from $C_{1-6}$ alkyl, $C_{1-6}$ haloalkyl, CN, $OR_a$, $C_{1-6}$ alkoxy, $C_{3-6}$ cycloalkyl,

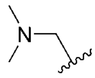 , 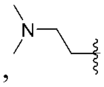 ,

4-12 membered heterocyclyl or 5-10 membered heteroaryl, wherein the 4-12 membered heterocyclyl or 5-10 membered heteroaryl is optionally substituted with 1, 2 or 3 $R_z$;
$R_z$ is selected from $C_{1-6}$ alkyl, halogen, CN, $C_{1-6}$ haloalkyl or $C_{1-6}$ alkoxy;

In another embodiment, the present invention provides a compound of formula (V-1), or a pharmaceutically acceptable salt, isotopic variant, tautomer or stereoisomer thereof:

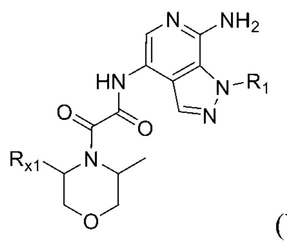

(V-1)

wherein,

$R_1$ is selected from $C_{1-6}$ alkyl, deuterated $C_{1-6}$ alkyl, $C_{3-6}$ cycloalkyl or $C_{1-6}$ haloalkyl;
$R_{x1}$ is a 5-10 membered heteroaryl group, which is optionally substituted by 1, 2 or 3 $R_y$ groups;
$R_y$ is selected from halogen, $C_{1-6}$ alkyl, $C_{1-6}$ haloalkyl, $SF_5$, CN, $OR_a$, $C_{1-6}$ alkoxy, $C_{3-6}$ cycloalkyl, 4-12 membered heterocyclyl or 5-10 membered heteroaryl, wherein the 5-10 membered heteroaryl is optionally substituted with 1, 2 or 3 $R_z$;
$R_z$ is selected from $C_{1-6}$ alkyl, halogen, CN, $C_{1-6}$ haloalkyl or $C_{1-6}$ alkoxy;
$R_a$ is selected from H, $C_{1-6}$ alkyl or $C_{1-6}$ haloalkyl.

In another more specific embodiment, the present invention provides a compound of formula (V-2), or a pharmaceutically acceptable salt, isotopic variant, tautomer or stereoisomer thereof:

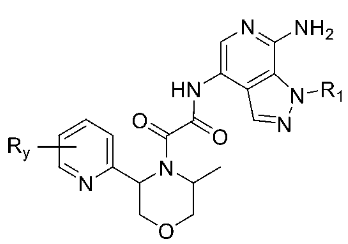

(V-2)

wherein,

$R_1$ is selected from methyl, $CD_3$, ethyl or cyclopropyl;

$R_y$ is selected from halogen, $C_{1-6}$ alkyl, $C_{1-6}$ haloalkyl, $SF_5$, CN, $OR_a$, $C_{1-6}$ alkoxy, $C_{3-6}$ cycloalkyl, 4-12 membered heterocyclyl or 5-10 membered heteroaryl, wherein the 5-10 membered heteroaryl is optionally substituted with 1, 2 or 3 $R_z$;

$R_z$ is selected from $C_{1-6}$ alkyl, halogen, CN, $C_{1-6}$ haloalkyl or $C_{1-6}$ alkoxy;

$R_a$ is selected from H, $C_{1-6}$ alkyl or $C_{1-6}$ haloalkyl.

In another more specific embodiment, the present invention provides a compound of formula (V-3), or a pharmaceutically acceptable salt, isotopic variant, tautomer or stereoisomer thereof:

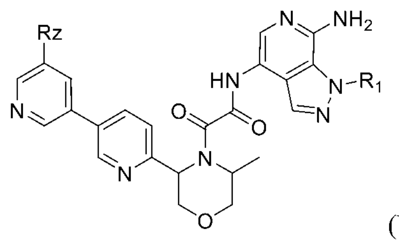

(V-3)

wherein,

$R_1$ is selected from methyl, $CD_3$, ethyl or cyclopropyl;

$R_z$ is selected from $C_{1-6}$ alkyl, halogen, CN, $C_{1-6}$ haloalkyl or $C_{1-6}$ alkoxy;

$R_a$ is selected from H, $C_{1-6}$ alkyl or $C_{1-6}$ haloalkyl.

In another embodiment, the present invention provides a compound of formula (VI-1) or (VII-1), or a pharmaceutically acceptable salt, isotopic variant, tautomer or stereoisomer thereof:

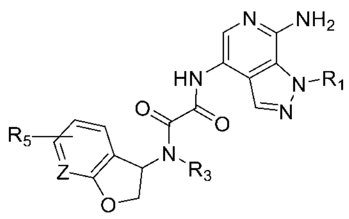

(VI-1) or

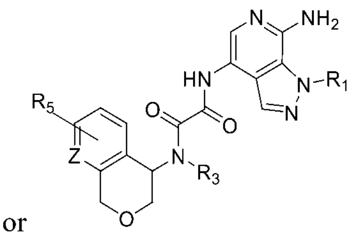

(VII-1)

$R_1$ is selected from H, $C_{1-6}$ alkyl, deuterated $C_{1-6}$ alkyl, $C_{3-6}$ cycloalkyl or $C_{1-6}$ haloalkyl;

$R_3$ is selected from H, $C_{1-6}$ alkyl, deuterated $C_{1-6}$ alkyl, $C_{3-6}$ cycloalkyl or 4-10 membered heterocyclyl; the $C_{1-6}$ alkyl or 4-10 membered heterocyclyl is optionally substituted by 1 or 2 $R_5$;

$R_5$ is selected from hydrogen, halogen, $C_{1-6}$ alkyl, $C_{1-6}$ alkoxy, $C_{1-6}$ haloalkyl, $C_{1-6}$ haloalkoxy, CN, $SF_5$, $C_{3-6}$ cycloalkyl, $-S(O)_2CF_3$, $-OCF_3$, $-SCF_3$, $-SCH_3$ or 5-10 membered heteroaryl; the 5-10 membered heteroaryl is optionally further substituted with $R_6$, $R_6$ is selected from H, CN, halogen, $C_{1-6}$ alkyl, $C_{1-6}$ haloalkyl, $SF_5$, $OR_a$ or 5-6 membered heteroaryl;

$R_a$ is selected from H, $C_{1-6}$ alkyl or $C_{1-6}$ haloalkyl;

Z is selected from N or CH.

In some embodiments, $R_3$ is selected from $C_{1-6}$ alkyl, deuterated $C_{1-6}$ alkyl, $C_{3-6}$ cycloalkyl, or 4-10 membered heterocyclyl; the $C_{1-6}$ alkyl or 4-10 membered heterocyclyl is optionally substituted with 1 or 2 $R_5$.

In another more specific embodiment, the present invention provides a compound of formula (VI-2) or (VII-2), or a pharmaceutically acceptable salt, isotopic variant, tautomer or stereoisomer thereof:

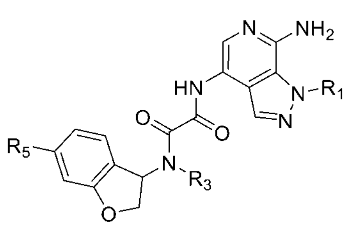
(VI-2) or
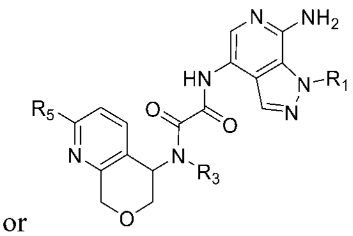
(VII-2)

$R_1$ is selected from H, methyl, $CD_3$, ethyl or cyclopropyl;
$R_3$ is selected from H, $C_{1-6}$ alkyl, deuterated $C_{1-6}$ alkyl, $C_{3-6}$ cycloalkyl or 4-10 membered heterocyclyl;
$R_5$ is selected from hydrogen, halogen, $C_{1-6}$ alkyl, $C_{1-6}$ alkoxy, $C_{1-6}$ haloalkyl, $C_{1-6}$ haloalkoxy, CN, $SF_5$, $C_{3-6}$ cycloalkyl, $-S(O)_2CF_3$, $-OCF_3$, $-SCF_3$, $-SCH_3$ or 5-10 membered heteroaryl;
in some embodiments, $R_3$ is selected from $C_{1-6}$ alkyl, deuterated $C_{1-6}$ alkyl, $C_{3-6}$ cycloalkyl, or 4-10 membered heterocyclyl.

In another more specific embodiment, the compound of formula (VI-2) or (VII-2), or a pharmaceutically acceptable salt, isotopic variant, tautomer or stereoisomer thereof:

$R_1$ is selected from H, methyl, $CD_3$, ethyl or cyclopropyl;
$R_3$ is selected from H, methyl, $CD_3$, ethyl or cyclopropyl;
$R_5$ is selected from hydrogen, F, Cl, Br, methyl, $CD_3$, ethyl, methoxy, trifluoromethyl, difluoromethyl, CN, $SF_5$, cyclopropyl, $-S(O)_2CF_3$, $-OCF_3$, $-OCF_2Cl$, $-SCF_3$, $-SCH_3$ or pyridyl.

In some embodiments, $R_3$ is selected from methyl, $CD_3$, ethyl or cyclopropyl.
In some embodiments, the compound is

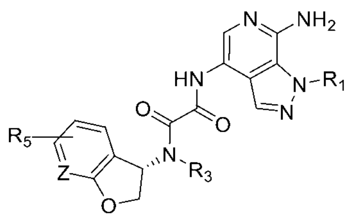
(VI-1A) or
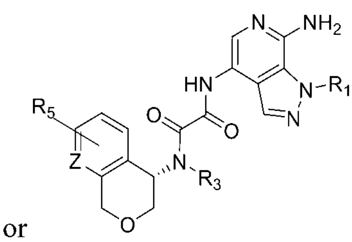
(VII-2A).

In some embodiments, the compound is

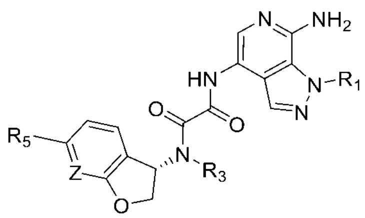
(VI-1B) or
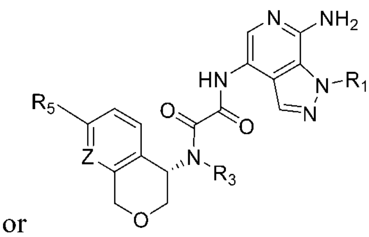
(VII-2B).

In some embodiments, the compound is

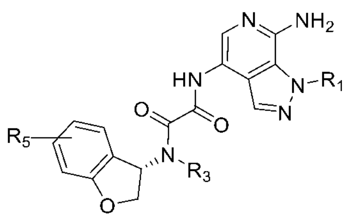
(VI-1C) or
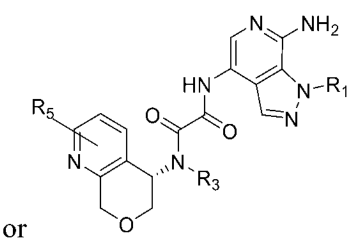
(VII-2C).

In some embodiments, the compound is

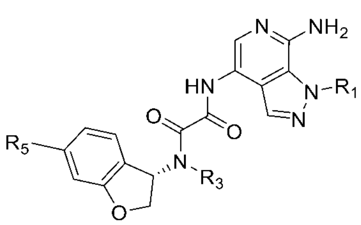 (VI-1D) or 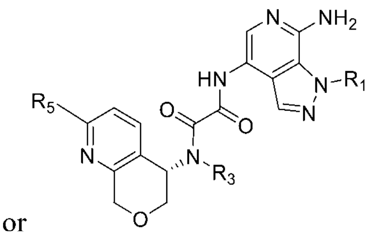 (VII-2D).

In some embodiments, the compound is

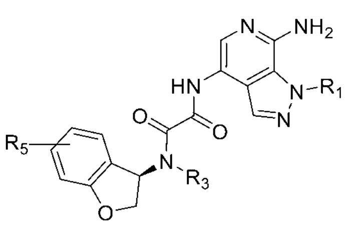 (VI-1E) or 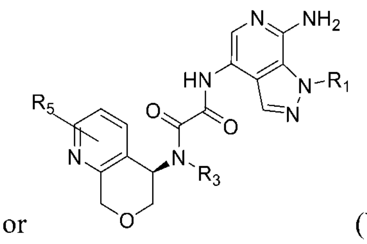 (VII-2E).

In some embodiments, the compound is

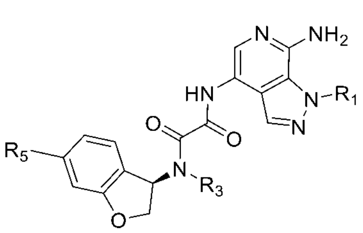 (VI-1F) or 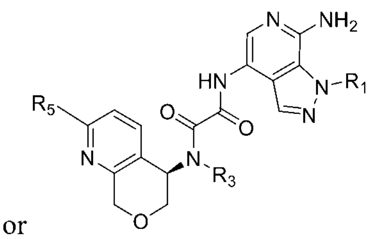 (VII-2F).

In some embodiments, $R_1$ is selected from methyl or $CD_3$.

In some embodiments, $R_3$ is selected from H, $C_{1\text{-}6}$ alkyl, deuterated $C_{1\text{-}6}$ alkyl or $C_{3\text{-}6}$ cycloalkyl. In some embodiments, $R_3$ is selected from $C_{1\text{-}6}$ alkyl, deuterated $C_{1\text{-}6}$ alkyl or $C_{3\text{-}6}$ cycloalkyl. In some embodiments, $R_3$ is selected from H, methyl, $CD_3$, ethyl or propyl. In some embodiments, $R_3$ is selected from methyl, $CD_3$, ethyl or propyl.

In some embodiments, $R_5$ is selected from hydrogen, halogen, $C_{1\text{-}6}$ alkyl, $C_{1\text{-}6}$ alkoxy, $C_{1\text{-}6}$ haloalkyl, $C_{1\text{-}6}$ haloalkoxy, CN, $SF_5$, $C_{3\text{-}6}$ cycloalkyl, $-S(O)_2CF_3$, $-OCF_3$, $-SCF_3$, $-SCH_3$ or pyridyl (e.g., pyridin-2-yl, pyridin-3-yl or pyridin-4-yl); the pyridyl is optionally further substituted by $R_6$, $R_6$ being selected from H, CN, halogen, $C_{1\text{-}6}$ alkyl, $C_{1\text{-}6}$ haloalkyl or $SF_5$. In some embodiments, $R_5$ is selected from hydrogen, F, Cl, Br, methyl, $CD_3$, ethyl, methoxy, trifluoromethyl, difluoromethyl, CN, $SF_5$, cyclopropyl-$S(O)_2CF_3$, $-OCF_3$, $-SCF_3$, $-SCH_3$ or pyridyl (e.g., pyridin-2-yl, pyridin-3-yl or pyridin-4-yl), the pyridyl being optionally substituted by halogen.

In some embodiments, Z is selected from $CH_2$ or NH.

In a more specific embodiment, the present invention provides a compound, or a tautomer, stereoisomer or pharmaceutically acceptable salt thereof, wherein the compound is selected from:

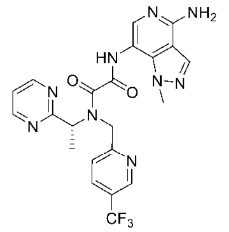, 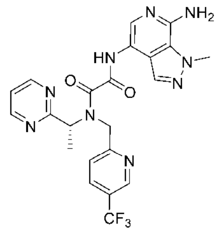, 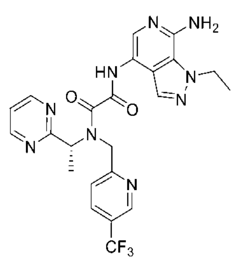, 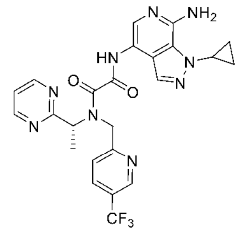, 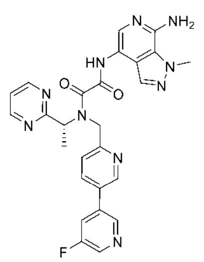,

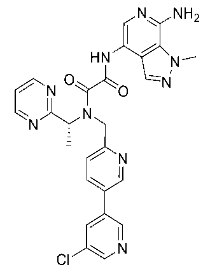,
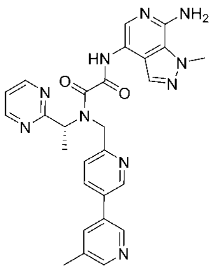,
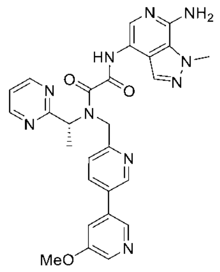,
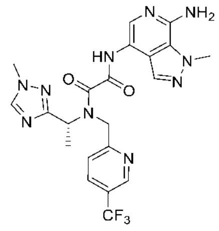,
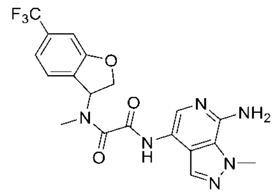,

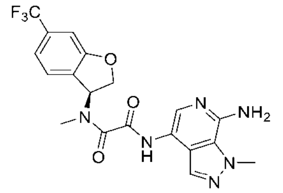,
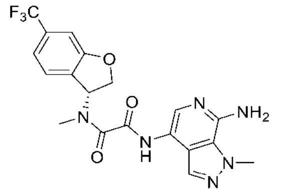,
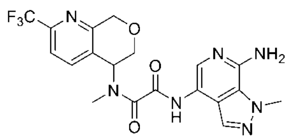,
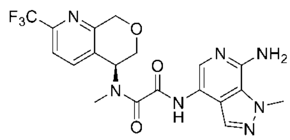,

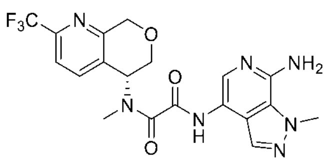,
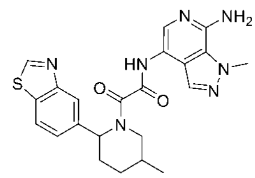,
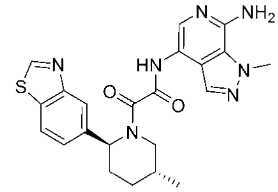,
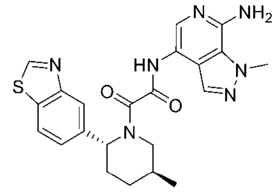,

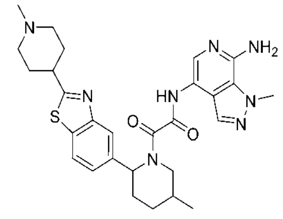,
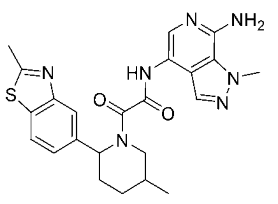,
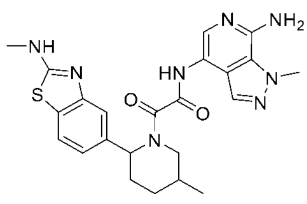,
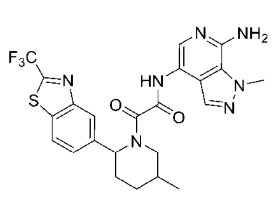,

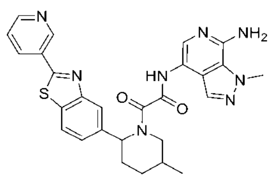,
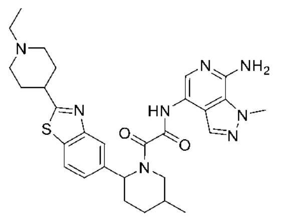,
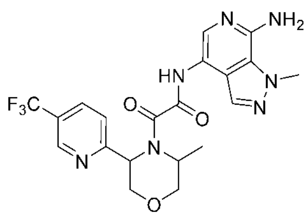,
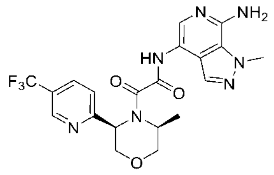,

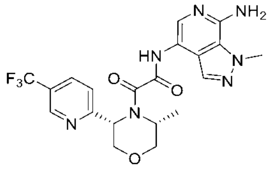,
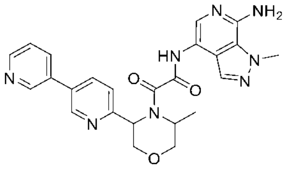,
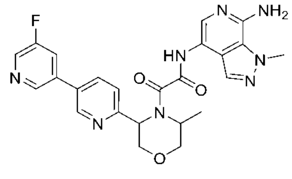,
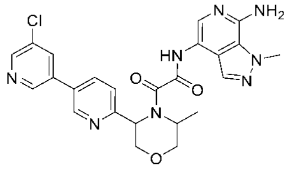,

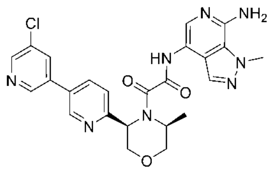,
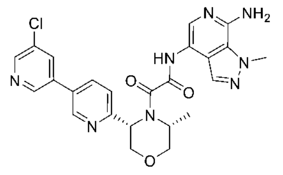,
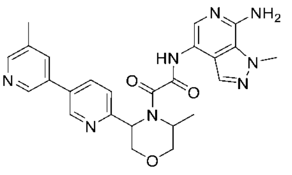,
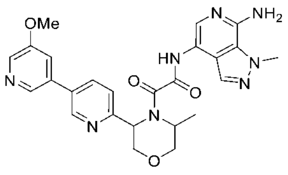,

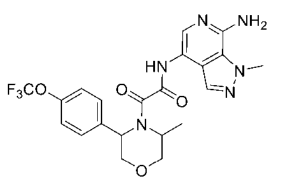 ,
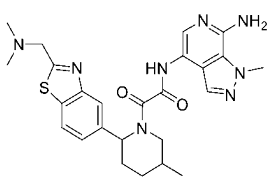 ,
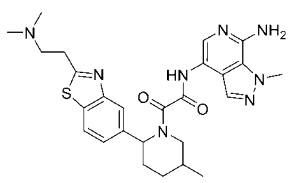 ,
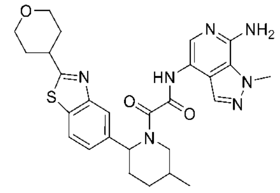 ,

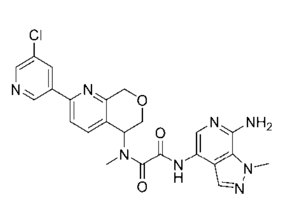 ,
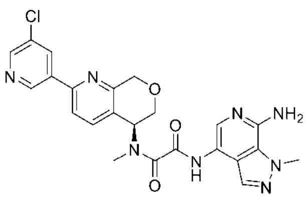 ,
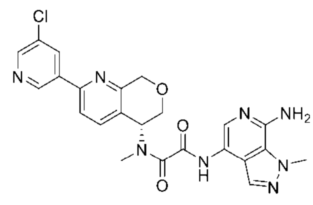 ,
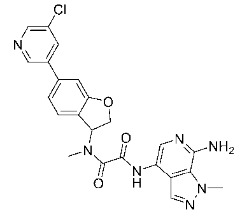 ,

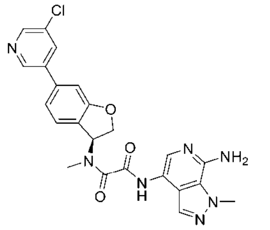 ,
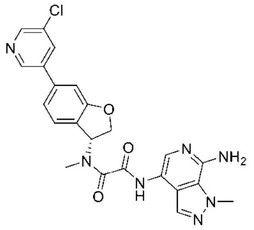 ,
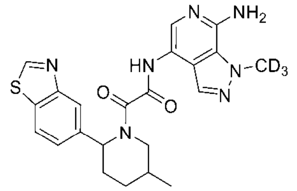 ,
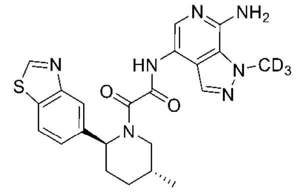 ,

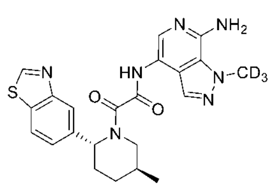 ,
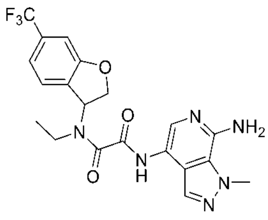 ,
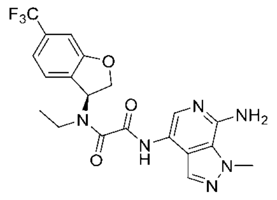 ,
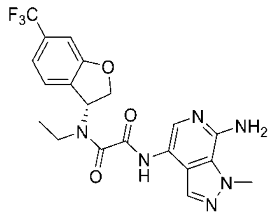 ,

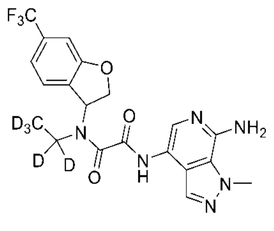 ,
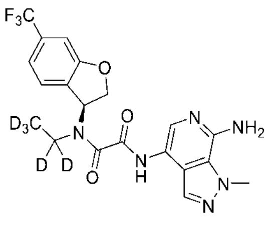 ,
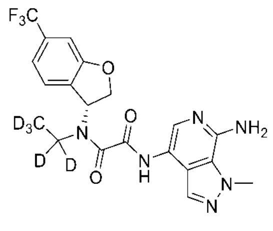 ,
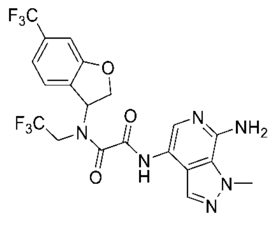 ,

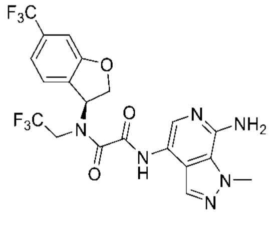 ,
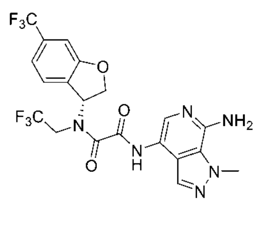 ,
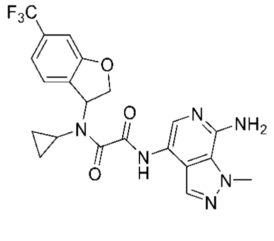 ,
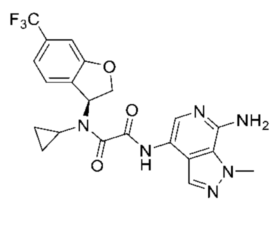 ,

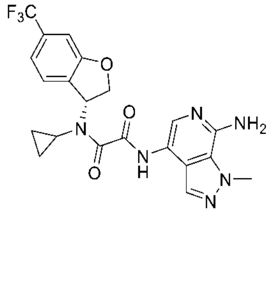 ,
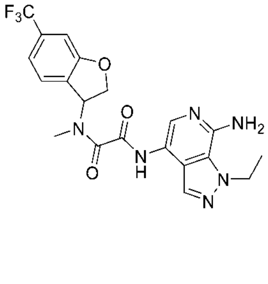 ,
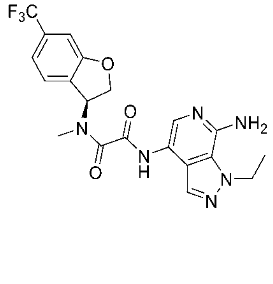 ,
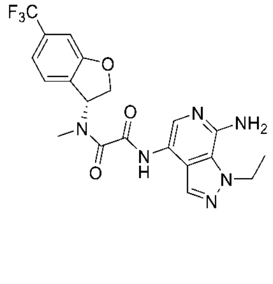 ,

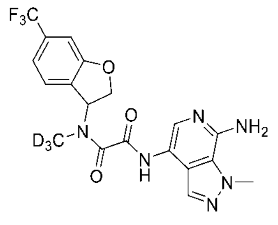 , 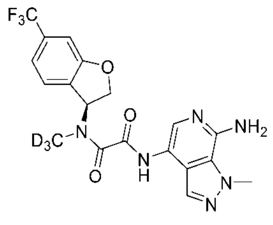 , 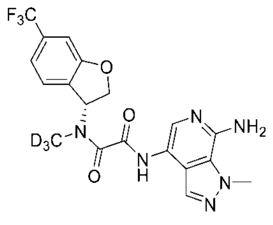 , 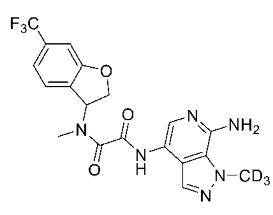 ,

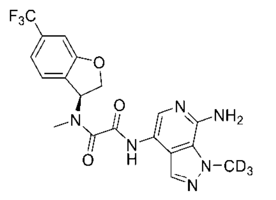 , 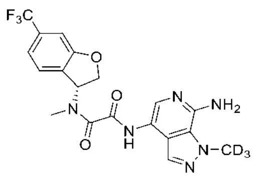 , 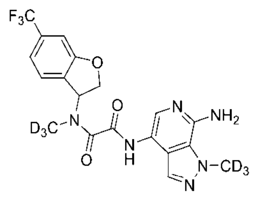 , 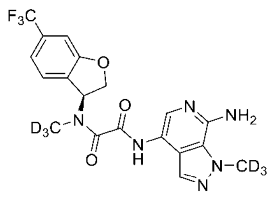 ,

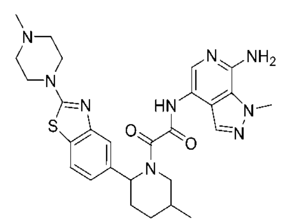 , 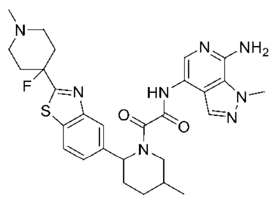 , 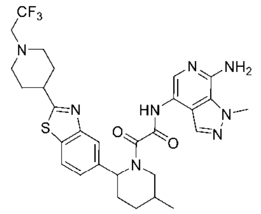 , 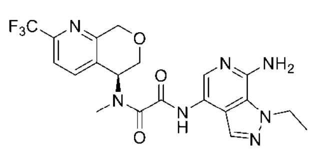 ,

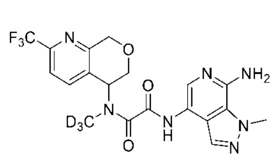 , 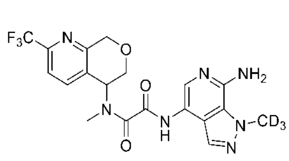 , 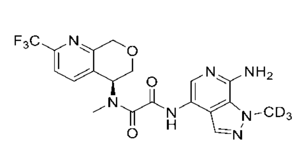 , 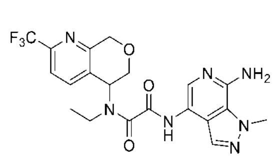 ,

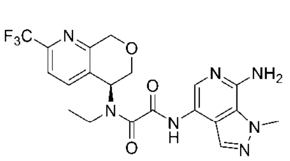 , 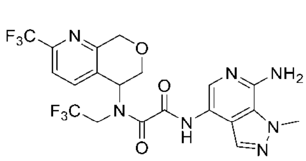 , 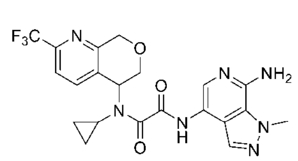 , 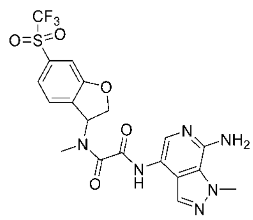 ,

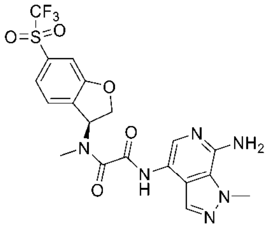 , 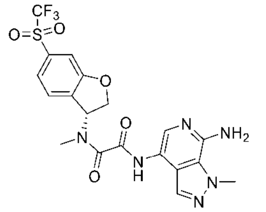 , 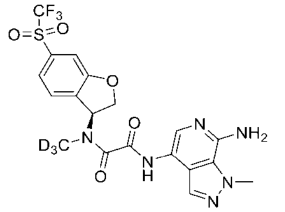 , 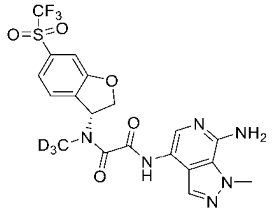 ,

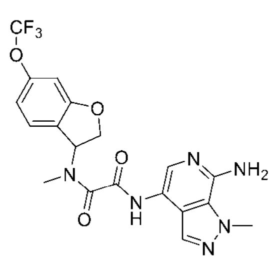 , 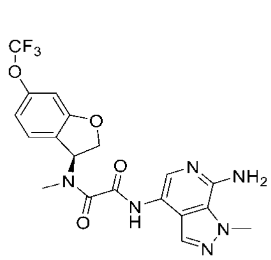 , 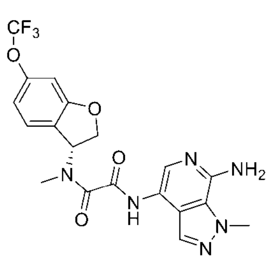 , 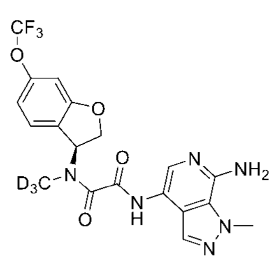 ,

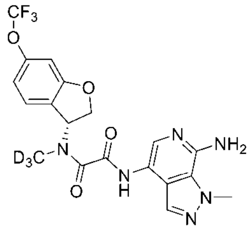,
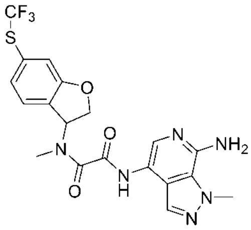,
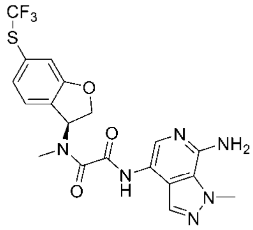,
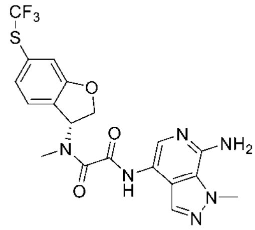,
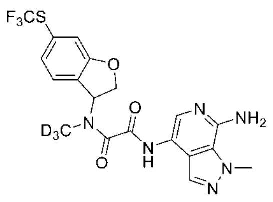,
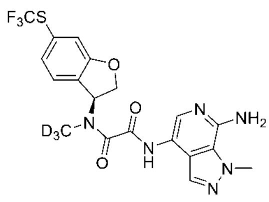,
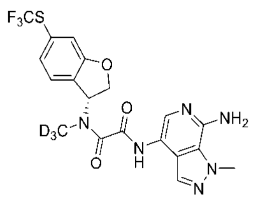,
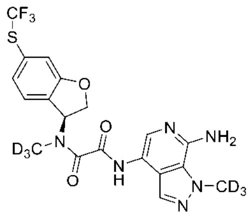,
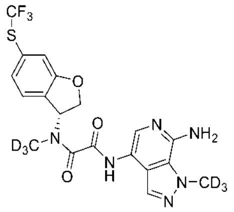,
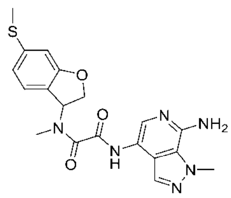,
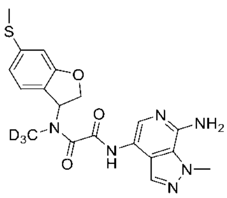,
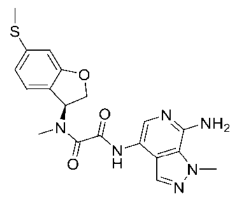,
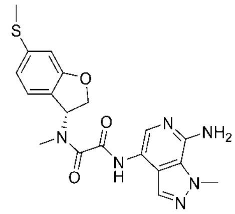,
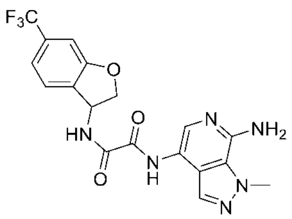,
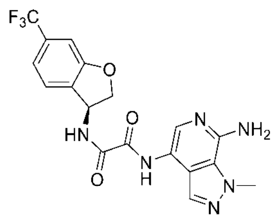,
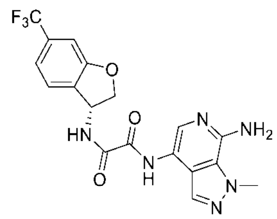,
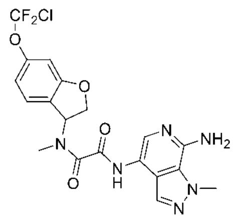

or

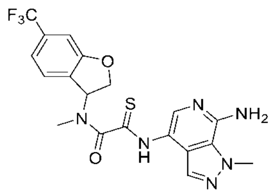.

The compounds of the present invention may include one or more asymmetric centers and may therefore exist in a variety of stereoisomeric forms, for example, enantiomers and/or diastereomeric forms. For example, the compounds of the present invention may be individual enantiomers, diastereomers or geometric isomers (e.g., cis and trans isomers), or may be in the form of a mixture of stereoisomers, including racemic mixtures and mixtures enriched in one or more stereoisomers. Isomers may be separated from the mixture by methods known to those skilled in the art, including chiral high pressure liquid chromatography (HPLC) and the formation and crystallization of chiral salts; or preferred isomers may be prepared by asymmetric synthesis.

The compounds of the invention may also exist as tautomers. For compounds that exist in different tautomeric forms, a compound is not limited to any specific tautomer, but is intended to encompass all tautomeric forms.

The compounds of the present invention can be in amorphous or crystalline form (polymorph). In addition, the compounds of the present invention can exist in one or more crystalline forms. Therefore, the present invention includes all amorphous or crystalline forms of the compounds of the present invention within its scope. The term "polymorph" refers to the crystalline form (or its salt, hydrate or solvate) of a compound with a specific crystal packing arrangement. All polymorphs have the same elemental composition. Different crystalline forms usually have different X-ray diffraction patterns, infrared spectra, melting points, densities, hardnesses, crystal shapes, photoelectric properties, stability and

solubility. Recrystallization solvents, crystallization rates, storage temperatures and other factors can lead to a dominant crystalline form. The various polymorphs of a compound can be prepared by crystallization under different conditions.

The present invention also includes isotope-labeled compounds (isotopic variants), which are equivalent to those described in formula (A), but with one or more atoms replaced by atoms whose atomic mass or mass number is different from the atomic mass or mass number common in nature. Examples of isotopes that can be introduced into the compounds of the present invention include isotopes of hydrogen, carbon, nitrogen, oxygen, phosphorus, sulfur, fluorine and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{36}Cl$, respectively. Compounds of the present invention containing other isotopes of the above-mentioned isotopes and/or other atoms, their prodrugs, and pharmaceutically acceptable salts of the compounds or the prodrugs all fall within the scope of the present invention. Certain isotope-labeled compounds of the present invention, such as those that introduce radioactive isotopes (e.g., $^{3}H$ and $^{14}C$), can be used for drug and/or substrate tissue distribution determinations. Tritium, i.e. $^{3}H$ and carbon-14, i.e. $^{14}C$ isotopes are particularly preferred because they are easy to prepare and detect. Furthermore, substitution with heavier isotopes, such as deuterium, i.e., $^{2}H$, may be preferred in some cases due to greater metabolic stability, and may provide therapeutic benefits, such as increased half-life *in vivo* or reduced dosage requirements. Isotope-labeled compounds of formula (A) of the present invention and their prodrugs can generally be prepared by replacing non-isotope-labeled reagents with readily available isotope-labeled reagents when performing the processes disclosed in the following schemes and/or examples and preparation examples.

*Pharmaceutical Compositions and Kits*

**In** another aspect, the present invention provides a pharmaceutical composition comprising a compound of the present invention (also referred to as an "active ingredient") and a pharmaceutically acceptable excipient. **In** some embodiments, the pharmaceutical composition comprises an effective amount of a compound of the present invention. **In** some embodiments, the pharmaceutical composition comprises a therapeutically effective amount of a compound of the present invention. **In** some embodiments, the pharmaceutical composition comprises a prophylactically effective amount of a compound of the present invention.

The pharmaceutically acceptable excipient used in the present invention refers to a non-toxic carrier, adjuvant or vehicle that does not impair the pharmacological activity of the compound formulated together. Pharmaceutically acceptable carriers, adjuvants or vehicles that can be used in the composition of the present invention include, but are not limited to, ion exchangers, aluminum oxide, aluminum stearate, lecithin, serum proteins (e.g., human serum albumin), buffer substances (e.g., phosphates), glycine, sorbic acid, potassium sorbate, partial glyceride mixtures of saturated vegetable fatty acids, water, salts or electrolytes (e.g., protamine sulfate), disodium hydrogen phosphate, potassium hydrogen phosphate, sodium chloride, zinc salts, silica gel, magnesium trisilicate, polyvinyl pyrrolidone, cellulose-based substances, polyethylene glycol, sodium carboxymethyl cellulose, polyacrylates, waxes, polyethylene-polyoxypropylene-block polymers, polyethylene glycol and lanolin.

The present invention further comprises a kit (e.g., a pharmaceutical package). The kit provided may include a compound of the invention, other therapeutic agents, and a first and second container (e.g., a vial, an ampoule, a bottle, a syringe and/or a dispersible package or other suitable container) containing the compound of the invention and other therapeutic agents. **In** some embodiments, the kit provided may also optionally include a third container containing a pharmaceutical excipient for diluting or suspending the compound of the invention and/or other therapeutic agents. **In** some embodiments, the compound of the invention and other therapeutic agents provided in the first container and the second container are combined to form a unit dosage form.

*Administration*

Pharmaceutical compositions provided by the invention can be administered by many ways, including but not limited to: oral administration, parenteral administration, inhalation administration, topical administration, rectal administration, nasal administration, oral administration, vaginal administration, administration by implant or other modes of administration. For example, parenteral administration as used herein includes subcutaneous administration, intradermal administration, intravenous administration, intramuscular administration, intraarticular administration, intraarterial administration, intrasynovial cavity administration, intrasternal administration, intrathecal administration, intralesional administration and intracranial injection or infusion technology.

Generally, an effective amount of the compounds provided herein is administered. The amount of the compound actually administered can be determined by a physician according to the relevant circumstances, including the condition being treated, the route of administration selected, the compound actually administered, the age, weight and response of the individual patient, the severity of the patient's symptoms, and the like.

When used to prevent the conditions described herein, the compounds provided herein are administered to a subject at risk of developing the condition, typically based on the advice of a physician and under the supervision of a

physician, at dosage levels as described above. Subjects at risk of developing a specific condition typically include those with a family history of the condition, or those identified by genetic testing or screening as being particularly susceptible to developing the condition.

The pharmaceutical compositions provided herein can also be administered long-term ("chronic administration"). Chronic administration refers to administration of a compound or its pharmaceutical composition over a long period of time, e.g., 3 months, 6 months, 1 year, 2 years, 3 years, 5 years, etc., or administration may continue indefinitely, e.g., for the rest of the subject's life. In some embodiments, long-term administration is intended to provide a constant level of the compound in the blood over a long period of time, e.g., within a therapeutic window.

Various methods of administration can be used to further deliver the pharmaceutical composition of the present invention. For example, in some embodiments, the pharmaceutical composition can be administered by bolus injection, for example, in order to increase the concentration of the compound in the blood to an effective level. The bolus dose depends on the target systemic level of the active component through the body, for example, an intramuscular or subcutaneous bolus dose slowly releases the active component, whereas a bolus injection (for example, by IV intravenous drip) delivered directly to the vein can be delivered more quickly, so that the concentration of the active component in the blood is quickly increased to an effective level. In other embodiments, the pharmaceutical composition can be given in a continuous infusion form, for example, by IV intravenous drip, so as to provide a steady-state concentration of the active component in the subject's body. In addition, in other embodiments, the pharmaceutical composition may be administered first as a bolus dose, and then subsequently as a continuous infusion.

Oral compositions can be in the form of bulk liquid solutions or suspensions or bulk powders. However, more generally, in order to facilitate accurate dosing, the composition is provided in a unit dosage form. The term "unit dosage form" refers to a physical discrete unit suitable as a unit dose for human patients and other mammals, each unit containing a predetermined amount of active substances suitable for producing the desired therapeutic effect and a suitable pharmaceutical excipient. Typical unit dosage forms include pre-filled, pre-measured ampoules or syringes of liquid compositions, or pills, tablets, capsules, etc. in the case of solid compositions. In this composition, the compound is usually a relatively small component (about 0.1 to about 50% by weight, or preferably about 1 to about 40% by weight), and the remainder is various carriers or excipients and processing aids useful for forming the desired administration form.

For oral dosage, a representative regimen is one to five oral doses per day, especially two to four oral doses, typically three oral doses. Using these dosage modes, each dose provides about 0.01 to about 20 mg/kg of the compound of the invention, and preferred doses each provide about 0.1 to about 10 mg/kg, especially about 1 to about 5 mg/kg.

In order to provide blood levels similar to those using an injectable dose, or lower blood levels than using an injectable dose, a transdermal dose is generally selected in an amount of about 0.01 to about 20% by weight, preferably about 0.1 to about 20% by weight, preferably about 0.1 to about 10% by weight, and more preferably about 0.5 to about 15% by weight.

From about 1 to about 120 hours, especially 24 to 96 hours, the injected dosage level is in the range of about 0.1 mg/kg/hour to at least 10 mg/kg/hour. To achieve adequate steady state levels, a preload bolus of about 0.1 mg/kg to about 10 mg/kg or more may also be administered. For a 40 to 80 kg human patient, the maximum total dose should not exceed about 2 g/d.

Liquid forms suitable for oral administration may include a suitable aqueous or non-aqueous carrier and buffers, suspending and dispersing agents, colorants, flavoring agents, etc. Solid forms may include, for example, any of the following components, or compounds of a similar nature: binders, e.g., microcrystalline cellulose, tragacanth, or gelatin; excipients, e.g., starch or lactose, disintegrants, e.g., alginic acid, Primojel, or corn starch; lubricants, e.g., magnesium stearate; glidants, e.g., colloidal silicon dioxide; sweeteners, e.g., sucrose or saccharin; or flavoring agents, e.g., peppermint, methyl salicylate, or orange flavoring.

Injectable compositions are typically based on sterile saline or phosphate buffered saline for injection, or other injectable excipients known in the art. As previously mentioned, in such compositions, the active compound is typically a minor component, often about 0.05 to 10% by weight, with the remainder being injectable excipients, etc.

Typically, transdermal compositions are formulated as topical ointments or creams containing active ingredients. When formulated as an ointment, the active ingredient is typically combined with a paraffin or water-miscible ointment base. Alternatively, the active ingredient can be formulated as an ointment together with, for example, an oil-in-water cream base. Such transdermal formulations are well known in the art and typically include other components for enhancing the stable skin penetration of the active ingredient or formulation. All such known transdermal formulations and components are included within the scope provided by the invention.

The compounds of the invention may also be administered by transdermal means. Therefore, transdermal administration may be achieved using patches of the reservoir or porous membrane type, or a variety of solid matrices.

The above components for oral administration, injection or topical administration of the composition are representative only. Other materials and processing techniques are described in Remington's Pharmaceutical Sciences, 17th edition, 1985, Mack Publishing Company, Easton, Pennsylvania, Part 8, which is incorporated herein by reference.

The compounds of the invention may also be administered in sustained release form or from a sustained release

delivery system. A description of representative sustained release materials can be found in Remington's Pharmaceutical Sciences.

The present invention also relates to pharmaceutically acceptable formulations of the compounds of the present invention. In one embodiment, the formulation comprises water. In another embodiment, the formulation comprises a cyclodextrin derivative. The most common cyclodextrins are α-, β- and γ-cyclodextrins consisting of 6, 7 and 8 α-1,4-linked glucose units, respectively, which optionally include one or more substituents on the linked sugar moiety, including but not limited to: methylated, hydroxyalkylated, acylated and sulfoalkyl ether substitutions. In some embodiments, the cyclodextrin is a sulfoalkyl ether β-cyclodextrin, for example, sulfobutyl ether β-cyclodextrin, also known as Captisol. See, for example, US5,376,645. In some embodiments, the formulation comprises hexapropyl-β-cyclodextrin (e.g., in water, 10-50%).

## Examples

The reagents used in the present invention are commercial reagents purchased directly or synthesized by common methods well known in the art.

Notes on commonly used abbreviations:

Abbreviations: PE = petroleum ether; EA = ethyl acetate; MeOH = methanol; DCM = dichloromethane; DCE = dichloroethane; $CH_3CN$ = acetonitrile; 1,4-dioxane = 1,4-dioxane; DMSO = dimethyl sulfoxide; HFIP = hexafluoroisopropanol; DMF = N,N-dimethylformamide; Hex = n-hexane; IPA = isopropanol; NMP = N-methylpyrrolidone; NMO = N-methylmorpholine-N-oxide; TEA = triethylamine; DIEA = diisopropylethylamine; CuI = cuprous iodide; CuCN = cuprous cyanide; triphosgene = triphosgene; p-TsOH = para-toluenesulfonic acid; $T_3P$ = 1-propylphosphonic acid cyclic anhydride; $TsN_3$ = para-toluenesulfonyl azide; PPA = polyphosphoric acid; BINAP = 1,1'-binaphthyl-2,2'-bis(diphenylphosphine); m-CPBA = meta-chloroperbenzoic acid; Hex = n-hexane.

*MTAPwt* = methylthioadenosine phosphorylase wild type;
*MTAPdel* = methylthioadenosine phosphorylase deficiency;
MTA = methylthioadenosine;
SAM = adenosylmethionine.

The specific reaction routes or steps exemplified below are used in the present invention, and are as follows:

### Example 1

**Preparation** of key intermediates al-a43

Synthesis of intermediates al, a7-a8, a27

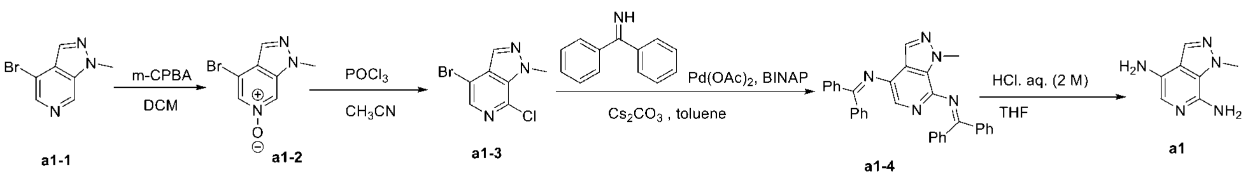

a1-1 a1-2 a1-3 a1-4 a1

Step 1: Starting material 4-bromo-1-pyrazolo[3,4-c]pyridine al-1 (1.1 g, 5.2 mmol) was dissolved in 20 mL of dichloromethane. Oxidant m-chloroperbenzoic acid (*m*-CPBA) (1.3 g, 7.8 mmol) was slowly added, reacting at room temperature for 3 hours before stopping the reaction, filtering, washing the filter cake with dichloromethane, and drying to obtain a white solid **a1-2** (1.0 g) in a yield of 84%.

Step 2: In an ice bath, the intermediate **a1-2** (1.0 g, 4.4 mmol) was dissolved in 10 mL of acetonitrile, and $POCl_3$ (1.0 mL) was slowly added dropwise. The mixture was reacted in an ice bath for 1 hour, before stopping the reaction. The solvent was evaporated under reduced pressure, 50 mL of ice water was added to the system, and the pH was adjusted to about 8 with a saturated aqueous solution of sodium bicarbonate. Following extraction with dichloromethane, drying over anhydrous sodium sulfate, and concentrating, the crude product was separated by reversed-phase flash column chromatography (C18, $CH_3CN/H_2O$, 4/5) to obtain a white solid **al-3** (900 mg) in a yield of 83%. LCMS ESI-MS m/z:

246 $[M+H]^+$.

Step 3: Under nitrogen protection, the intermediate from the previous step al-3(900 mg, 3.65 mmol), benzophenone imine (2.6 g, 14.6 mmol) and cesium carbonate (4.8 g, 14.6 mmol) were dissolved in 27 mL of anhydrous toluene, and catalyst $Pd(OAc)_2$ (164 mg, 0.73 mmol) and ligand BINAP (900 mg, 1.46 mmol) were added. The mixture was heated to 140°C and reacted for 7 hours, before stopping the reaction, filtering, and evaporating the solvent under reduced pressure. The crude product was directly separated by reversed-phase flash column chromatography (C18, $CH_3CN/H_2O$, 4/5) to obtain a white solid al-4 (400 mg) in a yield of 22%. LCMS ESI-MS m/z: 492 $[M+H]^+$.

Step 4: The intermediate **a1-4** (400 mg, 0.81 mmol) was dissolved in 2 mL tetrahydrofuran. 8 mL dilute hydrochloric acid (concentration: 2M) was added to react at room temperature for 3 hours, before stopping the reaction. A saturated aqueous solution of sodium bicarbonate was added to the reaction solution to adjust the pH to about 8, before extracting with ethyl acetate, drying over anhydrous sodium sulfate, concentrating, and separating the crude product by reversed-phase flash column chromatography (C18, $CH_3CN/H_2O$, 1/3) to obtain the target intermediate a1 (100 mg) in a yield of 75%. LCMS ESI-MS m/z: 164 $[M+H]^+$.

Similar starting materials/intermediates were used to synthesize the following target intermediates by analogy to the synthetic route of compound **a1.**

| Target intermediate | Intermediate structure | LC-MS $[M+H]^+$ |
|---|---|---|
| **a7** | 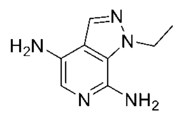 | 178 |
| **a8** | 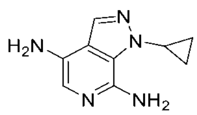 | 190 |
| **a27** | 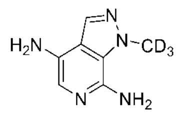 | 167 |

Synthesis of intermediates **a2-a5, a9-a26, a28-a32, a35-a37, a40-a42:**

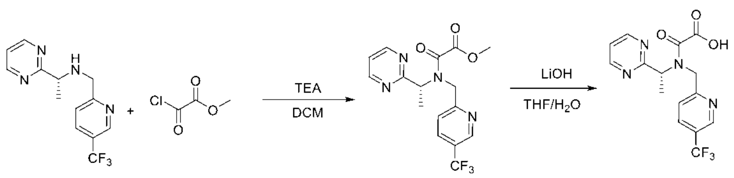


e1 a2-1 a2

Step 1: The intermediate **e1** (200 mg, 0.71 mmol) and triethylamine (143 mg, 1.42 mmol) were dissolved in 4 mL of dichloromethane. Methyl oxalyl chloride (134 mg, 1.1 mmol) was added to react at room temperature for 1 hour, before evaporating the solvent under reduced pressure to obtain a crude compound **a2-1,** which was directly used for the next step.

Step 2: The crude compound **a2-1** (30 mg, 0.05 mmol) was dissolved in a mixed solution of 6 mL tetrahydrofuran and water (v/v, 1/1). LiOH (65 mg, 1.62 mmol) was added and allowed to react at room temperature for 4 hours. Dilute hydrochloric acid (concentration: 4M) was added slowly to the system to adjust the pH to about 6, before evaporating the solvent under reduced pressure and separating the crude product by flash column chromatography (C18, $CH_3CN/H_2O$, 1/1) to obtain compound **a2** (110 mg) in an overall yield of 43% over the two steps. LCMS ESI-MS m/z: 355 $[M+H]^+$.

Similar starting materials/intermediates were used to synthesize the following target intermediates by analogy to the synthetic route of compound **a2** or intermediate **a2-1.**

| Target intermediate | Intermediate structure | LC-MS [M+H]+ |
|---|---|---|
| **a3** | 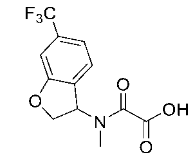 | 290 |
| **a4** | 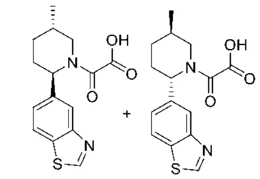 | 305 |
| **a5** | 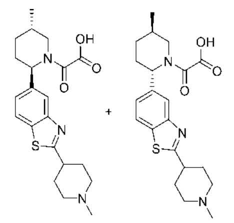 | 402 |
| **a9** | 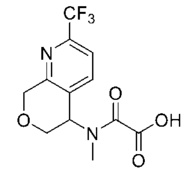 | 305 |
| **a10** | 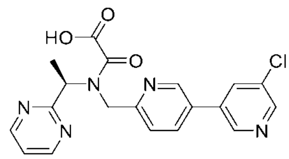 | 398 |
| **a11** | 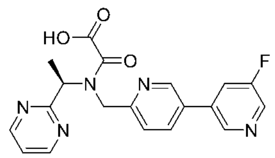 | 382 |
| **a12** | 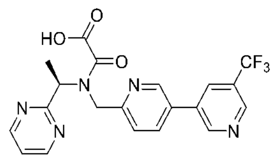 | 432 |
| **a13** | 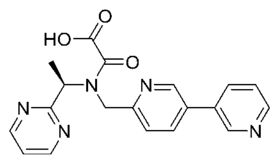 | 364 |
| **a14** | 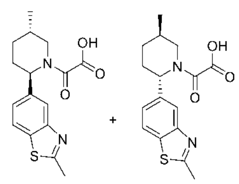 | 319 |

(continued)

| Target intermediate | Intermediate structure | LC-MS [M+H]+ |
|---|---|---|
| **a15** | 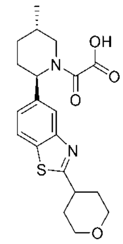 + 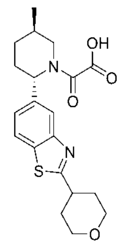 | 389 |
| **a16** | 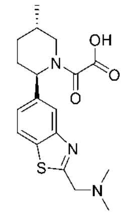 + 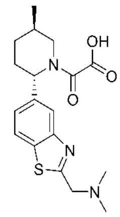 | 362 |
| **a17** | 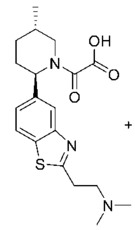 + 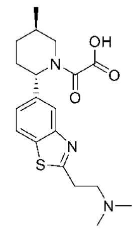 | 376 |
| **a18** | 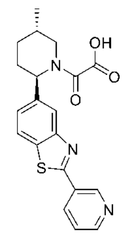 + 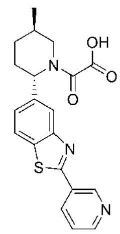 | 382 |
| **a19** | 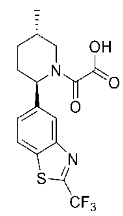 + 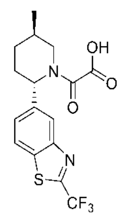 | 373 |
| **a20** | 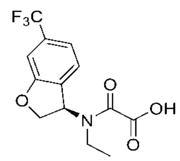 | 304 |
| **a21** | 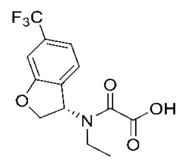 | 304 |

(continued)

| Target intermediate | Intermediate structure | LC-MS [M+H]+ |
|---|---|---|
| **a22** | 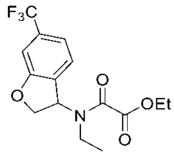 | 332 |
| **a23** | 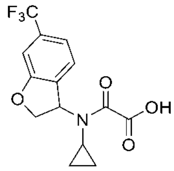 | 316 |
| **a24** | 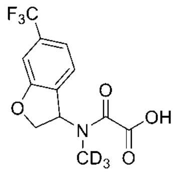 | 293 |
| **a25** | 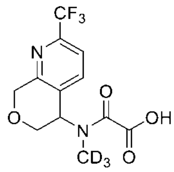 | 308 |
| **a26** | 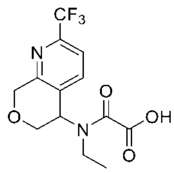 | 319 |
| **a28** | 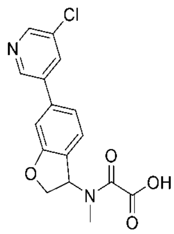 | 333 |
| **a29** | 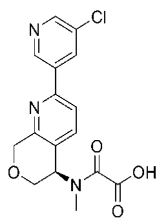 | 348 |
| **a30** | 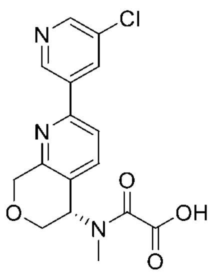 | 348 |

(continued)

| Target intermediate | Intermediate structure | LC-MS [M+H]+ |
|---|---|---|
| **a31** | 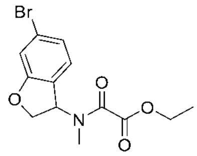 | 328 |
| **a32** | 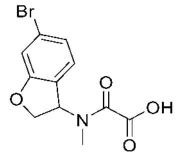 | 300 |
| **a35** | 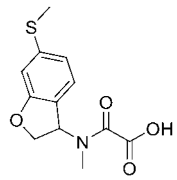 | 268 |
| **a36** | 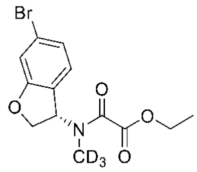 | 331 |
| **a37** | 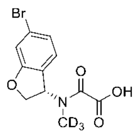 | 303 |
| **a40** | 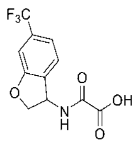 | 276 |
| **a41** | 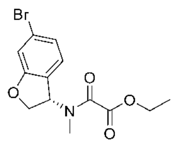 | 328 |
| **a42** | 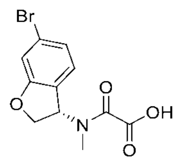 | 300 |

Synthesis of intermediate **a6:**

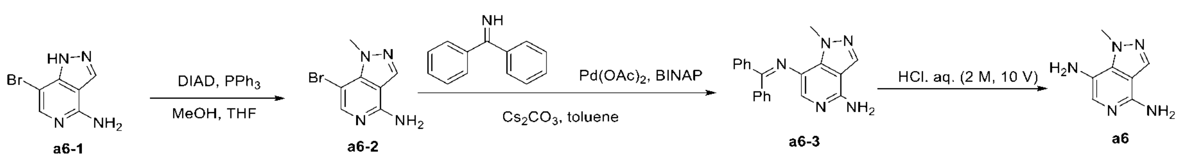


a6-1 a6-2 a6-3 a6

Step 1: In an ice bath, under nitrogen protection, starting material **a6-1** (800 mg, 3.75 mmol) and methanol (183 mg, 5.63 mmol) were dissolved in 16 mL of anhydrous tetrahydrofuran. Diisopropyl azodicarboxylate (DIAD) (134 mg, 1.1 mmol) and $PPh_3$ (1.18 g, 4.50 mmol) were added, reacting at room temperature for 1 hour. A solid was precipitated out and filtered. The filtrate was concentrated under reduced pressure to obtain a white solid **a6-2** (290 mg) in a yield of 34%. LCMS ESI-MS m/z: 227 $[M+H]^+$.

Step 2: Under nitrogen protection, the intermediate from the previous step **a6-2** (290 mg, 1.27 mmol), benzophenone imine (347 mg, 1.91 mmol) and cesium carbonate (1.7 g, 5.2 mmol) were dissolved in 9 mL of anhydrous toluene, before adding catalyst $Pd(OAc)_2$ (29 mg, 0.12 mmol) and ligand BINAP (159 mg, 0.25 mmol). The mixture was heated to 140°C and reacted for 48 hours before stopping the reaction, filtering, and evaporating the solvent under reduced pressure to obtain a crude product **a6-3.** LCMS ESI-MS m/z: 328 $[M+H]^+$.

Step 3: The crude product **a6-3** (230 mg, 0.70 mmol) was dissolved in 5 mL tetrahydrofuran. 3 mL dilute hydrochloric acid (concentration: 2M) was added and allowed to react at room temperature for 2 hours, before stopping the reaction. A saturated aqueous solution of sodium bicarbonate was added to the reaction solution to adjust the pH to about 8, before extracting with ethyl acetate, drying over anhydrous sodium sulfate, concentrating, and separating the crude product by reversed-phase flash column chromatography (C18, $CH_3CN/H_2O$, 3/5) to obtain the target intermediate **a6** (70 mg) in a yield of 61%. LCMS ESI-MS m/z: 164 $[M+H]^+$.

Synthesis of intermediates **a33, a34, a38, a43:**

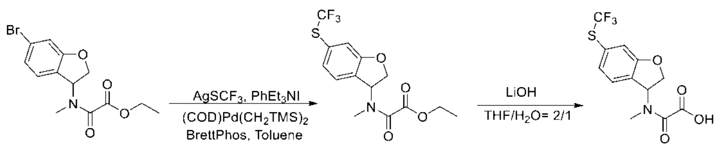


a31 a33 a34

Step 1: Under nitrogen protection, the intermediate **a31(120** mg, 0.36 mmol), ligand BrettPhos (39.2 mg, 0.07 mmol) and catalyst $Pd(COD)(CH_2TMS)_2$ (28.4 mg, 0.07 mmol) were dissolved in 3 mL of anhydrous toluene. The mixture was heated to 80°C and stirred for 10 minutes. $AgSCF_3$ (99 mg, 0.47 mmol) and triethylphenylammonium iodide $PhEt_3NI$ (145 mg, 0.47 mmol) were added to the reaction solution, and the reaction allowed to continue at 80°C for 2 hours before stopping the reaction, filtering, and evaporating the solvent under reduced pressure. The crude product was separated by reversed-phase flash column chromatography (C18, $CH_3CN/H_2O$, 9/10) to obtain a brown oil **a33** (100 mg) in a yield of 78%. LCMS ESI-MS m/z: 350 $[M+H]^+$.

Step 2: The intermediate **a33** (100 mg, 0.28 mmol) was dissolved in a mixed solution of 2 mL tetrahydrofuran and water (v/v, 1/1). Aqueous LiOH (0.5 mL, 1N) was added dropwise and allowed to react at room temperature for 1 hour, before stopping the reaction. Dilute hydrochloric acid was added to the reaction solution to adjust the pH to about 5, before evaporating the solvent under reduced pressure and separating the crude product by reversed-phase flash column chromatography (C18, $CH_3CN/H_2O$, 1/1) to obtain the target intermediate **a34** (50 mg) in a yield of 54%. LCMS ESI-MS m/z: 322 $[M+H]^+$.

Similar starting materials/intermediates were used to synthesize the following target intermediates by analogy to the synthetic route of compound **a34.**

| Target intermediate | Intermediate structure | LC-MS $[M+H]^+$ |
|---|---|---|
| **a38** | 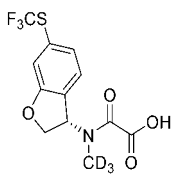 | 325 |
| **a43** | 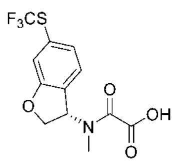 | 322 |

Synthesis of intermediate **a39:**

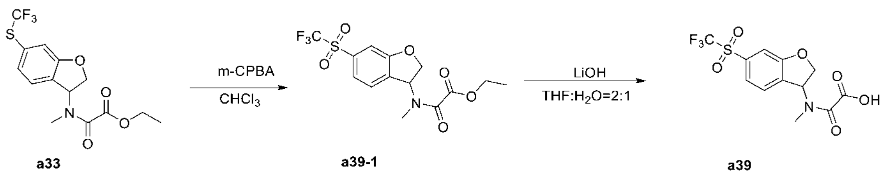


a33 a39-1 a39

Step 1: In an ice bath, the intermediate **a33** (360 mg, 1.03 mmol) was dissolved in 8 mL of chloroform. After stirring for 5 minutes, m-CPBA (531 mg, 3.09 mmol) was added to the reaction solution. The mixture was heated to 60°C to react for 12 hours before stropping the reaction and filtering. 10 mL of saturated sodium thiosulfate aqueous solution was added to the reaction solution, before extracting with dichloromethane, drying over anhydrous sodium sulfate, and evaporating the solvent under reduced pressure to obtain a brown oil **a39-1** (480 mg) in a yield of 90%. LCMS ESI-MS m/z: 382 $[M+H]^+$.

Step 2: In an ice bath, the intermediate **a39-1** (480 mg, 0.92 mmol) was dissolved in 7 mL of tetrahydrofuran. After stirring for 5 minutes, LiOH aqueous solution (1.0 mL, 2N) was added to the reaction solution. The mixture was reacted at room temperature for 1 hour, before stopping the reaction and evaporating the solvent under reduced pressure. Dilute hydrochloric acid was then added to the reaction solution to adjust the pH to about 5, before extracting with ethyl acetate, drying over anhydrous sodium sulfate, and concentrating. The crude product was separated by reversed-phase flash column chromatography (C18, $CH_3CN/H_2O$, 1/1) to obtain a white solid **a39** (190 mg) in a yield of 58%. LCMS ESI-MS m/z: 354 $[M+H]^+$.

**Preparation of key intermediates b1-b5**

Synthesis of intermediates **b1, b2, b4-b9**

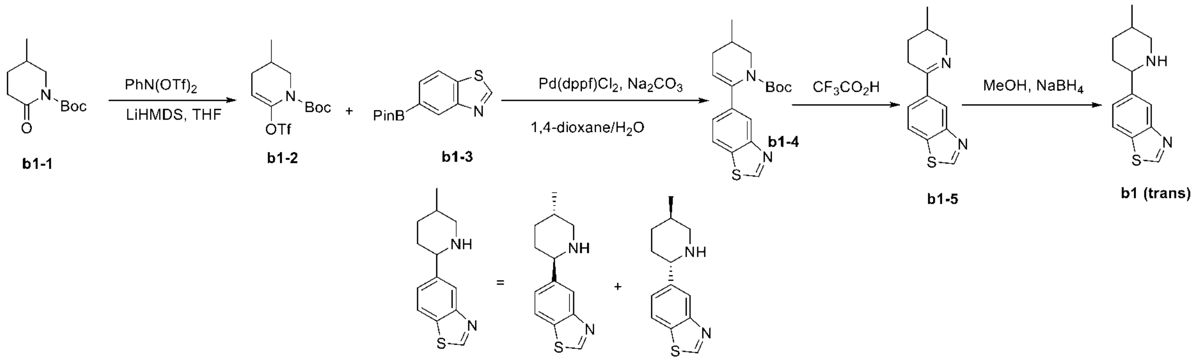


b1-1 b1-2 b1-3 b1-4 b1-5 b1 (trans)

b1 (trans)

First step: At -78°C, under nitrogen protection, starting material **b1-1** (8.8 g, 41.3 mmol) was dissolved in 88 mL of anhydrous tetrahydrofuran, and a tetrahydrofuran solution of LiHMDS (10.4 g, 61.9 mmol, 61.9 mL) was slowly added dropwise. Upon completion of the addition, the reaction solution was stirred at -78°C for 1.5 hours. N-phenylbis(trifluoromethanesulfonyl)imide (20.1 g, 51.6 mmol) was added to the reaction solution, which was heated to room temperature and allowed to react for 2 hours before stopping the reaction. 50 mL of ice water was added to the reaction solution, before extracting with methyl tert-butyl ether, drying over anhydrous sodium sulfate, concentrating, and separating the crude product by reversed-phase flash column chromatography (C18, $CH_3CN$) to obtain a red oil **b1-2** (10.1 g) in a yield of 71%. LCMS ESI-MS m/z: 346 $[M+H]^+$.

Step 2: Under nitrogen protection, the oil from the previous step **b1-2** (2.1 g, 6.08 mmol) was dissolved together with 5-boronic acid pinacol ester-1,3-benzothiazole **b1-3** (1.1 g, 7.62 mmol) and sodium carbonate (1.9 g, 18.2 mmol) in 40 mL of a mixed solution of 1,4-dioxane and water (v/v, 3/1), and a catalyst $Pd(dppf)Cl_2$ (220 mg, 0.30 mmol) was added. The mixture was heated to 80°C and reacted for 2 hours before stopping the reaction and filtering. To the reaction solution was added 100 mL of water, before extracting with ethyl acetate, drying over anhydrous sodium sulfate, and concentrating to obtain a white solid **b1-3** (2.7 g) in a yield of 96%. LCMS ESI-MS m/z: 331 $[M+H]^+$.

Step 3: The white solid **b1-3** (2.7 g, 8.17 mmol) was dissolved in 15 mL trifluoroacetic acid and allowed to react at

room temperature for 1 hour before stopping the reaction. The solvent was evaporated under reduced pressure, and a saturated aqueous solution of sodium bicarbonate added to the reaction solution to adjust the pH to about 8, before extracting with ethyl acetate, drying over anhydrous sodium sulfate, and concentrating to obtain a yellow oil **b1-4** (1.8 g) in a yield of 96%. LCMS ESI-MS m/z: 231 [M+H]$^+$.

Step 4: In an ice bath, the oil **b1-4** (1.0 g, 4.34 mmol) was dissolved in 20 mL methanol. Reducing agent $NaBH_4$ (250 mg, 6.51 mmol) was added, reacting in the ice bath for 1 hour before stropping the reaction. To the reaction solution was added 100 mL of ice water, before extracting with ethyl acetate, drying with anhydrous sodium sulfate, concentrating, and separating the crude product by reversed-phase flash column chromatography (C18, $CH_3CN/H_2O$, 3/5) to obtain a yellow oil **b1** (370 mg) in a yield of 37%. LCMS ESI-MS m/z: 233 [M+H]$^+$.

Similar starting materials/intermediates were used to synthesize the following target intermediates by analogy to the synthetic route of compound **b1.**

| Target intermediate | Intermediate structure | LC-MS [M+H]$^+$ |
|---|---|---|
| **b2 (trans)** | 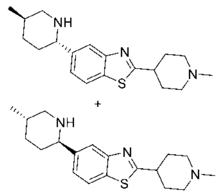 + | 330 |
| **b4 (trans)** | 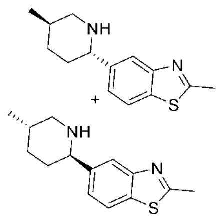 + | 247 |
| **b5 (trans)** | 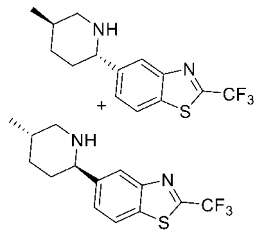 + | 301 |
| **b6 (trans)** | 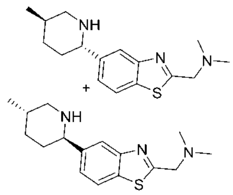 + | 290 |
| **b7 (trans)** | 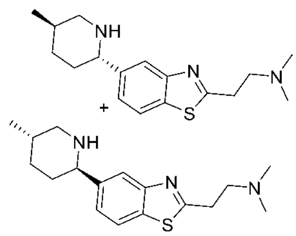 + | 304 |
| **b8 (trans)** | 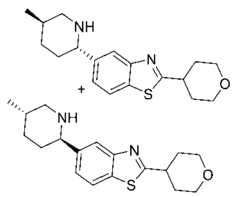 + | 317 |

(continued)

| Target intermediate | Intermediate structure | LC-MS [M+H]+ |
|---|---|---|
| **b9 (trans)** | 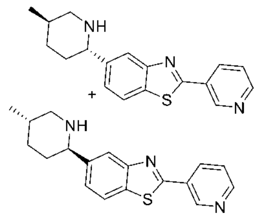 | 310 |

Synthesis of intermediates **b3 and b10-b13**

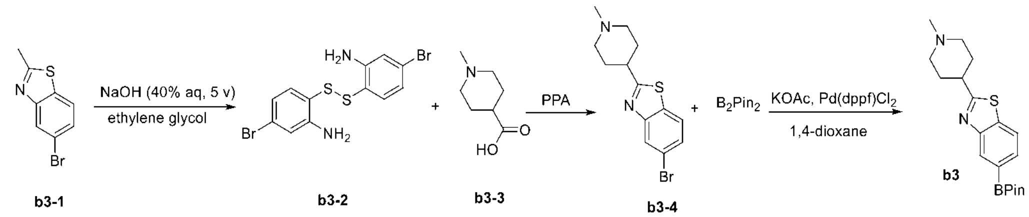


b3-1 b3-2 b3-3 b3-4 b3

Step 1: Under nitrogen protection, starting material 2-methyl-5-bromo-1,3-benzothiazole **b3-1** (1.2 g, 5.26 mmol) was dissolved in 6 mL of ethylene glycol, and an aqueous solution of NaOH (40%, 6 mL) was slowly added dropwise. Upon completion of the addition, the temperature was raised to 140°C, allowing reaction to proceed for 4 hours before cooling to room temperature. 50 mL of ice water was added to the reaction solution, and the pH was adjusted to about 6 with dilute hydrochloric acid. The reaction solution was extracted with dichloromethane, dried over anhydrous sodium sulfate, and concentrated to obtain a yellow solid b3-2 (900 mg) in a yield of 42%. LCMS ESI-MS m/z: 405 $[M+H]^+$.

Step 2: 1-methyl-4-piperidinic acid **b3-3** (630 mg, 4.44 mmol) was dissolved in 14 mL PPA, heated to 160°C, and stirred for 30 minutes. The intermediate **b3-2** (900 mg, 2.22 mmol) from the previous step was added, and the mixture was heated to 180°C for 3 hours before stopping the reaction. 100 mL of ice water was poured into the reaction solution. The pH was adjusted to about 8 with a saturated aqueous solution of sodium bicarbonate, before extracting with ethyl acetate, drying over anhydrous sodium sulfate, and concentrating. The crude product was separated by flash column chromatography (C18, $CH_3CN/H_2O$, 3/5) to obtain a yellow solid **b3-4** (250 mg) in a yield of 36%. LCMS ESI-MS m/z: 311 $[M+H]^+$.

Step 3: Under nitrogen protection, the yellow solid **b3-4** (3.0 g, 9.64 mmol) was dissolved together with pinacol diboronate (2.9 g, 11.6 mmol) and KOAc (2.8 g, 28.9 mmol) in 30 mL 1,4-dioxane, and catalyst Pd(dppf)$Cl_2$ (390 mg, 0.48 mmol) was added. The mixture was heated to 90°C and reacted for 2 hours before stopping the reaction and filtering. 100 mL of water was added to the reaction solution, before extracting with ethyl acetate, drying over anhydrous sodium sulfate, and concentrating to obtain a brown solid **b3** (2.7 g) in a yield of 78%. LCMS ESI-MS m/z: 359 $[M+H]^+$.

Similar starting materials/intermediates were used to synthesize the following target intermediates by analogy to the synthetic route of compound **b3.**

| Target intermediate | Intermediate structure | LC-MS [M+H]+ |
|---|---|---|
| **b10** | 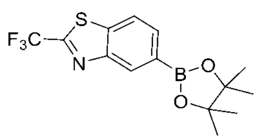 | 330 |
| **b11** | 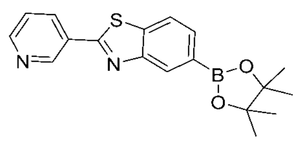 | 339 |

(continued)

| Target intermediate | Intermediate structure | LC-MS [M+H]+ |
|---|---|---|
| **b12** | 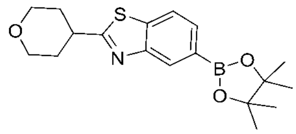 | 346 |
| **b13** | 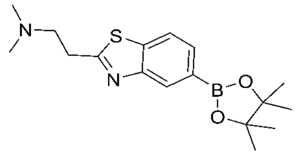 | 333 |

**Preparation of key intermediates c1-c4**

Synthesis of intermediates **c1-c4**

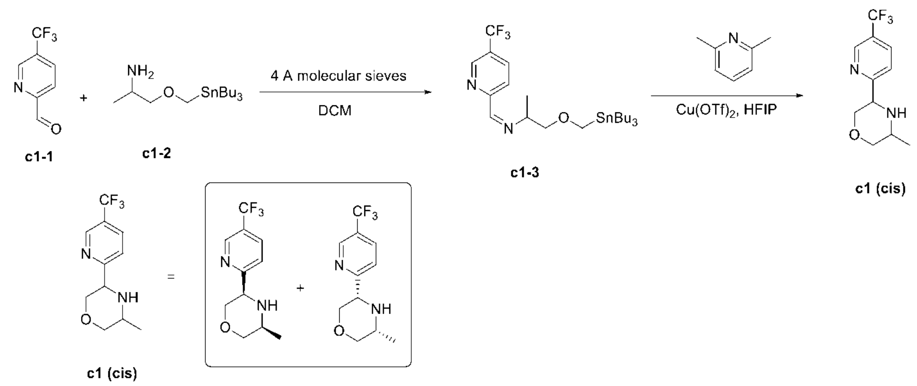


c1-1 c1-2 c1-3 c1 (cis)

c1 (cis)

Step 1: Under nitrogen protection, starting material 5-trifluoromethyl-pyridine-2-carboxaldehyde **c1-1** (558 mg, 3.53 mmol) and starting material **c1-2** (508 mg, 1.34 mmol) were dissolved in 5.0 mL of dichloromethane. A 4A molecular sieve (1.0 g) was added, and the solution reacted at room temperature for 12 hours before stopping the reaction and filtering. The filtrate was concentrated under reduced pressure to obtain a yellow oil **c1-3** (600 mg) in a yield of 84%. LCMS ESI-MS m/z: 537 $[M+H]^+$.

Step 2: Under nitrogen protection, 2,6-dimethylpyridine (48 mg, 0.45 mmol) and catalyst $Cu(OTf)_2$ (162 mg, 0.45 mmol) were dissolved in 3 mL hexafluoroisopropanol and reacted at room temperature for 7 hours. Intermediate **c1-3** (600 mg, 1.12 mmol) was added dropwise to the reaction solution. Upon completion of the addition, the reaction was continued for 10 hours before stopping. 6 mL of aqueous ammonia was added to the reaction solution, stirring for 1 hour before extracting with dichloromethane, drying over anhydrous sodium sulfate, concentrating, and separating the crude product by flash column chromatography (PE/EA, 1/1) to obtain a cis-configured intermediate **c1** (140 mg) in a yield of 51%. LCMS ESI-MS m/z: 247 $[M+H]^+$.

The following target molecules were synthesized from similar skeletal structures by analogy to the synthetic route of compound **c1.**

| Target intermediate | Intermediate structure | LC-MS [M+H]+ |
|---|---|---|
| **c2** | 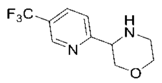 | 233 |

(continued)

| Target intermediate | Intermediate structure | LC-MS [M+H]+ |
|---|---|---|
| **c3 (cis)** | 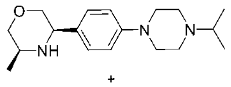<br>+<br>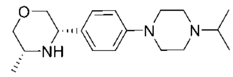 | 304 |
| **c4 (cis)** | 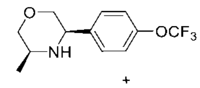<br>+<br>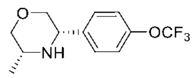 | 262 |

**Preparation of key intermediates d1-d17**

Synthesis of intermediates **d1-d3, d5-d8, d11, d15-d17**

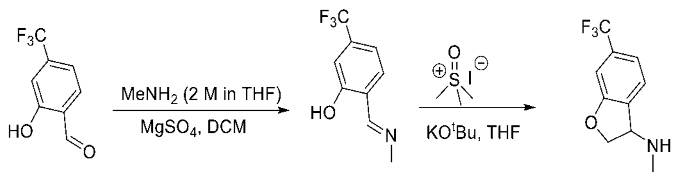


**d1-1** **d1-2** **d1**

Step 1: Under nitrogen protection, the starting material 2-hydroxy-4-trifluoromethylbenzaldehyde **d1-1** (1.0 g, 5.26 mmol) and methylamine tetrahydrofuran solution (5.3 mL, 2M) were dissolved in 20 mL of anhydrous dichloromethane. Magnesium sulfate (2.5 g, 21.0 mmol) was added, reacting at room temperature for 12 hours before stopping the reaction and filtering. The filtrate was concentrated under reduced pressure to obtain a yellow solid **d1-2** (470 mg) in a yield of 44%. LCMS ESI-MS m/z: 204 $[M+H]^+$.

Step 2: Under nitrogen protection, trimethylsulfoxide iodide (1.27 g, 5.78 mmol) and potassium tert-butoxide (649 mg, 5.78 mmol) were dissolved in 13 mL of anhydrous tetrahydrofuran and stirred for 30 minutes. Intermediate **d1-2** (470 mg, 2.31 mmol) was added to the reaction solution, and the temperature raised to 50°C for 4 hours before cooling the reaction to room temperature. Potassium tert-butoxide (260 mg, 2.31 mmol) was added to the reaction solution, and the reaction was allowed to continue at room temperature for 12 hours before stopping the reaction and filtering. The solvent was evaporated under reduced pressure, and the crude product was separated by reversed-phase flash column chromatography (C18, $CH_3CN/H_2O$, 1/1) to obtain a white product **d1** (40 mg) in a yield of 8%. LCMS ESI-MS m/z: 218 $[M+H]^+$.

The following intermediates were synthesized by analogy to the synthetic route of intermediate **d1.**

| Target intermediate | Intermediate structure | LC-MS [M+H]+ |
|---|---|---|
| **d2** | 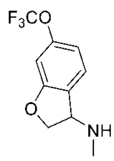 | 234 |
| **d3** | 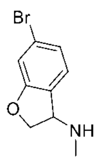 | 228 |

(continued)

| Target intermediate | Intermediate structure | LC-MS [M+H]+ |
|---|---|---|
| **d5** | 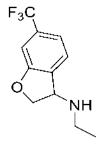 | 232 |
| ***d6*** | 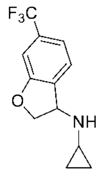 | 244 |
| **d7** | 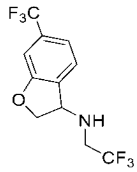 | 286 |
| **d8** | 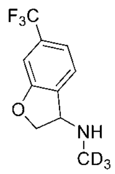 | 221 |
| **d11** | 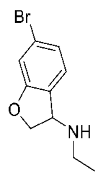 | 242 |
| **d15** | 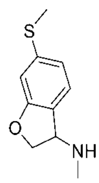 | 228 |
| **d16** | 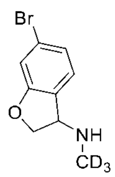 | 231 |
| **d17** | 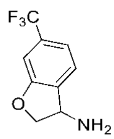 | 204 |

Synthesis of intermediates **d4, d9, d10:**

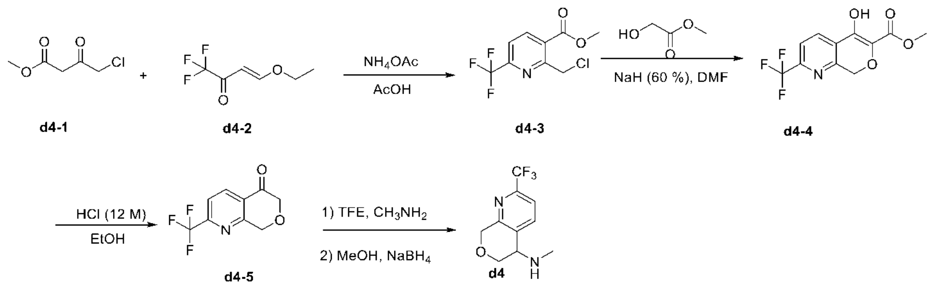


d4-1 d4-2 d4-3 d4-4 d4-5 d4

Step 1: Under nitrogen protection, starting material **d4-1** (12.2 g, 81.0 mmol) and starting material **d4-**2 (13.6 g, 81.0 mmol) were dissolved in 20 mL of acetic acid. Ammonium acetate (12.5 g, 162 mmol) was added, and the solution heated to 120°C to undergo reaction for 1 hour before cooling to room temperature. 100 ml of ice water was added to the reaction solution, which was extracted three times with methyl tert-butyl ether. The organic phases were combined, dried over anhydrous sodium sulfate, concentrated, and the crude product was separated by flash column chromatography (PE/EA, 20/1) to obtain a white solid **d4-**3 (10.5 g) in a yield of 51%. LCMS ESI-MS m/z: 254 $[M+H]^+$.

Step 2: Under nitrogen protection, the intermediate **d4-3** (10.5 g, 41.4 mmol) and methyl 2-hydroxyacetate (7.5 g, 82.8 mmol) were dissolved in 105 mL DMF before adding NaH (3.3 g, 82.8 mmol, 60%) and reacting at room temperature for 2 hours. 300 mL of ice water was added to the reaction solution, which was extracted three times with ethyl acetate. The organic phases were combined, dried over anhydrous sodium sulfate, and concentrated to obtain a yellow oil **d4-4** (5.0 g) in a yield of 44%. LCMS ESI-MS m/z: 276 $[M+H]^+$.

Step 3: The intermediate **d4-4** (5.0 g, 18.2 mmol) was dissolved in 50 mL of ethanol. Concentrated hydrochloric acid (100 mL, 12M) was added, and the solution was heated to 100°C to undergo reaction for 1 hour before cooling to room temperature. 300 mL of ammonia water was added dropwise to the reaction solution, before extracting three times with ethyl acetate, combining the organic phases, drying over anhydrous sodium sulfate, and concentrating. The crude product was separated by flash column chromatography (PE/EA, 10/1) to obtain a yellow solid **d4-5** (366 mg) in a yield of 9%. LCMS ESI-MS m/z: 218 $[M+H]^+$.

Step 4: Under nitrogen protection, the intermediate **d4-5** (110 mg, 0.51 mmol) and methylamine tetrahydrofuran solution (1.3 mL, 2M) were dissolved in 1 mL of trifluoroethanol and stirred at room temperature for 16 hours. $NaBH_4$ (96 mg, 2.5 mmol) and 0.25 mL methanol were added to the reaction solution, and the reaction was continued at room temperature for 1 hour before stopping the reaction and filtering. The filtrate was concentrated under reduced pressure, and the crude product was separated by reversed-phase flash column chromatography (C18, $CH_3CN/H_2O$, 7/10) to obtain a white solid **d4** (60 mg) in a yield of 51%. LCMS ESI-MS m/z: 233 $[M+H]^+$.

The following intermediates were synthesized by analogy to the synthetic route of **d4.**

| Target intermediate | Intermediate structure | LC-MS $[M+H]^+$ |
|---|---|---|
| **d9** | 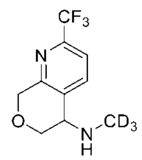 | 236 |
| **d10** | 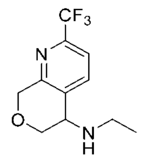 | 247 |

Synthesis of intermediates **d12-d14:**

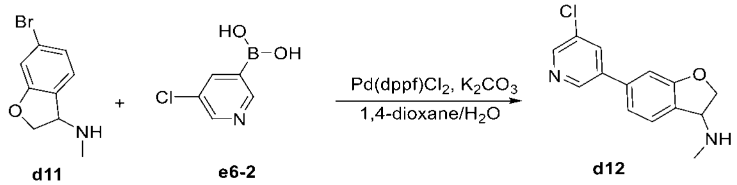


d11 e6-2 d12

Step 1: Under nitrogen protection, the intermediate **d11** (1.0 g, 4.4 mmol), potassium carbonate (1.2 g, 8.77 mmol) and the starting material 5-chloro-pyridine-3-boronic acid **e6-2** (1.0 g, 4.4 mmol) were dissolved in 20 mL of a mixed solution of 1,4-dioxane and water (v/v, 4/1), and catalyst Pd(dppf)$Cl_2$ (321 mg, 0.44 mmol) was added. The solution was heated to 100°C for 1 hour, filtered, and the solvent evaporated under reduced pressure. 100 mL of water was added to the mixture, before extracting with ethyl acetate, drying over anhydrous sodium sulfate, concentrating, and separating the crude product by reversed-phase flash column chromatography (C18, $CH_3CN/H_2O$, 6/10) to obtain a yellow oil **d12** (960 mg) in a yield of 84%. LC-MS: $[M+H]^+$ = 261.

The following intermediates were synthesized by analogy to the synthetic route of **d12.**

| Target intermediate | Intermediate structure | LC-MS $[M+H]^+$ |
|---|---|---|
| **d13** | 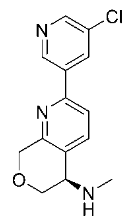 | 276 |
| **d14** | 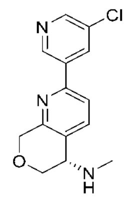 | 276 |

**Preparation** of **key intermediates el-e9**

Synthesis of intermediates **el-e4**

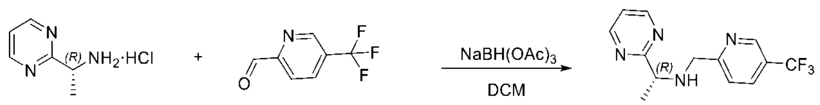


e1-1 e1-2 e1

Steps: Under nitrogen protection, starting material **el-1** (558 mg, 3.53 mmol) and starting material **el-**2 (618 mg, 3.53 mmol) were dissolved in 13 mL of dichloromethane. $NaBH(OAc)_3$ (2.25 g, 10.6 mmol) was added, reacting at room temperature for 1 hour before stopping the reaction. The solvent was evaporated under reduced pressure, and the mixture dissolved in 2 mL of methanol. Aqueous ammonia was added to the system to adjust the pH to about 8, and the solvent was evaporated under reduced pressure. The mixture was separated by reversed-phase flash column chromatography (C18, $CH_3CN/H_2O$, 4/5) to obtain a green oil **e1** (320 mg) in a yield of 29%. LC-MS: $[M+H]^+$ = 283.

The following target molecules were synthesized from similar skeletal structures by analogy to the synthetic route of compound **c1.**

| Target intermediate | Intermediate structure | LC-MS $[M+H]^+$ |
|---|---|---|
| **e2** | 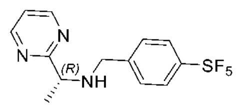 | 340 |
| **e3** | 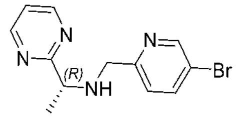 | 293 |
| **e4** | 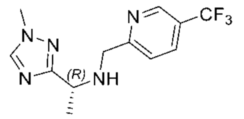 | 286 |

Synthesis of intermediate **e5**

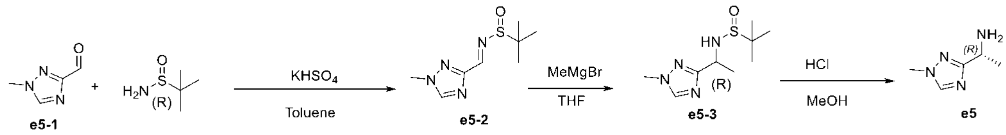


Step 1: Under nitrogen protection, starting material 1-methyl-1,2,4-triazole-3-carboxaldehyde e5-1 (3.1 g, 27.9 mmol) and starting material (R)-configured tert-butylsulfenamide (3.4 g, 27.9 mmol) were dissolved in 62 mL of anhydrous toluene. $KHSO_4$ was added (3.8 g, 27.9 mmol), heating to 50°C to undergo reaction for 12 hours, then cooling to room temperature and filtering. The filtrate was evaporated under reduced pressure to remove the solvent to obtain an oil **e5-2** (4.9 g) in a yield of 91%. LCMS ESI-MS m/z: 215 $[M+H]^+$.

Step 2: At -78°C under nitrogen protection, oil **e5-2** (4.9 g, 22.87 mmol) was dissolved in 98 mL of anhydrous tetrahydrofuran, and a hexane solution of Grignard reagent methylmagnesium bromide (45.8 mL, 45.73 mmol) was added dropwise. Upon completion of the addition, the reaction was allowed to continue at this temperature for 45 minutes. The temperature was slowly raised to room temperature, and 100 mL of a saturated aqueous solution of sodium bicarbonate was added to the system to quench the reaction. The organic solvent was evaporated under reduced pressure, before extracting with dichloromethane, drying over anhydrous sodium sulfate, concentrating, and separating the crude product by reversed-phase flash column chromatography (C18, $CH_3CN/H_2O$, 7/10) to obtain an oil **e5-3** (1.1 g), in a yield of 21%. LCMS ESI-MS m/z: 231 $[M+H]^+$.

Step 3: Under nitrogen protection, the intermediate **e5-3** (2.1 g, 9.12 mmol) was dissolved in 42 mL of methanol, and a solution of hydrogen chloride in dioxane (3.7 mL, 14.6 mmol) was added. The reaction was allowed to proceed at room temperature for 1 hour, before stopping the reaction. 30 mL of water was added to the system before extracting with methyl tert-butyl ether, drying over anhydrous sodium sulfate, and concentrating to obtain compound **e5** (1.8 g) in a yield of 95%. LCMS ESI-MS m/z: 127 $[M+H]^+$.

Synthesis of intermediates **e6-e9**

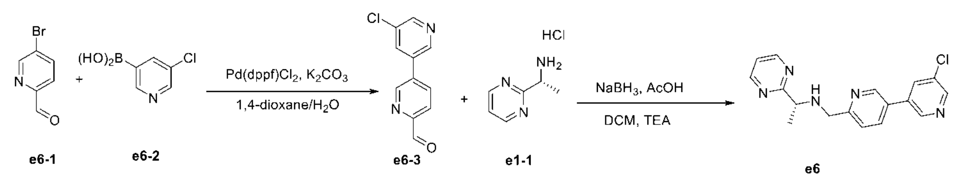


Step 1: Under nitrogen protection, starting material 5-bromo-2-pyridine **c6-1** (2.9 g, 15.7 mmol), potassium carbonate (4.4 g, 31.5 mmol) and starting material 5-chloro-pyridine-3-boronic acid **e6-2** (22.5 g, 15.7 mmol) were dissolved in 58 mL of a mixed solution of 1,4-dioxane and water (v/v, 2/1), and catalyst $Pd(dppf)Cl_2$ (929 mg, 1.57 mmol)

was added. The system was heated to react at 100°C for 1 hour, filtered, and the solvent was evaporated under reduced pressure. 100 mL of water was added to the mixture, before extracting with ethyl acetate, drying over anhydrous sodium sulfate, concentrating, and separating the crude product by flash column chromatography (PE/EA, 5/1) to obtain a yellow solid **e6-3** (2.5 g) in a yield of 73%. LC-MS: $[M+H]^+$ = 219.

Step 2: In an ice bath, under nitrogen protection, the intermediate **e6-3** (1.0 g, 4.5 mmol), triethylamine (0.9 g, 9.15 mmol) and starting material **el-1** (700 mg, 5.03 mmol) were dissolved in 20 mL of dichloromethane, and reducing agent $NaBH_3$ (1.1 g, 5.48 mmol) and acetic acid (0.3 g, 5.03 mmol) were added. The reaction was allowed to proceed for 2 hours in the ice bath before stopping the reaction. Aqueous ammonia was added to the mixture to adjust the pH to about 8, the solvent was evaporated under reduced pressure, and the crude product was separated by reversed-phase flash column chromatography (C18, $CH_3CN/H_2O$, 3/5) to obtain a yellow oil e6 (612 mg), in a yield of 41%. LC-MS: $[M+H]^+$ = 326.

The following target molecules were synthesized from similar skeletal structures by analogy to the synthetic route of compound **e6.**

| Target intermediate | Intermediate structure | LC-MS $[M+H]^+$ |
|---|---|---|
| **e7** | 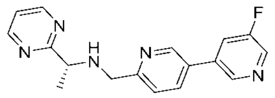 | 310 |
| **e8** | 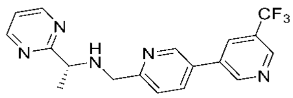 | 360 |
| **e9** | 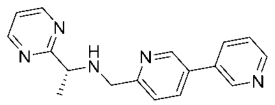 | 292 |

**Chiral separation of key intermediates f1-f24**

Synthesis of intermediates **f1, f2**

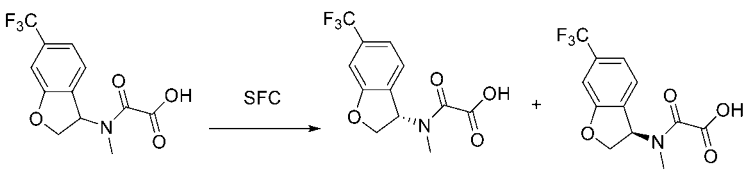


**a3** **f1 (*S*)** **f2 (*R*)**

Separation conditions: chromatographic column: CHIRALPAK AS3; mobile phase: Hex (0.1%TFA): EtOH=90:10; flow rate: 1.0 mL/min;);

**f1** (Retention time: 2.767 min; 105 mg, yield 35%); $^1$H NMR (300 MHz, DMSO-$d_6$) $\delta$ 7.50 (dd, J=26.1, 7.8 Hz, 1H), 7.39-7.23 (m, 2H), 5.92 (ddd, J=163.8, 8.8, 4.0 Hz, 1H), 4.82-4.57 (m, 2H), 2.58 (d, J=39.0 Hz, 3H).

**f2** (Retention time: 3.853 min; 110 mg, yield 37%).

Synthesis of intermediates **f3, f4**

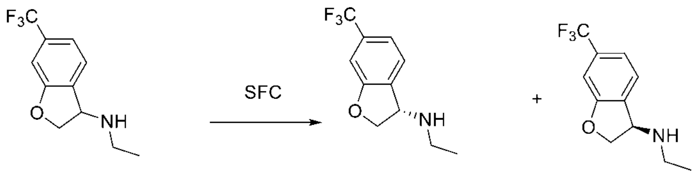


d5 f3 (*S*) f4 (*R*)

Separation conditions: chromatographic column: CHIRALPAK IF, 2*25 cm, 5 μm; mobile phase A: Hex (0.5% 2M $NH_3$-MeOH), mobile phase B: MeOH: DCM=1:1; flow rate: 20 mL/min;
**f3** (Retention time: 1.203 min; 150 mg);
**f4** (Retention time: 1.593 min; 150 mg).

Synthesis of intermediates **f5-f6**

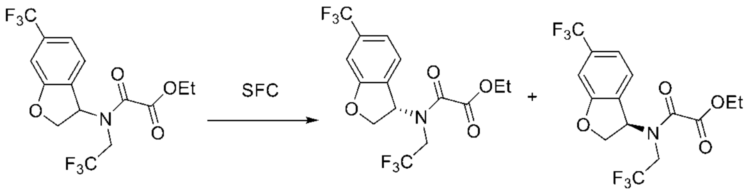


a22 f5 (*S*) f6 (*R*)

Separation conditions: chromatographic column: CHIRAL ART Cellulose-SB 5*25 cm, 5 μm; mobile phase A: Hex (0.5% 2M $NH_3$-MeOH), mobile phase B: MeOH: DCM=1:1; flow rate: 1 mL/min;
**f5** (Retention time: 12.455 min; 110 mg);
**f6** (Retention time: 13.892 min; 110 mg).

Synthesis of intermediates **f7-f8**

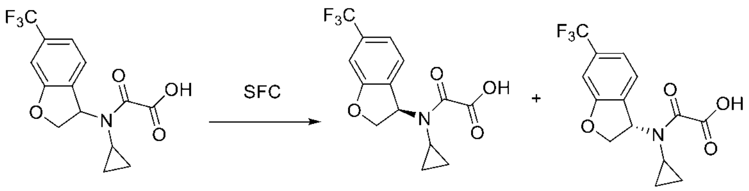


a23 f7 (*R*) f8 (*S*)

Separation conditions: chromatographic column CHIRALPAK IF, 2*25 cm, 5 μm mobile phase A: Hex (0.1% TFA), mobile phase B: EtOH, flow rate: 20.0 mL/min;
**f7** (Retention time: 12.417 min; 130 mg);
**f8** (Retention time: 17.931 min; 115 mg).

Synthesis of intermediates **f9-f10**

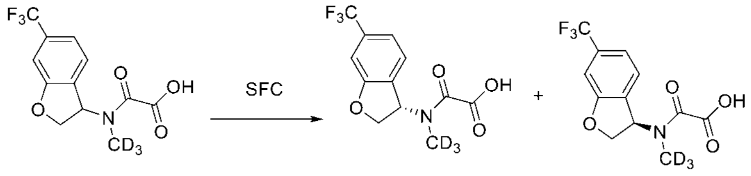


a24 f9 (*S*) f10 (*R*)

Separation conditions: chromatographic column: CHIRALPAK AS-H, 2*25 cm, 5 μm; mobile phase A: Hex (0.4% TFA), mobile phase B: EtOH, flow rate: 20.0 mL/min;
**f9** (Retention time: 9.022 min; 65 mg);
**f10** (Retention time: 12.626 min; 60 mg).

Synthesis of intermediates **f11-f12**

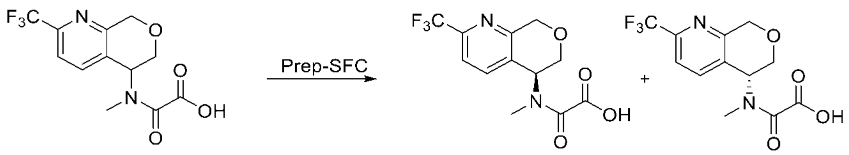


a9 f11 (*S*) f12 (*R*)

Separation conditions: Chromatographic column: CHIRALPAK AS-H, 5*25 cm, 5 μm; Mobile phase A: Hex (0.4% TFA), Mobile phase B: MeOH; EtOH=1:1, Flow rate: 20.0 mL/min;
**f11** (Retention time: 12.527 min; 83 mg);
**f12** (Retention time: 17.993 min; 82 mg).

Synthesis of intermediates **f13-f14**

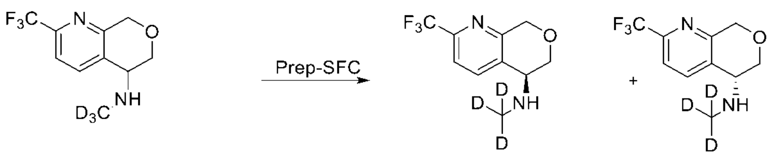


d9 f13 (*S*) f14 (*R*)

Separation conditions: chromatographic column: CHIRAL ART Cellulose-SB, 2*25 cm, 5 μm; mobile phase A: Hex (0.4% DEA), mobile phase B: isopropanol, flow rate: 20.0 mL/min;
**f13** (Retention time: 11.775 min; 140 mg);
**f14** (Retention time: 12.988 min; 140 mg).

Synthesis of intermediates **f15-f16**

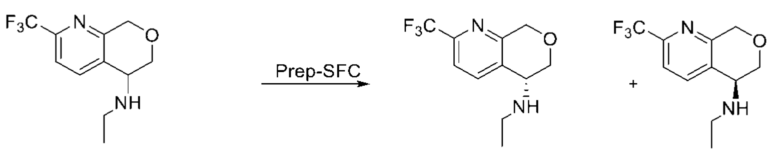


d10 f15 (*R*) f16 (*S*)

Separation conditions: chromatographic column: Lux 5 μm Cellulose-4, 2.12*25 cm, 5 μm; mobile phase A: Hex (0.4% DEA), mobile phase B: isopropanol, flow rate: 20.0 mL/min;
**f13** (Retention time: 8.451 min; 170 mg);
**f14** (Retention time: 10.379 min; 170 mg).

Synthesis of intermediates **f17-f18**

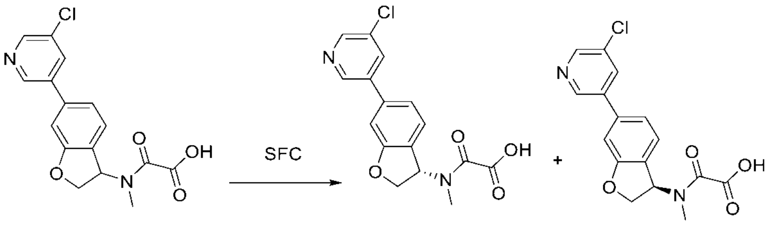


**a28** **f17 (*S*)** **f18 (*R*)**

Separation conditions: chromatographic column: CHIRALPAK AS3; mobile phase A: Hex (0.1%TFA)/(MeOH: EtOH=1:1) = 85/15; flow rate: 1.0 mL/min;
**f17** (Retention time: 4.784 min; 82 mg);
**f18** (Retention time: 6.610 min; 80 mg).

Synthesis of intermediates **f19-f20**

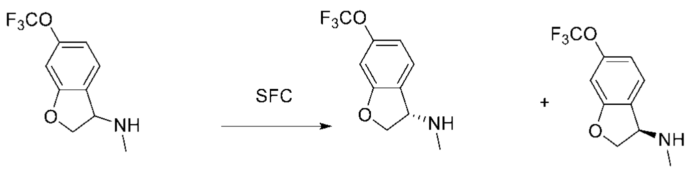


**d2** **f19 (*S*)** **f20 (*R*)**

Separation conditions: Chromatographic column: CHIRALPAK IF 2*25 cm, 5 μm; Mobile phase A: Hex (0.5% 2M $NH_3$-MeOH), mobile phase B: EtOH; Flow rate: 20 mL/min;
**F19** (Retention time: 5.453 min; 160 mg);
**F19** (Retention time: 7.197 min; 170 mg).

Synthesis of intermediate **f21-f22** (9.5 g resolution)

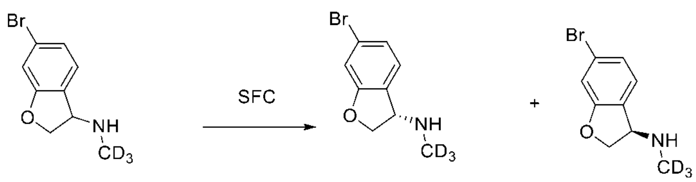


**d16** **f21 (*S*)** **f22 (*R*)**

Separation conditions: Chromatographic column: CHIRALPAK IF, 2*25 cm, 5 μm; Mobile phase A: Hex (0.5% 2M $NH_3$-MeOH), mobile phase B: EtOH; Flow rate: 20 mL/min;
**f21** (Retention time: 21.302 min; 4.0 g);

**f22** (Retention time: 25.063 min; 4.5 g).

Synthesis of intermediate **f23-f24** (10.1 g resolution)

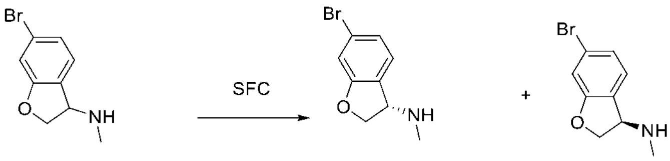


d3 f23 (*S*) f24 (*R*)

Separation conditions: Chromatographic column:UniChiralCNZ-5H, 2*25 cm, 5 μm; Mobile phase A: Hex (0.5% 2M $NH_3$-MeOH), mobile phase B: EtOH; Flow rate: 25 mL/min;
**f23** (Retention time: 9.872 min; 4.1 g);
**f24** (Retention time: 15.440 min; 5.1 g).

**Example 2:** Synthesis of target molecules **P1-P33** and control molecule **A1**

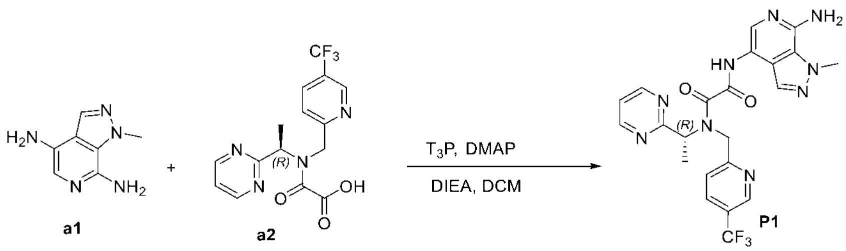


a1 a2 P1

Steps: Under nitrogen protection, intermediate a1 (100 mg, 0.61 mmol), 4-dimethylaminopyridine DMAP (225 mg, 1.84 mmol) and DIEA (396 mg, 3.1 mmol) were dissolved in 3 mL of dichloromethane, a dichloromethane solution of intermediate **b1** (217 mg, 0.61 mmol, 0.5 mL) was added dropwise, and a condensation reagent $T_3P$ (780 mg, 2.5 mmol) was added. The mixture was reacted at room temperature for 12 hours. The solvent was evaporated under reduced pressure, and the crude product eparated by reversed-phase flash column chromatography (C18, $CH_3CN/H_2O$, 4/5) to obtain the target compound **P1** (34 mg) in a yield of 11%. LCMS ESI-MS m/z: 500 $[M+H]^+$.

$^1$H NMR (300 MHz, DMSO-$d_6$) δ 10.64 (d, *J*=45.5 Hz, 1H), 8.89-8.68 (m, 3H), 8.20-8.06 (m, 1H), 7.82 (d, *J*=32.6 Hz, 1H), 7.70-7.42 (m, 2H), 7.37 (dt, *J*=8.8, 4.9 Hz, 1H), 6.22 (d, *J*=19.1 Hz, 2H), 5.73 (dq, *J*=41.0, 7.0 Hz, 1H), 5.29-4.63 (m, 2H), 4.23 (d, *J*=15.5 Hz, 3H), 1.59 (dd, *J*=30.7, 7.0 Hz, 3H).

Similar starting materials or intermediates (replacing **a2** with intermediates **a3-a5, a9-a21, a23-a30, a32-a35, a37-a40, f1-f2, f5-f12, f17-f18 or a43,** etc.) were used to synthesize the following target molecules by analogy to the synthetic route of **P1.**

*Indicates chiral center, which was not resolved.

| Compound | Target molecular structure | Separation conditions/$^1$H NMR/ | LC-MS $[M+H]^+$ |
|---|---|---|---|
| **P2** | 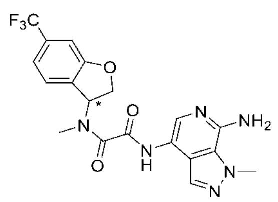 | $^1$H NMR (400 MHz, DMSO-$d_6$) δ 10.73 (d, *J*=60.6 Hz, 1H), 8.04 (s, 1H), 7.96-7.83 (m, 1H), 7.63 (dd, *J*=54.0, 7.8 Hz, 1H), 7.40-7.34 (m, 1H), 7.29 (dd, *J*=4.6, 1.6 Hz, 1H), 6.28-5.86 (m, 3H), 4.82 (ddd, *J*=12.7, 10.6, 9.0 Hz, 1H), 4.69 (ddd, *J*=12.2, 10.5, 4.1 Hz, 1H), 4.28 (s, 3H), 2.68 (d, *J*=61.7 Hz, 3H). | 435 |

(continued)

| Compound | Target molecular structure | Separation conditions/$^1$H NMR/ | LC-MS [M+H]$^+$ |
|---|---|---|---|
| **P2a** | 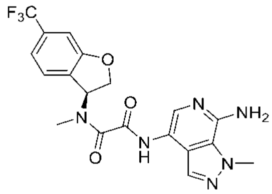 | $^1$H NMR (300 MHz, DMSO-$d_6$) $\delta$ 10.73 (d, $J$=44.0 Hz, 1H), 8.06-7.81 (m, 2H), 7.62 (dd, $J$=40.8, 7.8 Hz, 1H), 7.37 (q, $J$=6.5, 5.2 Hz, 1H), 7.29 (d, $J$=3.7 Hz, 1H), 6.36-5.84 (m, 3H), 4.88-4.62 (m, 2H), 4.28 (s, 3H), 2.68 (d, $J$=46.0 Hz, 3H). | 435 |
| **P2b** | 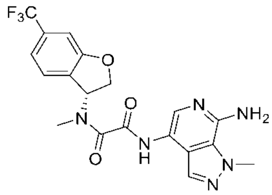 | $^1$H NMR (300 MHz, DMSO-$d_6$) $\delta$ 10.74 (d, $J$=44.2 Hz, 1H), 8.07-7.80 (m, 2H), 7.62 (dd, $J$=40.7, 7.9 Hz, 1H), 7.37 (t, $J$=6.7 Hz, 1H), 7.32-7.24 (m, 1H), 6.39-5.82 (m, 3H), 4.89-4.61 (m, 2H), 4.28 (s, 3H), 2.68 (d, $J$=46.1 Hz, 3H). | 435 |
| **P3** | 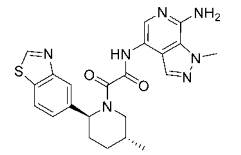 + 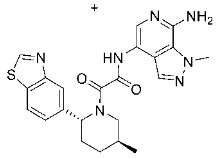 | $^1$H NMR (300 MHz, DMSO-$d_6$) $\delta$ 10.78 (d, $J$=20.6 Hz, 1H), 9.42 (d, $J$=3.9 Hz, 1H), 8.27-7.72 (m, 4H), 7.53 (dd, $J$=30.0, 8.9 Hz, 1H), 6.22 (d, $J$=15.2 Hz, 2H), 5.54 (d, $J$=140.4 Hz, 1H), 4.29 (s, 2H), 4.22 (s, 1H), 3.49 (d, $J$=12.3 Hz, 1H), 3.38 (s, 1H), 2.36 (d, $J$=15.4 Hz, 1H), 2.18 (d, $J$=13.3 Hz, 1H), 1.91 (s, 1H), 1.76 (s, 1H), 1.38 (d, $J$=13.8 Hz, 1H), 1.23 (s, 1H), 1.09 (d, $J$=7.0 Hz, 3H), 0.97-0.81 (m, 1H). | 450 |
| **P3a** | 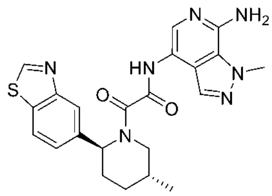 | Separation conditions: (Chromatographic column: CHIRALPAK IF3; Mobile phase A: (Hex: DCM=1:1)(0.1%DEA): EtOH=70:30; Flow rate: 1 mL/min; Retention time: 2.579 min.<br>$^1$H NMR (300 MHz, DMSO-$d_6$) $\delta$ 10.80 (d, $J$=20.6 Hz, 1H), 9.42 (d, $J$=4.3 Hz, 1H), 8.18 (dd, $J$=19.0, 10.1 Hz, 1H), 8.01 (d, $J$=21.9 Hz, 1H), 7.89 (s, 1H), 7.78 (d, $J$=6.8 Hz, 1H), 7.53 (dd, $J$=30.9, 9.0 Hz, 1H), 6.25 (d, $J$=14.8 Hz, 2H), 5.54 (d, $J$=142.8 Hz, 1H), 4.26 (d, $J$=19.9 Hz, 4H), 3.49 (d, $J$=13.6 Hz, 1H), 2.35 (d, $J$=16.2 Hz, 1H), 2.16 (s, 1H), 1.91 (s, 1H), 1.74 (s, 1H), 1.39 (s, 1H), 1.08 (d, $J$=6.9 Hz, 3H). | 450 |
| **P3b** | 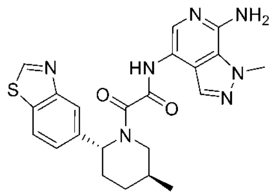 | Separation conditions: Same as above; Retention time: 4.342 min.<br>$^1$H NMR (300 MHz, DMSO-$d_6$) $\delta$ 10.80 (d, $J$=20.8 Hz, 1H), 9.43 (s, 1H), 8.18 (dd, $J$=18.8, 10.3 Hz, 1H), 8.02 (d, $J$=22.5 Hz, 1H), 7.89 (s, 1H), 7.78 (d, $J$=7.0 Hz, 1H), 7.53 (dd, $J$=31.4, 9.1 Hz, 1H), 6.24 (d, $J$=15.1 Hz, 2H), 5.78 (s, 1H), 4.26 (d, $J$=19.9 Hz, 4H), 3.49 (d, $J$=13.8 Hz, 1H), 2.36 (d, $J$=16.3 Hz, 1H), 2.16 (s, 1H), 1.91 (s, 1H), 1.74 (s, 1H), 1.40 (s, 1H), 1.09 (d, $J$=6.9 Hz, 3H). | 450 |

(continued)

| Compound | Target molecular structure | Separation conditions/$^1$H NMR/ | LC-MS [M+H]$^+$ |
|---|---|---|---|
| **P4** | 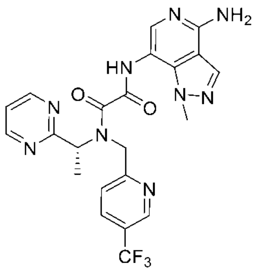 | $^1$H NMR (400 MHz, DMSO-$d_6$) $\delta$ 10.51-9.98 (m, 1H), 8.91-8.63 (m, 3H), 8.21-8.07 (m, 2H), 7.78-7.61 (m, 1H), 7.54-7.35 (m, 2H), 7.09-6.77 (m, 2H), 5.80 (dq, *J*=13.9, 6.9 Hz, 1H), 5.30-4.69 (m, 2H), 4.05 (d, *J*=27.3 Hz, 3H), 1.71-1.48 (m, 3H). | 500 |
| **P5** | 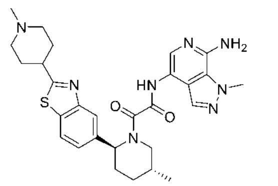 + 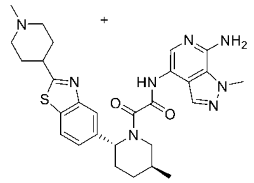 | $^1$H NMR (400 MHz, DMSO-$d_6$) $\delta$ 10.79 (d, *J*=30.9 Hz, 1H), 8.08 (t, *J*=10.0 Hz, 1H), 7.97 (s, 1H), 7.89 (d, *J*=6.8 Hz, 1H), 7.78 (d, *J*=7.6 Hz, 1H), 7.52-7.35 (m, 1H), 6.23 (d, *J*=19.7 Hz, 2H), 5.51 (d, *J*=191.5 Hz, 1H), 4.29 (s, 2H), 4.23 (s, 1H), 3.47 (d, *J*=13.9 Hz, 1H), 3.06 (s, 1H), 2.91-2.78 (m, 2H), 2.55 (s, 1H), 2.36 (s, 1H), 2.32 (s, 1H), 2.19 (s, 3H), 2.05 (q, *J*=11.8, 11.4 Hz, 4H), 1.82 (q, *J*=15.4, 14.3 Hz, 4H), 1.39 (s, 1H), 1.23 (s, 1H), 1.08 (d, *J*=7.0 Hz, 3H), 0.84 (d, *J*=7.3 Hz, 1H). | 547 |
| **P6** | 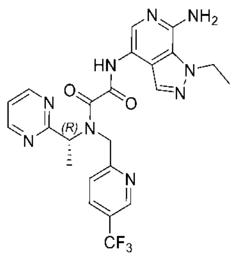 | $^1$H NMR (400 MHz, DMSO-$d_6$) $\delta$ 10.67 (d, *J*=62.7 Hz, 1H), 8.87-8.73 (m, 3H), 8.20-8.13 (m, 1H), 7.96-7.46 (m, 3H), 7.40 (dt, *J*=12.6, 4.9 Hz, 1H), 6.25 (d, *J*=26.9 Hz, 2H), 5.75 (dq, *J*=57.7, 7.0 Hz, 1H), 5.27-4.90 (m, 2H), 4.71-4.57 (m, 2H), 1.61 (dd, *J*=40.7, 7.1 Hz, 3H), 1.32 (dt, *J*=19.2, 7.1 Hz, 3H). | 514 |
| **P7** | 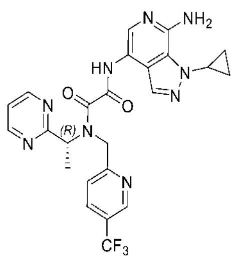 | $^1$H NMR (400 MHz, DMSO-$d_6$) $\delta$ 10.65 (d, *J*=60.9 Hz, 1H), 8.87-8.73 (m, 3H), 8.21-8.06 (m, 1H), 7.81 (d, *J*=28.4 Hz, 1H), 7.70-7.45 (m, 2H), 7.40 (dt, *J*=12.1, 4.9 Hz, 1H), 6.35 (d, *J*=25.8 Hz, 2H), 5.74 (dq, *J*=57.9, 7.0 Hz, 1H), 5.25-4.68 (m, 2H), 4.07 (ddq, *J*=21.5, 10.7, 5.9, 5.1 Hz, 1H), 1.61 (dd, *J*=39.7, 7.1 Hz, 3H), 1.24-1.15 (m, 4H). | 526 |
| **P8** | 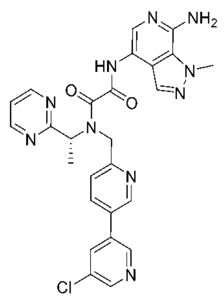 | $^1$H NMR (300 MHz, DMSO-$d_6$) $\delta$ 10.68 (d, *J*=49.1 Hz, 1H), 8.96-8.87 (m, 1H), 8.85-8.74 (m, 3H), 8.72-8.64 (m, 1H), 8.30 (d, *J*=33.4 Hz, 2H), 8.14 (dd, *J*=15.9, 8.1 Hz, 1H), 7.85 (d, *J*=32.2 Hz, 1H), 7.60 (dd, *J*=16.2, 7.3 Hz, 1H), 7.40 (t, *J*=6.1 Hz, 1H), 6.23 (d, *J*=24.3 Hz, 2H), 5.74 (dd, *J*=44.5, 7.1 Hz, 1H), 5.17-4.68 (m, 2H), 4.21 (s, 3H), 1.62 (dd, *J*=26.7, 7.1 Hz, 3H). | 543 |
| **P9** | 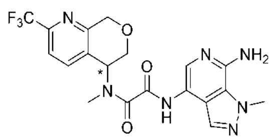 | $^1$H NMR (300 MHz, DMSO-$d_6$) $\delta$ 8.20 (d, *J*=8.1 Hz, 1H), 8.04-7.82 (m, 4H), 6.28 (d, *J*=2.6 Hz, 2H), 5.72-5.09 (m, 1H), 4.94-4.66 (m, 2H), 4.28 (d, *J*=4.4 Hz, 3H), 4.16 (dd, *J*=12.3, 5.1 Hz, 2H), 2.80 (d, *J*=46.1 Hz, 3H). | 450 |

(continued)

| Compound | Target molecular structure | Separation conditions/$^1$H NMR/ | LC-MS [M+H]$^+$ |
|---|---|---|---|
| **P9a** | 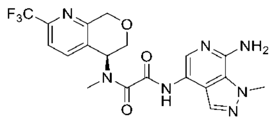 | $^1$H NMR (400 MHz, DMSO-$d_6$) $\delta$ 10.78 (d, *J*=61.3 Hz, 1H), 8.31-7.77 (m, 4H), 6.26 (d, *J*=3.6 Hz, 2H), 5.43 (dt, *J*=220.6, 5.0 Hz, 1H), 4.89 (dd, *J*=16.2, 5.3 Hz, 1H), 4.74 (dd, *J*=23.3, 16.2 Hz, 1H), 4.27 (dd, *J*=6.4, 3.0 Hz, 3H), 4.21-4.11 (m, 2H), 2.91-2.65 (m, 3H). | 450 |
| **P9b** | 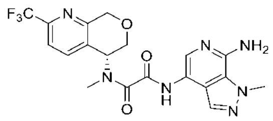 | $^1$H NMR (400 MHz, DMSO-$d_6$) $\delta$ 10.77 (d, *J*=61.9 Hz, 1H), 8.27-7.75 (m, 4H), 6.26 (d, *J*=3.4 Hz, 2H), 5.43 (dt, *J*=220.6, 5.2 Hz, 1H), 4.89 (dd, *J*=16.2, 5.3 Hz, 1H), 4.74 (dd, *J*=23.2, 16.1 Hz, 1H), 4.28 (d, *J*=5.8 Hz, 3H), 4.21-4.11 (m, 2H), 2.80 (d, *J*=62.6 Hz, 3H). | 450 |
| **P10** | 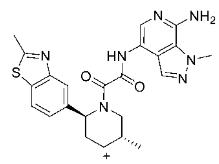 + 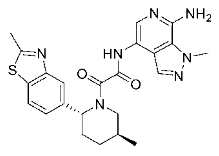 | $^1$H NMR (400 MHz, DMSO-$d_6$) $\delta$ 10.80 (d, *J*=31.2 Hz, 1H), 8.31 (s, 1H), 8.05 (t, *J*=9.7 Hz, 1H), 7.96 (d, *J*=9.4 Hz, 1H), 7.88 (d, *J*=3.5 Hz, 1H), 7.77 (d, *J*=9.3 Hz, 1H), 7.52-7.36 (m, 1H), 6.24 (d, *J=18.8* Hz, 2H), 5.75 (s, 1H), 4.26 (d, *J*=24.8 Hz, 3H), 3.52-3.28 (m, 2H), 2.80 (d, *J*=5.6 Hz, 3H), 2.32 (d, *J*=15.2 Hz, 2H), 1.89 (s, 1H), 1.72 (s, 1H), 1.37 (d, *J*=14.6 Hz, 1H), 1.23 (s, 1H), 1.08 (d, *J*=6.9 Hz, 3H). | 464 |
| **P11** | 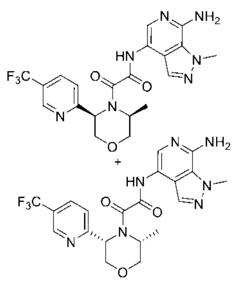 | $^1$H NMR (300 MHz, DMSO-$d_6$) $\delta$ 10.81 (d, *J*=55.3 Hz, 1H), 8.99 (d, *J*=21.8 Hz, 1H), 8.27 (t, *J*=7.7 Hz, 1H), 8.07-7.54 (m, 3H), 6.26 (d, *J*=18.7 Hz, 2H), 5.50 (d, *J*=15.4 Hz, 1H), 5.02 (dd, *J*=54.7, 12.1 Hz, 1H), 4.24 (s, 3H), 4.02 (s, 1H), 3.87 (d, *J*=12.2 Hz, 1H), 3.77-3.59 (m, 3H), 0.88 (dd, *J*=49.9, 6.9 Hz, 3H). | 464 |
| **PIIa** | 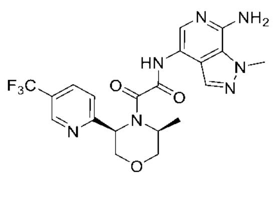 | Separation conditions: Chromatographic columns: CHIRALPAK IF3; Mobile phase A: MtBE (0.1%DEA): (MeOH: DCM=1:1)=10: 90; Flow rate: 1.0 mL/min;<br>Retention time: 0.955 min.<br>$^1$H NMR (300 MHz, DMSO-$d_6$) $\delta$ 10.78 (d, *J*=48.4 Hz, 1H), 9.07-8.92 (m, 1H), 8.27 (td, *J*=7.9, 7.5, 2.4 Hz, 1H), 8.05-7.78 (m, 2H), 7.74-7.65 (m, 1H), 6.25 (d, *J*=19.1 Hz, 2H), 5.63-5.40 (m, 1H), 5.02 (dd, *J*=55.0, 12.1 Hz, 1H), 4.34-3.95 (m, 4H), 3.91-3.58 (m, 3H), 1.23 (s, 1H), 1.16 (s, 1H), 0.88 (dd, *J*=50.1, 6.9 Hz, 5H). | 464 |
| **P11b** | 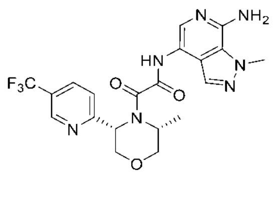 | Separation conditions: same as above; retention time: 1.310 min.<br>$^1$H NMR (300 MHz, DMSO-$d_6$) $\delta$ 10.77 (d, *J*=47.9 Hz, 1H), 8.98 (d, *J*=21.8 Hz, 1H), 8.27 (t, *J*=7.6 Hz, 1H), 8.07-7.85 (m, 2H), 7.80-7.60 (m, 1H), 6.25 (d, *J*=19.1 Hz, 2H), 5.50 (d, *J*=14.8 Hz, 1H), 5.02 (dd, *J*=54.9, 12.0 Hz, 1H), 4.27 (d, *J*=14.8 Hz, 3H), 3.95 (dd, *J*=47.9, 8.9 Hz, 1H), 3.72 (dd, *J*=14.9, 9.7 Hz, 3H), 0.88 (dd, *J*=50.1, 6.9 Hz, 3H). | 464 |

(continued)

| Compound | Target molecular structure | Separation conditions/$^1$H NMR/ | LC-MS [M+H]$^+$ |
|---|---|---|---|
| **P12** | 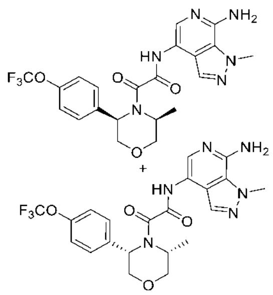 | $^1$H NMR (300 MHz, DMSO-$d_6$) $\delta$ 10.77 (s, 1H), 7.90 (d, *J*=25.1 Hz, 2H), 7.67 (d, *J*=8.2 Hz, 2H), 7.48-7.24 (m, 2H), 6.26 (s, 2H), 5.29 (d, *J*=130.4 Hz, 1H), 4.78-4.49 (m, 1H), 4.28 (s, 3H), 3.98-3.68 (m, 4H), 0.99-0.76 (m, 3H). | 479 |
| **P13a** | 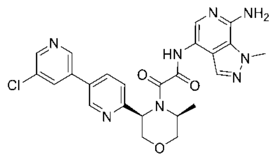 | Separation conditions: Chromatographic columns: CHIRALPAK IE, 2*25 cm, 5μm; Mobile phase A: MtBE (0.5% $NH_3$-MeOH), Mobile phase: MeOH/DCM-1/1; Flow rate: 20 mL/min; Retention time 12.234 min. $^1$H NMR (400 MHz, DMSO-$d_6$) $\delta$ 10.83 (d, *J*=50.4 Hz, 1H), 9.06-8.92 (m, 2H), 8.69 (d, *J*=2.4 Hz, 1H), 8.44-8.33 (m, 1H), 8.28 (dd, *J*=8.5, 2.5 Hz, 1H), 7.97 (d, *J*=33.9 Hz, 1H), 7.86-7.75 (m, 1H), 7.56 (d, *J*=8.3 Hz, 1H), 6.36 (d, *J*=16.8 Hz, 2H), 5.52-5.42 (m, 1H), 5.07 (dd, *J*=75.4, 11.9 Hz, 1H), 4.27 (d, *J*=23.1 Hz, 3H), 3.95 (dd, *J*=63.6, 8.6 Hz, 1H), 3.71 (q, *J*=12.6, 10.6 Hz, 3H), 0.95 (dd, *J*=64.4, 7.0 Hz, 3H). | 507 |
| **P13b** | 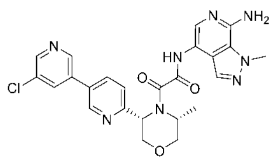 | Separation conditions: Same as above; Retention time 28.299 min.<br>$^1$H NMR (400 MHz, DMSO-$d_6$) $\delta$ 10.90 (d, *J*=49.8 Hz, 1H), 9.08-8.91 (m, 2H), 8.71-8.64 (m, 1H), 8.43-8.23 (m, 2H), 8.07-7.55 (m, 3H), 6.66 (s, 2H), 5.52-5.41 (m, 1H), 5.07 (dd, *J*=76.3, 11.9 Hz, 1H), 4.28 (d, *J*=21.7 Hz, 3H), 3.94 (dd, *J*=61.2, 9.6 Hz, 1H), 3.71 (q, *J*=12.2, 10.3 Hz, 3H), 0.95 (dd, *J*=63.8, 6.9 Hz, 3H). | 507 |
| **P14** | 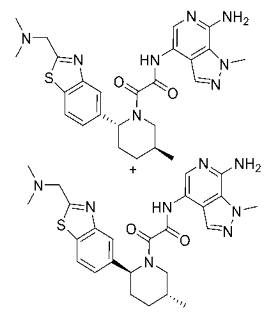 | $^1$H NMR (300 MHz, DMSO-$d_6$) $\delta$ 10.79 (d, *J*=24.9 Hz, 1H), 8.23 - 7.74 (m, 4H), 7.45 (dd, *J*=31.0, 8.1 Hz, 1H), 6.24 (d, *J*=15.0 Hz, 2H), 5.51 (d, *J*=143.6 Hz, 1H), 4.26 (d, *J*=19.5 Hz, 3H), 3.88 (d, *J*=4.0 Hz, 2H), 3.48 (d, *J*=13.7 Hz, 2H), 2.32 (d, *J*=2.1 Hz, 6H), 2.13 (d, *J*=16.2 Hz, 1H), 1.82 (d, *J*=51.2 Hz, 2H), 1.39 (s, 1H), 1.08 (d, *J*=6.9 Hz, 3H). | 507 |
| **P15** | 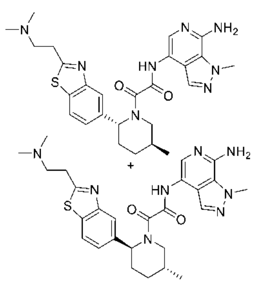 | $^1$H NMR (400 MHz, DMSO-$d_6$) $\delta$ 10.80 (d, *J*=30.2 Hz, 1H), 8.22 (s, 2H), 8.11-7.75 (m, 4H), 7.43 (dd, *J*=40.3, 8.9 Hz, 1H), 6.25 (d, *J*=18.8 Hz, 2H), 5.76 (s, 1H), 5.28 (s, 1H), 4.26 (d, *J*=24.6 Hz, 3H), 3.47 (d, *J*=13.8 Hz, 1H), 2.79 (s, 2H), 2.28 (s, 6H), 2.20-1.67 (m, 4H), 1.39 (s, 1H), 1.08 (d, *J*=7.0 Hz, 3H). | 521 |

(continued)

| Compound | Target molecular structure | Separation conditions/$^1$H NMR/ | LC-MS [M+H]$^+$ |
|---|---|---|---|
| **P16** | 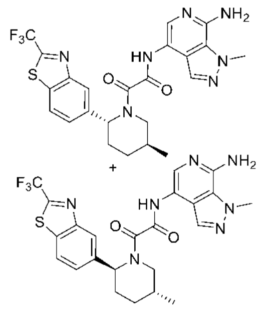 + | $^1$H NMR (400 MHz, DMSO-$d_6$) $\delta$ 10.77 (d, $J$=44.7 Hz, 1H), 8.38 (dd, $J$=15.3, 8.5 Hz, 1H), 8.26 (d, $J$=21.3 Hz, 1H), 7.93 (d, $J$=37.3 Hz, 1H), 7.80-7.64 (m, 2H), 6.23 (d, $J$=24.0 Hz, 2H), 5.56 (d, $J$=179.3 Hz, 1H), 4.26 (d, $J$=28.0 Hz, 4H), 3.51 (d, $J$=13.6 Hz, 1H), 2.36 (d, $J$=16.3 Hz, 1H), 2.18 (s, 1H), 1.91 (s, 1H), 1.73 (s, 1H), 1.40 (s, 1H), 1.09 (d, $J$=7.0 Hz, 3H). | 518 |
| **P17** | 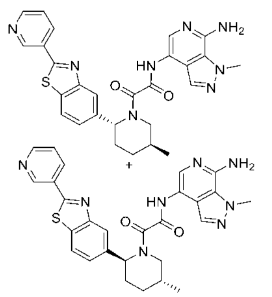 + | $^1$H NMR (400 MHz, DMSO-$d_6$) $\delta$ 10.82 (d, $J$=33.9 Hz, 1H), 9.29 (d, $J$=6.7 Hz, 1H), 8.77 (d, $J$=4.7 Hz, 1H), 8.47 (t, $J$=10.1 Hz, 1H), 8.29-8.11 (m, 2H), 8.03 (d, $J$=28.1 Hz, 1H), 7.85 (d, $J$=41.1 Hz, 1H), 7.67-7.58 (m, 1H), 7.50 (d, $J$=8.5 Hz, 1H), 6.31 (s, 2H), 5.55 (d, $J$=191.3 Hz, 1H), 4.26 (d, $J$=32.8 Hz, 3H), 3.55-3.34 (m, 2H), 2.90 (d, $J$=13.0 Hz, 1H), 2.38 (d, $J$=16.2 Hz, 1H), 2.22 (d, $J$=35.6 Hz, 1H), 1.92 (s, 1H), 1.77 (d, $J$=13.2 Hz, 1H), 1.65-1.23 (m, 3H), 1.16-0.93 (m, 3H), 0.94-0.83 (m, 1H). | 527 |
| **P18** | 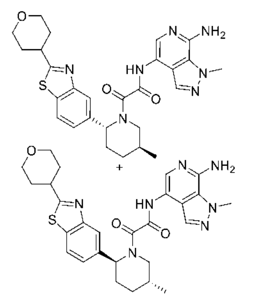 + | $^1$H NMR (400 MHz, Methanol-$d_4$) $\delta$ 8.03-7.67 (m, 4H), 7.42 (s, 1H), 5.66 (d, $J$=145.1 Hz, 1H), 4.41-4.23 (m, 3H), 4.03 (d, $J$=11.5 Hz, 2H), 3.75 (d, $J$=13.9 Hz, 1H), 3.67-3.51 (m, 2H), 3.48-3.33 (m, 2H), 2.31 (s, 2H), 2.15-1.83 (m, 6H), 1.44 (d, $J$=13.0 Hz, 1H), 1.20-1.08 (m, 3H). | 534 |
| **P19a** | 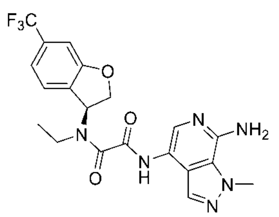 | $^1$H NMR (400 MHz, DMSO-$d_6$) $\delta$ 10.79 (d, $J$=53.9 Hz, 1H), 8.19-7.87 (m, 2H), 7.65 (dd, $J$=65.9, 7.8 Hz, 1H), 7.39-7.23 (m, 2H), 6.61 (s, 2H), 5.96-5.85 (m, 1H), 4.94 4.58 (m, 2H), 4.29 (s, 3H), 3.47-3.04 (m, 2H), 1.08-0.90 (m, 3H). | 449 |
| **P19b** | 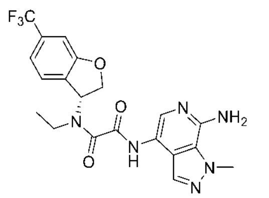 | $^1$H NMR (400 MHz, DMSO-$d_6$) $\delta$ 10.73 (d, $J$=54.6 Hz, 1H), 8.12-7.77 (m, 2H), 7.75-7.48 (m, 1H), 7.31 (ddt, $J$=20.5, 15.5, 6.5 Hz, 2H), 6.44-6.12 (m, 2H), 6.00-5.81 (m, 1H), 4.98-4.58 (m, 2H), 4.29 (d, $J$=6.5 Hz, 3H), 3.56-3.38 (m, 1H), 3.12 (ddd, $J$=49.6, 13.9, 7.1 Hz, 1H), 0.98 (dt, $J$=62.3, 6.8 Hz, 3H). | 449 |
| **P20a** | 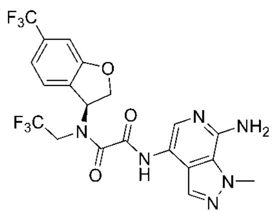 | $^1$H NMR (400 MHz, DMSO-$d_6$) $\delta$ 10.81 (s, 1H), 8.08 (d, $J$=75.4 Hz, 1H), 7.84-7.50 (m, 2H), 7.30 (t, $J$=8.6 Hz, 1H), 7.21 (d, $J$=14.4 Hz, 1H), 6.29 (d, $J$=9.5 Hz, 2H), 6.01-5.63 (m, 1H), 4.86 (q, $J$=10.1 Hz, 1H), 4.80-4.58 (m, 2H), 4.27 (d, $J$=2.6 Hz, 3H), 4.09 (q, $J$=12.6, 10.0 Hz, 1H). | 503 |

(continued)

| Compound | Target molecular structure | Separation conditions/$^1$H NMR/ | LC-MS [M+H]$^+$ |
|---|---|---|---|
| **P20b** | 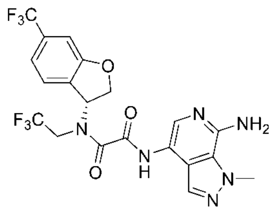 | $^1$H NMR (400 MHz, DMSO-$d_6$) $\delta$ 10.84 (s, 1H), 8.08 (d, *J*=52.3 Hz, 1H), 7.82 (s, 1H), 7.69-7.50 (m, 1H), 7.29 (d, *J*=8.0 Hz, 1H), 7.20 (d, *J*=10.3 Hz, 1H), 6.49 (s, 2H), 5.82 (d, *J*=125.2 Hz, 1H), 4.95-4.62 (m, 3H), 4.28 (s, 3H), 4.09 (s, 1H). | 503 |
| **P21a** | 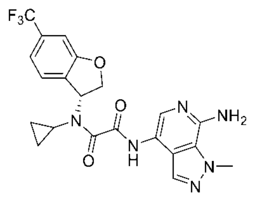 | $^1$H NMR (400 MHz, DMSO-$d_6$) $\delta$ 10.52 (s, 1H), 7.94 (s, 1H), 7.83 (s, 1H), 7.58 (d, *J*=7.8 Hz, 1H), 7.31 (d, *J*=7.9 Hz, 1H), 7.24 (s, 1H), 6.25 (s, 2H), 5.93 (dd, *J*=9.5, 4.2 Hz, 1H), 4.85 (t, *J*=9.9 Hz, 1H), 4.67 (dd, *J*=10.3, 4.4 Hz, 1H), 4.28 (s, 3H), 2.79 (p, *J*=3.2 Hz, 1H), 0.72-0.58 (m, 2H), 0.56-0.40 (m, 2H). | 461 |
| **P21b** | 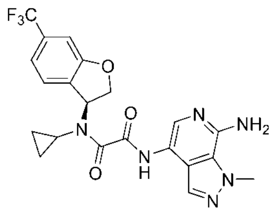 | $^1$H NMR (400 MHz, DMSO-$d_6$) $\delta$ 10.52 (s, 1H), 7.94 (s, 1H), 7.83 (s, 1H), 7.58 (d, *J*=7.8 Hz, 1H), 7.31 (dd, *J*=8.2, 1.6 Hz, 1H), 7.24 (d, *J*=1.6 Hz, 1H), 6.25 (s, 2H), 5.93 (dd, *J*=9.5, 4.3 Hz, 1H), 4.85 (t, *J*=9.9 Hz, 1H), 4.67 (dd, *J*=10.2, 4.4 Hz, 1H), 4.28 (s, 3H), 2.82-2.75 (m, 1H), 0.65 (dddd, *J*=15.7, 11.2, 6.4, 2.2 Hz, 2H), 0.55-0.41 (m, 2H). | 461 |
| **P22** | 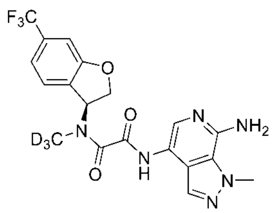 | $^1$H NMR (400 MHz, DMSO-$d_6$) $\delta$ 10.81 (d, *J*=59.0 Hz, 1H), 8.16-7.96 (m, 2H), 7.88-7.67 (m, 1H), 7.58-7.25 (m, 2H), 6.55 (s, 2H), 6.10 (ddd, *J*=169.8, 9.0, 4.1 Hz, 1H), 4.87-4.65 (m, 2H), 4.30 (s, 3H). | 438 |
| **P23** | 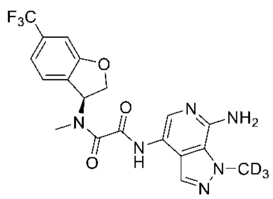 | $^1$H NMR (300 MHz, Methanol-$d_4$) $\delta$ 8.06-7.83 (m, 2H), 7.71-7.51 (m, 1H), 7.31 (d, *J*=7.9 Hz, 1H), 7.18 (dd, *J*=3.7, 1.5 Hz, 1H), 6.30 (ddd, *J*=70.9, 8.9, 3.9 Hz, 1H), 4.85-4.78 (m, 1H), 4.70 (ddd, *J*=10.8, 7.0, 3.9 Hz, 1H), 2.83 (d, *J*=52.3 Hz, 3H). | 438 |
| **P24** | 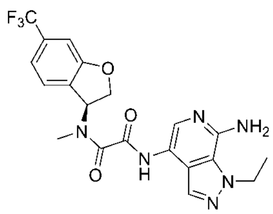 | $^1$H NMR (300 MHz, DMSO-$d_6$) $\delta$ 10.69 (d, *J*=44.0 Hz, 1H), 8.36-8.15 (m, 2H), 7.78-7.51 (m, 2H), 7.41-7.33 (m, 1H), 7.30 (d, *J*=2.3 Hz, 1H), 6.87 (s, 2H), 6.37-5.87 (m, 1H), 4.82 (dt, *J*=9.2, 5.0 Hz, 1H), 4.69 (td, *J*=10.8, 4.1 Hz, 1H), 4.54-4.40 (m, 2H), 2.76 (s, 1H), 2.60 (s, 1H), 1.51 (td, *J*=7.3, 3.1 Hz, 3H). | 449 |
| **P25** | 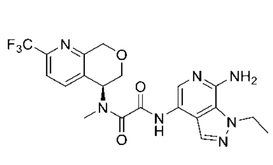 | $^1$H NMR (300 MHz, DMSO-$d_6$) $\delta$ 10.71 (d, *J*=38.4 Hz, 1H), 8.35-8.12 (m, 3H), 8.06-7.85 (m, 1H), 7.67 (d, *J*=30.7 Hz, 1H), 6.61 (s, 2H), 5.71 (s, 1H), 5.18 (s, 1H), 4.90 (d, *J*=15.9 Hz, 1H), 4.77 (d, *J*=15.5 Hz, 1H), 4.47 (q, *J*=7.2 Hz, 2H), 4.17 (dd, *J*=14.3, 5.0 Hz, 2H), 2.80 (d, *J*=50.2 Hz, 3H), 1.51 (t, *J*=7.2 Hz, 3H). | 464 |
| **P26** | 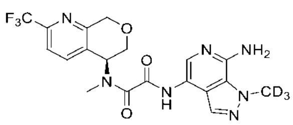 | $^1$H NMR (300 MHz, DMSO-$d_6$) $\delta$ 10.81 (d, *J*=46.7 Hz, 1H), 8.23 - 8.12 (m, 1H), 8.03-7.83 (m, 3H), 6.44 (s, 2H), 5.16 (s, 1H), 4.95-4.68 (m, 2H), 4.17 (dd, *J*=11.7, 5.0 Hz, 2H), 2.80 (d, *J*=46.0 Hz, 3H). | 453 |

(continued)

| Compound | Target molecular structure | Separation conditions/$^1$H NMR/ | LC-MS $[M+H]^+$ |
|---|---|---|---|
| **P27** | 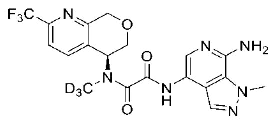 | $^1$H NMR (400 MHz, DMSO-$d_6$) $\delta$ 10.79 (d, *J*=61.9 Hz, 1H), 8.23 - 7.82 (m, 4H), 6.28 (d, *J*=3.3 Hz, 2H), 5.43 (dt, *J*=222.3, 5.1 Hz, 1H), 4.94-4.68 (m, 2H), 4.28 (d, *J*=5.9 Hz, 3H), 4.21-4.12 (m, 2H). | 453 |
| **P28a** | 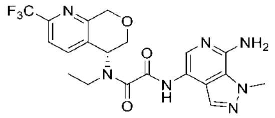 | $^1$H NMR (300 MHz, DMSO-$d_6$) $\delta$ 10.76 (d, *J*=48.9 Hz, 1H), 8.29-7.79 (m, 4H), 6.27 (d, *J*=3.5 Hz, 2H), 5.52-5.10 (m, 1H), 4.95-4.67 (m, 2H), 4.28 (d, *J*=3.8 Hz, 3H), 4.19 (t, *J*=5.5 Hz, 2H), 3.51 (ddd, *J*=43.3, 14.3, 7.1 Hz, 1H), 3.01 (dq, *J*=13.7, 6.7 Hz, 1H), 1.09 (dt, *J*=32.7, 6.9 Hz, 3H). | 464 |
| **P28b** | 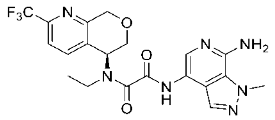 | $^1$H NMR (300 MHz, DMSO-$d_6$, ppm) $\delta$ 10.76 (d, *J*=49.0 Hz, 1H), 8.29-7.75 (m, 4H), 6.27 (d, *J*=3.5 Hz, 2H), 5.30 (dt, *J*=98.0, 5.4 Hz, 1H), 5.01-4.68 (m, 2H), 4.28 (d, *J*=3.8 Hz, 3H), 4.24-4.13 (m, 2H), 3.70-3.41 (m, 1H), 3.01 (dq, *J*=13.7, 6.8 Hz, 1H), 1.09 (dt, *J*=32.6, 6.9 Hz, 3H). | 464 |
| **P29** | 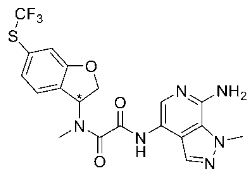 | $^1$H NMR (300 MHz, DMSO-$d_6$) $\delta$ 10.77 (d, *J*=43.0 Hz, 1H), 8.01 (d, *J*=31.4 Hz, 2H), 7.56 (dd, *J*=39.6, 7.8 Hz, 1H), 7.40-7.33 (m, 1H), 7.31-7.26 (m, 1H), 6.49-5.87 (m, 3H), 4.86-4.65 (m, 2H), 4.29 (s, 3H), 2.67 (d, *J*=42.3 Hz, 3H). | 467 |
| **P29a** | 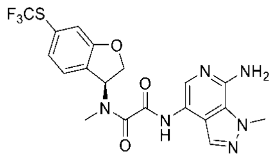 | $^1$H NMR (400 MHz, DMSO-$d_6$) $\delta$ 10.73 (d, *J*=54.1 Hz, 1H), 8.04-7.80 (m, 2H), 7.56 (dd, *J*=52.5, 7.8 Hz, 1H), 7.37-7.31 (m, 1H), 7.27 (d, *J*=6.0 Hz, 1H), 6.31-5.85 (m, 1H), 6.24 (s, 2H), 4.84-4.64 (m, 2H), 4.27 (s, 3H), 2.67 (d, *J*=56 Hz, 3H). | 467 |
| **P30** | 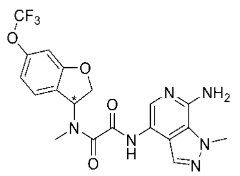 | $^1$H NMR (300 MHz, DMSO-$d_6$) $\delta$ 10.75 (d, *J*=44.3 Hz, 1H), 8.00 (q, *J*=26.3, 25.0 Hz, 2H), 7.50 (dd, *J*=39.8, 8.0 Hz, 1H), 6.98 (d, *J*=5.2 Hz, 2H), 6.26 (s, 2H), 5.80 (d, *J*=7.3 Hz, 1H), 4.89-4.57 (m, 2H), 4.28 (s, 3H), 2.67 (d, *J*=40.0 Hz, 3H). | 451 |
| **P30a** | 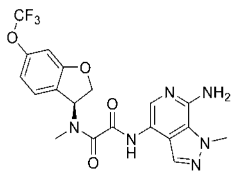 | $^1$H NMR (300 MHz, DMSO-$d_6$) $\delta$ 10.71 (d, *J*=44.4 Hz, 1H), 8.08-7.80 (m, 2H), 7.50 (dd, *J*=40.1, 8.3 Hz, 1H), 7.05-6.92 (m, 2H), 6.25 (s, 2H), 5.81 (dd, *J*=8.6, 3.7 Hz, 1H), 4.91-4.63 (m, 2H), 4.28 (s, 3H), 2.67 (d, *J*=40.9 Hz, 3H). | 451 |
| **P30b** | 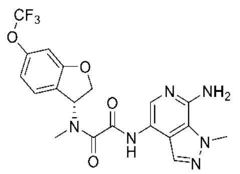 | $^1$H NMR (300 MHz, DMSO-$d_6$) $\delta$ 10.71 (d, J=44.9 Hz, 1H), 8.06-7.80 (m, 2H), 7.50 (dd, *J*=40.1, 8.3 Hz, 1H), 7.05-6.91 (m, 2H), 6.25 (s, 2H), 5.81 (dd, *J*=8.6, 3.8 Hz, 1H), 4.90-4.60 (m, 2H), 4.27 (s, 3H), 2.67 (d, *J*=40.8 Hz, 3H). | 451 |
| **P31** | 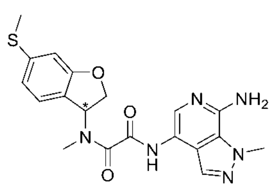 | $^1$H NMR (400 MHz, DMSO-$d_6$) $\delta$ 10.75 (d, *J*=61.1 Hz, 1H), 8.10 (d, *J*=41.6 Hz, 1H), 7.99-7.83 (m, 1H), 7.31 (dd, *J=52.1,* 7.9 Hz, 1H), 6.87 (dt, *J*=8.0, 2.2 Hz, 1H), 6.82 (dd, *J*=9.1, 1.6 Hz, 1H), 6.39 (d, *J*=8.9 Hz, 2H), 5.94 (ddd, *J*=200.8, 8.7, 3.5 Hz, 1H), 4.77-4.65 (m, 1H), 4.58 (ddd, *J*=16.2, 10.5, 3.7 Hz, 1H), 4.28 (s, 3H), 2.64 (d, *J*=47.0 Hz, 3H), 2.48 (d, *J*=3.7 Hz, 3H). | 413 |

(continued)

| Compound | Target molecular structure | Separation conditions/$^1$H NMR/ | LC-MS [M+H]$^+$ |
|---|---|---|---|
| **P32** | 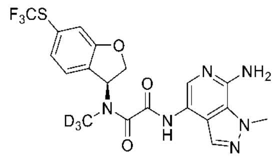 | $^1$H NMR (300 MHz, DMSO-$d_6$) $\delta$ 10.72 (d, $J$=43.3 Hz, 1H), 8.04-7.80 (m, 2H), 7.56 (dd, $J$=40.1, 7.7 Hz, 1H), 7.40-7.25 (m, 2H), 6.32-5.83 (m, 3H), 4.87-4.60 (m, 2H), 4.28 (s, 3H). $^{19}$F NMR (282 MHz, DMSO-$d_6$, ppm) $\delta$ -41.90, -41.92. | 470 |
| **P33** | 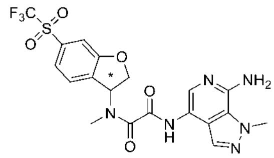 | $^1$H NMR (400 MHz, DMSO-$d_6$) $\delta$ 10.77 (d, $J$=57.3 Hz, 1H), 8.06-7.90 (m, 2H), 7.84-7.76 (m, 2H), 7.63 (d, $J$=1.7 Hz, 1H), 6.40-6.00 (m, 3H), 4.98-4.71 (m, 2H), 4.28 (s, 3H), 2.73 (d, $J$=77.6 Hz, 3H). | 499 |
| **A1** | 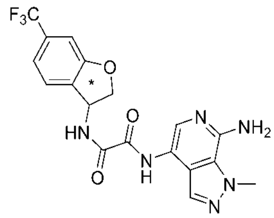 | $^1$H NMR (300 MHz, DMSO-$d_6$) $\delta$ 10.59 (s, 1H), 9.64 (d, $J$=7.9 Hz, 1H), 7.85 (s, 1H), 7.69 (s, 1H), 7.53 (d, $J$=7.8 Hz, 1H), 7.30-7.16 (m, 2H), 6.25 (s, 2H), 5.74 (q, $J$=7.7 Hz, 1H), 4.82 (t, $J$=9.5 Hz, 1H), 4.59 (dd, $J$=9.6, 5.6 Hz, 1H), 4.27 (s, 3H). | 421 |
| **H1a** | 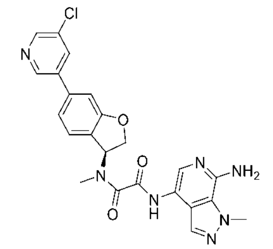 | $^1$H NMR (400 MHz, DMSO-$d_6$) $\delta$ 10.76 (d, $J$=60.4 Hz, 1H), 8.89 (t, $J$=2.4 Hz, 1H), 8.63 (d, $J$=2.4 Hz, 1H), 8.27 (t, $J$=2.3 Hz, 1H), 8.07-7.82 (m, 2H), 7.64-7.35 (m, 3H), 6.27 (s, 2H), 5.83 (dd, $J$=8.7, 3.7 Hz, 1H), 4.84-4.60 (m, 2H), 4.29 (s, 3H), 2.70 (d, $J$=*52.0* Hz, 3H). | 478 |
| **H1b** | 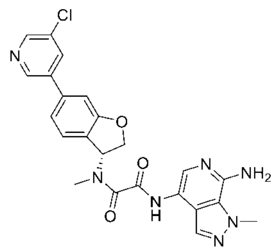 | $^1$H NMR (400 MHz, DMSO-$d_6$) $\delta$ 10.75 (d, $J$=62.4 Hz, 1H), 8.89 (t, $J$=2.3 Hz, 1H), 8.64 (t, $J$=2.1 Hz, 1H), 8.27 (t, $J$=2.2 Hz, 1H), 8.16-7.83 (m, 2H), 7.63-7.33 (m, 3H), 6.28 (s, 2H), 5.83 (dd, $J$=8.8, 3.8 Hz, 1H), 4.85-4.60 (m, 2H), 4.29 (s, 3H), 2.70 (d, $J$=52.0 Hz, 3H). | 478 |
| **H2a** | 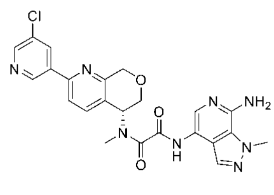 | $^1$H NMR (300 MHz, DMSO-$d_6$) $\delta$ 10.81 (d, $J$=45.7 Hz, 1H), 9.26 (t, $J$=2.3 Hz, 1H), 8.72 (t, $J$=2.0 Hz, 1H), 8.56 (q, $J$=2.3 Hz, 1H), 8.23 - 8.04 (m, 2H), 8.03-7.94 (m, 1H), 7.87 (t, $J$=8.6 Hz, 1H), 6.31 (s, 2H), 5.70 (s, 1H), 4.97-4.85 (m, 1H), 4.75 (t, $J$=16.4 Hz, 1H), 4.28 (d, $J$=4.5 Hz, 3H), 4.19 (d, $J$=4.8 Hz, 1H), 4.14 (d, $J$=4.5 Hz, 1H), 2.82 (d, $J$=38.3 Hz, 3H). | 493 |
| **H2b** | 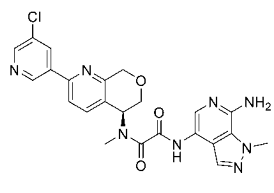 | $^1$H NMR (300 MHz, DMSO-$d_6$) $\delta$ 10.85 (d, $J$=45.4 Hz, 1H), 9.25 (t, $J$=2.4 Hz, 1H), 8.72 (t, $J$=2.1 Hz, 1H), 8.55 (q, $J$=2.3 Hz, 1H), 8.24-8.12 (m, 1H), 8.06 (d, $J$=7.9 Hz, 1H), 7.99 (s, 1H), 7.86 (d, $J$=8.2 Hz, 1H), 6.57 (s, 2H), 5.09 (s, 1H), 4.90 (dd, $J$=15.9, 4.7 Hz, 1H), 4.74 (t, $J$=16.3 Hz, 1H), 4.29 (d, $J$=4.0 Hz, 3H), 4.16 (dd, $J$=14.2, 4.8 Hz, 2H), 2.82 (d, $J$=37.6 Hz, 3H). | 493 |

**Example 3**

MTAP-deficient cells are sensitive to PRMT5-MTA inhibitors due to the accumulation of MTA, whereas *MTAP* wild-type (*MTAP* normal) cells have no MTA accumulation effect and are independent of PRMT5. By testing activity in both, it was shown that the molecules of the present invention have an inhibitory and selective effect on PRMT5 at the cellular level.

HCT116 wild-type and MTAP-deficient cells were cultured in McCoy's 5A medium containing 10% FBS and 1% penicillin-streptomycin, and cultured in a 37°C, 5% $CO_2$ constant temperature incubator. 40 μL of cell suspension was introduced into each well of a 384-well microplate. 40 nL of different concentrations of test compounds were added to each well using an Echo, before culturing in a 37°C, 5% $CO_2$ constant temperature incubator for 7-10 days. 40 μL of CTG solution (Promega, Cat No. G7573) was added to each well and incubated in a 37°C, 5% $CO_2$ constant temperature incubator for 30 minutes in the dark. The luminescence value was read using an Envision multi-function microplate reader (Perkin Elmer, catalog no. Envision 2104), in which the light signal is proportional to the amount of ATP in the system, and the ATP content directly characterizes the number of viable cells in the system.

Calculation of $IC_{50}$:

Y=lower platform signal + (upper platform signal - lower platform signal) / (1+10^((Log$IC_{50}$-X)×Hill slope))

X: log value of compound concentration
Y: Inhibition rate (%)

Table 1: 2D antiproliferative effect of different compounds on *HTC116-MTAP del* and wild type colorectal cancer HCT-116 cell lines.

| **Compound** | **HCT116-*MTAPdel*/$IC_{50}$/nM** | **HCT116-*MTAPwt*/$IC_{50}$/nM** | **HCT116WT *IMTAPdel* selectivity** |
|---|---|---|---|
| **P1** | 9 | 196 | 22 |
| **P2** | 32 | 2680 | 84 |
| **P2a** | 5 | 1079 | 216 |
| **P2b** | 118 | >10000 | 85 |
| **P3** | 13 | 827 | 64 |
| **P3a** | 666 | >10000 | 15 |
| **P3b** | 2 | 275 | 138 |
| **P4** | 813 | >10000 | >12 |
| **P5** | 4 | 36 | 9 |
| **P6** | 18 | 1297 | 72 |
| **P7** | 56 | 2517 | 45 |
| **P8** | 4 | 161 | 40 |
| **P9** | 24 | 1423 | 59 |
| **P9a** | 12 | 3998 | 333 |
| **P9b** | 883 | >10000 | >11 |
| **P10** | 10 | 800 | 80 |
| **P11** | 15 | 855 | 57 |
| **P11a** | 1907 | >10000 | >5 |
| **P11b** | 8 | 427 | 53 |

(continued)

| Compound | HCT116-*MTAPdel*/$IC_{50}$/nM | HCT116-*MTAPwt*/$IC_{50}$/nM | HCT116WT /*MTAPdel* selectivity |
|---|---|---|---|
| **P12** | 5 | 607 | 121 |
| **P13a** | 616 | >10000 | >16 |
| **P13b** | 2 | 506 | 253 |
| **P14** | 11 | 295 | 27 |
| **P15** | 6 | 203 | 34 |
| **P16** | 11 | 891 | 81 |
| **P17** | 8 | 606 | 76 |
| **P18** | 14 | 900 | 64 |
| **P19a** | 6 | 1084 | 181 |
| **P19b** | 261 | 3862 | 15 |
| **P20a** | 28 | 6018 | 215 |
| **P20b** | 883 | >10000 | 11 |
| **P21a** | 978 | >10000 | 10 |
| **P21b** | 6 | 847 | 141 |
| **P22** | 6 | 961 | 160 |
| **P23** | 17 | 1709 | 101 |
| **P24** | 2409 | >10000 | >4 |
| **P25** | 1255 | >10000 | >8 |
| **P26** | 12 | 4554 | 380 |
| **P27** | 15 | 2109 | 141 |
| **P28a** | 986 | 2340 | 2 |
| **P28** | 10 | 1188 | 119 |
| **P29** | 20 | 3296 | 165 |
| **P29a** | 9 | 2352 | 261 |
| **P30** | 13 | 3135 | 241 |
| **P30a** | 7 | 1546 | 221 |
| **P30b** | 191 | >10000 | >52 |
| **P31** | 21 | 3585 | 171 |
| **P32** | 9 | 1748 | 194 |
| **P33** | 9 | 1458 | 162 |
| **H1a** | 9 | 2319 | 258 |
| **H1b** | 300 | >10000 | >33 |
| **H2a** | 224 | >10000 | >45 |
| **H2b** | 4 | 1231 | 308 |
| **A1** | >10000 | >10000 | N.D. |
| **AM9747** | 7 | 237 | 34 |
| **GSK3326595** | 17 | 72 | 4 |
| **TNG908** | 43 | 1186 | 27 |
| N.D.= Not detected | | | |

The above experimental results show that the superior compounds of the present invention have a significant anti-proliferative effect on *MTAP*-deficient tumor cells by inhibiting PRMT5, but only weakly inhibit the wild-type, and would be expected to offer better safety. The activity and selectivity of some molecules outperform those of the control molecules **AM9747, TNG908** and **GSK3326595.**

The molecules of the present invention have activity that is comparable to or better than **AM9747,** but much higher selectivity than **AM9747,** and much higher activity and selectivity than **TNG908.**

| **AM9747** structure: | **GSK3326595** structure: | **TNG908** structure: |
|---|---|---|
| 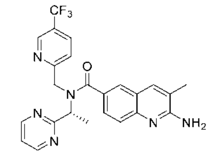 | 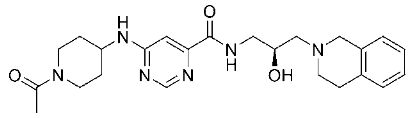 | 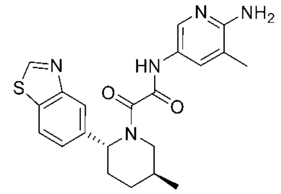 |

A **comparison of P2a, P22, P29a and P32 with the control molecule A1 proves that alkyl substitution (especially methyl substitution or deuterated methyl substitution) of this series of molecules is crucial for activity; therefore deuteration is conducive to improvements in metabolism, allowing activity to be maintained for longer.**

**Example 4**

**In-cell Arginine Symmetric Dimethylation Inhibition Analysis (In-cell western (ICW) assay)**

HCT116 wild-type or MTAP-deficient cells (HTC116-*MTAP del*) cultured in RPMI 1640 medium containing 10% fetal bovine serum and 1% penicillin-streptomycin were seeded in 384-well microplates in a volume of 30 μL and incubated overnight at 37°C, 5% $CO_2$. 60 nL of different concentrations of compounds were added to each well using an Echo, and incubated at 37°C, 5% $CO_2$ for 96 hours.

Next, 50 μL of 4% paraformaldehyde solution was added to each well and incubated at room temperature for 20 minutes to fix the cells. The solution in the wells was removed, and each well was rinsed four times with PBS solution containing 0.1% Tween 20 (PBST). To each well was then added 30 μL of ice-cold methanol. After standing at -20°C for 10 minutes, the methanol was removed and the wells washed four times with PBST. 30 μL of Odyssey blocking solution containing 0.5% Tween 20 was added to each well and shaken for 2 hours at room temperature. The blocking solution was removed and 30 μL of primary antibody (Symmetric Di-Methyl Arginine Motif [sdma-RG] MultiMab™ Rabbit mAb mix) diluted at 1:500 in Odyssey blocking solution containing 0.5% Tween 20 was added to each well. The plates were incubated overnight at 4°C, before removing the primary antibody and rinsing each well four times with PBST, 5 minutes per rinse. Secondary antibody (goat anti-rabbit IRDye 800CW, 1:800) and nuclear stain (DRAQ5, 1:10000) diluted in Odyssey blocking solution containing 0.5% Tween 20 were then added to each well before incubating at room temperature for 2 hours in the dark. The secondary antibody was removed, and the wells rinsed four times with PBST.

The sdme-RG and DRAQ5 signals were scanned at 800 nm and 700 nm respectively on a Li-Cor Odyssey instrument and the signal values recorded. The sdme-RG/DRAQ5 ratio was used to calculate the percentage inhibition of arginine symmetric dimethylation (SDMA), and the $IC_{50}$ value calculated using GraphPad Prism software.

Table 1: Arginine symmetric methylation inhibitory effect of different compounds in HTC116-*MTAP del* and wild type colorectal cancer HCT-116 cell lines

| **Compound** | **SDMA/HCT116-*MTAPdel*/$IC_{50}$/nM** | **SDMA/HCT116-*MTAPwt*/$IC_{50}$/nM** | **Selectivity *wt*/*MTAPdel*** |
|---|---|---|---|
| **P2a** | 0.5 | 1527 | 3054 |
| **P3b** | 0.6 | 578 | 963 |
| **P9a** | 0.9 | 2450 | 2722 |
| **P22** | 0.2 | 298 | 1490 |
| **P29a** | 0.4 | 268 | 670 |
| **P32** | 0.3 | 256 | 853 |
| **TNG908** | 2.6 | 2211 | 850 |

(continued)

| Compound | SDMA/HCT116-*MTAPdel*/$IC_{50}$/nM | SDMA/HCT116-*MTAPwt*/$IC_{50}$/nM | Selectivity *wt*/*MTAPdel* |
|---|---|---|---|
| **AM9747** | 0.6 | 332 | 553 |
| N.D.= Not detected | | | |

The above experimental results show that the superior compounds of the present invention have a significant inhibitory effect on the symmetric methylation of arginine in MTAP-deficient tumor cells by inhibiting PRMT5, while only weakly inhibiting wild-type cells.

The molecules of the present invention have activity that is comparable to or better than **AM9747,** but much higher selectivity than **AM9747** and much higher activity than **TNG908.**

**Example 5:**

Liver microsomal stability assay, conducted as follows:

The compounds of the present invention were subjected to liver microsomal stability testing. The compounds to be tested were co-incubated with liver microsomes of different species with or without the addition of NADPH. The final concentration of the test compounds in the test system was 1 μM, the final concentration of NADPH was 1 mM, and the final concentration of liver microsomes was 0.5 mg/mL. The compound concentrations in the incubation supernatant at different time points over a 60 minute period were detected, and pharmacokinetic parameters (e.g., clearance $Cl_{int}$) calculated.

The results showed that the molecules of the present invention have good metabolic stability, particularly in the human body. Some molecules have a lower rate of clearance in human liver microsome metabolism than **AMG9747,** and are thus metabolized more slowly in the body.

| Compound | Human $Cl_{int}$/(mL/min/kg) | Mouse $Cl_{int}$/(mL/min/kg) |
|---|---|---|
| **P1** | 18.0 | 177 |
| **P2** | 13.0 | N.D. |
| **P2a** | 7.4 | 55 |
| **P3** | 23.9 | N.D. |
| **P5** | 10.7 | N.D. |
| **P9** | 5.0 | N.D. |
| **P9a** | 2.1 | 10.0 |
| **P11** | 16.5 | N.D. |
| **P15** | 9.7 | N.D. |
| **P19a** | 20.0 | 110 |
| **P20a** | 19.2 | N.D. |
| **P22** | 7.8 | 46.2 |
| **P28** | 4.8 | N.D. |
| **P29** | 25.9 | N.D. |
| **P29a** | 49.7 | N.D. |
| **P30** | 9.4 | N.D. |
| **P32** | 32 | 242 |
| **H2b** | 21.3 | N.D. |
| **AM9747** | 27.1 | 169 |
| N.D. = Not detected | | |

The above results indicate that the molecules of the present invention have good metabolic stability in liver microsomes. The deuterated compounds **P22** and **P32** have comparable or improved metabolism with respect to the non-deuterated molecules **P2a** or **P29a,** and would be expected to exhibit good *in vivo* exposure when administered *in vivo.*

**Example 6:**

Membrane permeability evaluation testing: Caco-2 assay

The membrane permeability of the molecules of the invention was evaluated. Samples were analyzed by LC-MS to estimate the apparent permeability coefficient ($P_{app}$) of the compounds in Caco-2 monolayer cells, where the pH of the apical compartment is 6.5 and the pH of the basolateral compartment is 7.4. Inhibitors of efflux transporters P-gp, BCRP and MRP2 (50 μM quinidine, 30 μM benzbromarone and 20 μM sulfasalazine) can block the active efflux transport of the compounds, [generating] data for calculating apparent permeability ($P_{app}$):

$$P_{app}=(V_A\times[drug]_{acceptor})/(Area\times Time\times[drug]_{initial,donor})$$

where $V_A$ is the volume of the receptor pores (unit:mL), Area is the surface area of the membrane (0.143 $cm^2$ for a Transwell-96 well permeable support), and time is the total transport time in seconds.

$$Efflux\ Ratio=P_{app}(B\text{-}A)/P_{app}(A\text{-}B)$$

The Caco-2 assay results obtained for the molecules of the present invention are shown below:

| Compound | Papp(A-B) ($10^{-6}$ cm/s) | $P_{app}(B\text{-}A)$ ($10^{-6}$ cm/s) | Efflux |
|---|---|---|---|
| **P2** | 13.5 | 20.5 | 1.5 |
| **P3** | 2.66 | 34.2 | 13 |
| **P9a** | 8.65 | 25.1 | 2.9 |

The above results show that the molecules of the present invention have good membrane permeability, and would be expected to achieve good tumor inhibition effects thanks to better *in vivo* pharmacokinetics.

Membrane permeability evaluation testing: MDCK-MDR1 assay

The membrane permeability of the molecules of the present invention was evaluated to predict their permeability to the brain.

The specific procedure was: 1. MDCK-MDR1 cells were pre-cultured at a density of 1.56*$10^6$ cells/mL in a 96-well plate at 37°C for 7 days. 2. The MDCKII-MDR1 plate was taken out of the incubator, rinsed twice with pre-warmed HBSS (10 mM HEPES, pH 7.4), and then incubated at 37°C for 30 minutes. 3. The stock solution of the compound to be tested was diluted with DMSO to obtain a 0.2 mM solution, and then diluted with HBSS (10 mM HEPES, pH 7.4) to obtain a 1 μM working solution. A blank control group was also diluted with DMSO to obtain a 0.2 mM solution, and then diluted with HBSS (10 mM HEPES, pH 7.4) to obtain a 1 μM working solution. The final concentration of DMSO in the culture system was 0.5%. 4. The transport rate of the drug in the apical to basolateral direction was determined: 125 μL of the 1 μM test compound solution was added to a Transwell insert (top compartment), before immediately transferring a 50 μL sample ($D_0$ sample) from the top comparment to a new 96-well plate. The receiving plate wells (basolateral compartment) were filled with 235 μL HBSS (10 mM HEPES, pH 7.4) and incubated at 37°C for 2 hours. 5. Upon completion of the incubation, 50 μL samples were transferred from the donor and acceptor sides to the wells of a new 96-well plate, before adding 4 volumes of cold acetonitrile containing an appropriate internal standard (IS). Prior to LC-MS/MS analysis, 100 μL of the supernatant was taken and mixed with an appropriate volume of ultrapure water.

$$P_{app}=(V_A\times[drug]_{acceptor})/(Area\times Time\times[drug]_{initial,donor})$$

where $V_A$ is the volume of the receptor pores (unit:mL), Area is the surface area of the membrane (0.143 $cm^2$ for a Transwell-96 well permeable support), and time is the total transport time in seconds.

$$\text{Efflux Ratio} = P_{app}(B\text{-}A)/P_{app}(A\text{-}B)$$

The MDCK-MDR1 assay results of the molecules of the present invention are shown below:

| Compound | Papp(A-B) ($10^{-6}$ cm/s) | $P_{app}(B\text{-}A)$ ($10^{-6}$ cm/s) | Efflux |
|---|---|---|---|
| **P2a** | 14.8 | 22.1 | 1.5 |
| **P22** | 14.9 | 20.6 | 1.38 |
| **P29a** | 7.6 | 13.3 | 1.75 |
| **P32** | 10.54 | 13.49 | 1.28 |
| **P28** | 10.7 | 34.0 | 3.2 |
| **P33** | 6.86 | 28.79 | 4.2 |
| **AM9747** | 12.79 | 28.29 | 2.2 |

The above results show that most of the molecules of the present invention have good membrane permeability. The deuterated compounds **P22** and **P32** have lower efflux ratios than the non-deuterated molecules **P2a or P29a,** and would be expected to give higher brain concentrations when administered *in vivo.*

**Example 7:**

Mouse pharmacokinetics testing

CD1 female mice were used as test animals and administered the compounds orally/intravenously (oral dosage was 10 mg/kg and intravenous dosage was 2 mg/kg).

Test protocol: Each oral and intravenous group consisted of three mice. In the oral administration groups, plasma samples were collected before (0 h) and after (at 0.25, 0.5, 1, 2, 4, 8 and 24 h) administration. In the intravenous groups, plasma samples were collected before (0 h) and after (at 0.083, 0.25, 0.5, 1, 2, 4, 8 and 24 h) administration. Plasma drug concentrations after oral and intravenous administration were determined by LC/MS/MS, and the collected data analyzed using AB Sciex QTRAP 6500 software. Test results are shown below:

| Compound | | P3b | P9a | P19a | P30 |
|---|---|---|---|---|---|
| *IV* (2 mg/kg) | $T_{1/2}$ (h) | 0.32 | 1.2 | 2.1 | 2.0 |
| | $C_0$ (ng/mL) | 1652 | 5653 | 1211 | 1067 |
| | $AUC_{0-24}$ (h*ng/mL) | 383 | 3993 | 1484 | 1407 |
| | Cl (mL/min/kg) | 86.6 | 8.3 | 21.5 | 23.0 |
| *PO* (10 mg/kg) | $T_{1/2}$ (h) | 1.1 | 1.1 | 6.8 | 1.5 |
| | $T_{max}$ (h) | 0.25 | 0.25 | 0.5 | 0.25 |
| | $C_{max}$ (ng/mL) | 883 | 10838 | 1100 | 1854 |
| | $AUC_{0-24}$ (h*ng/mL) | 586 | 15396 | 4387 | 5146 |
| | F (%) | 31 | 77 | 59 | 74 |

| Compound | | P2a | P22 | P32 |
|---|---|---|---|---|
| *IV* (2 mg/kg) | $T_{1/2}$ (h) | 1.2 | 2.6 | 1.4 |
| | $C_0$ (ng/mL) | 345 | 1140 | 733 |
| | $AUC_{0-24}$ (h*ng/mL) | 402 | 896 | 788 |
| | C1 (mL/min/kg) | 82.4 | 35.5 | 41.8 |

(continued)

| Compound | | P2a | P22 | P32 |
|---|---|---|---|---|
| | $T_{1/2}$ (h) | 1.2 | 2.2 | 1.7 |
| | $T_{max}$ (h) | 0.25 | 0.25 | 0.25 |
| *PO* (10 mg/kg) | $C_{max}$ (ng/mL) | 707 | 1177 | 798 |
| | $AUC_{0-24}$ (h*ng/mL) | 1379 | 2662 | 2585 |
| | F (%) | 69 | 62 | 75 |

The above test results show that the compounds of the present invention have good oral absorption effects and a high *in vivo* exposure. Due to their superior selectivity compared to reported molecules, such as **GSK3326595,** they would be expected to offer greater therapeutic effects.

In addition, a comparison of compounds **P2a** and **P22** proves that replacing the methyl group of compound **P2a** with deuterium gives **P22** a lower *in vivo* clearance rate, a longer half-life, a higher $C_{max}$, and a higher *in vivo* exposure, and thus the deuterated compound **P22** is superior to **P2a.**

Similarly, deuteration of the **P32** molecule also results in higher *in vivo* exposure.

**Example 8:**

**hERG testing**

The CHO hERG-Duo cell line stably expressing hERG channels was purchased from B'SYS GmbH and screened for monoclonal clones using the patch clamp system of Pharmaron Beijing Co., Ltd. The cells were cultured in a medium containing F12 (HAM) medium, 10% fetal bovine serum, 100 U/mL penicillin-streptomycin, 100 μg/mL hygromycin, and 100 μg/mL G418.

The membrane was depolarized to +30 mV for 4.8 seconds before returning the voltage to -50 mV for 5.2 seconds to remove inactivation and measure the deactivation tail current. The sampling interval was 15 seconds, and the peak tail current size was used to quantify the hERG current amplitude.

A blank vehicle was applied to the cells to establish a baseline. Test samples were introduced after stabilizing the hERG current for at least 5 minutes. In the presence of the test compound, the hERG current at different working concentrations was recorded for no less than 5 minutes until reaching a steady state, before capturing 5 sweeps. If a steady state was not reached within 10 minutes, the mean peak current of the last 5 sweeps was taken instead of the steady-state value. Cisapride was used as a positive control. The inhibitory effect of 5 concentrations of the test compounds on hERG current was detected in two independent tests (n=2) to determine the $IC_{50}$.

**Calculation formula:**

$$\text{Peak current inhibition} = \left(1 - \frac{\text{Peak tail current}_{\text{compound}} - \text{Peak tail current}_{\text{postive control}}}{\text{Peak tail current}_{\text{Blank vehicle}} - \text{Peak tail current}_{\text{postive control}}}\right) \times 100$$

| Test drug | $IC_{50}$/μM |
|---|---|
| **cisapride** | 0.014 |
| **P29a** | 1.5 |
| **P32** | 2.9 |

The above results show that the molecules of the present invention have a weak effect on the heart, and the deuterated compound **P32** is safer than **P29a.**

**Example 9:**

*In vivo* efficacy testing in BALB/c nude mice, conducted as follows:

NCI-H838 (*MTAPdel*) non-small cell lung adenocarcinoma tumor cells were cultured and inoculated into 6-8 week old female BALB/c nude mice (weighing about 20 g), with all mice inoculated subcutaneously. The mice were cultured in an SPF-grade environment, and all mice had free access to commercially certified standard diets. Test compounds were orally administered daily once the average tumor volume of the mice grew to about 140 $mm^3$. The control group received blank solvent (saline containing 0.1% Tween 80 and 0.5% methyl cellulose), while the test group received a drug dose of 75 mg/kg or 100 mg/kg, twice a day. Tumor volume was measured in two dimensions with calipers three times a week, and the animals were weighed every day. After 23 consecutive days of administration, the inhibition rate (TGI/100%) was calculated based on the final tumor volume. Volume calculation formula: $V=1/2a*b^2$, where *a* denotes the long diameter of the tumor, and *b* the short diameter of the tumor.

| Test drug | Dosage | Tumor volume ($mm^3$)-D1 | Tumor volume ($mm^3$)-D23 | TGI |
|---|---|---|---|---|
| Control group | 0 | 140 | 2641 | 0% |
| **P2a** | 100 mg/kg, *BID* | 140 | 180 | 97% |
| **P2a** | 75 mg/kg, *BID* | 140 | 350 | 92% |

The results show that the molecules of the present invention exhibit good *in vivo* efficacy for *MTAP*-deficient tumor cells at different doses, with low toxicity to mice.

$$TGI=[1-[\text{volume of test group (D23-D1)/volume of control group (D23-D1)}]]\times 100\%$$

**Example 10:**

*In vivo* efficacy testing in BALB/c nude mice, conducted as follows:

HUP-T4 (*MTAPdel*) human pancreatic cancer tumor cells (MEM medium with 10% fetal bovine serum) were cultured and inoculated into 6-8 week old female BALB/c nude mice (weighing about 25 g), with all mice subcutaneously inoculated. The mice were cultured in an SPF-grade environment, and all mice had free access to commercially certified standard diets. Test compounds were orally administered daily once the average tumor volume of the mice grew to about 176 $mm^3$. The control group received blank solvent (0.1% TW80+0.5% methylcellulose+99.4% saline), while the test group received a drug dose of 75 mg/kg or 50 mg/kg, twice a day. Tumor volume was measured in two dimensions with calipers three times a week, and the animals were weighed every day. After 28 consecutive days of administration, the inhibition rate (TGI/100%) was calculated based on the final tumor volume. Voume calculation formula: $V=1/2a*b^2$, where *a* denotes the long diameter of the tumor, and *b* the short diameter of the tumor.

| Test drug | Dosage | Tumor volume ($mm^3$)-D1 | Tumor volume ($mm^3$)-D28 | TGI |
|---|---|---|---|---|
| Control group | 0 | 176 | 998 | 0% |
| **P22** | 75 mg/kg, *BID* | 176 | 146 | 104% |
| **P22** | 50 mg/kg, *BID* | 176 | 158 | 102% |
| **P32** | 75 mg/kg, *BID* | 176 | 140 | 104% |
| **P32** | 50 mg/kg, *BID* | 176 | 198 | 97% |

The results indicate that the molecules of the present invention have good *in vivo* efficacy for *MTAP*-deficient pancreatic cancer cells and can provoke tumor regression.

$$TGI=[1-[\text{volume of test group (D28-D1)/volume of control group (D28-D1)}]]\times 100\%$$

**Claims**

**1.** A compound of formula (I), or a pharmaceutically acceptable salt, isotopic variant, tautomer or stereoisomer thereof:

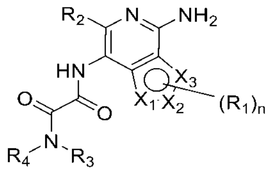

(I)

wherein,

$X_1$, $X_2$ and $X_3$ are each independently selected from -CH=, -O-, -S-, -N= or -NH-, and at least one of $X_1$, $X_2$ and $X_3$ is -N= or -NH-, the circle indicating aromaticity;
$R_1$ is selected from H, $C_{1-6}$ alkyl, deuterated $C_{1-6}$ alkyl, $C_{3-6}$ cycloalkyl or $C_{1-6}$ haloalkyl;
$R_2$ is selected from H, $C_{1-6}$ alkyl or halogen;
$R_3$ and $R_4$ are independently selected from H, $C_{1-6}$ alkyl, $C_{3-6}$ cycloalkyl or 4-10 membered heterocyclyl, wherein the $C_{1-6}$ alkyl, $C_{3-6}$ cycloalkyl or 4-10 membered heterocyclyl in $R_3$ or $R_4$ is each optionally substituted by 1 or 2 $R_5$, wherein $R_5$ is selected from hydrogen, deuterium, halogen, $C_{1-6}$ alkyl, $C_{1-6}$ alkoxy, $C_{1-6}$ haloalkyl, $C_{1-6}$ haloalkoxy, CN, $SF_5$, $C_{3-6}$ cycloalkyl, $-S(O)_2CF_3$, $-OCF_3$, $-SCF_3$, $-SCH_3$ or 5-10 membered heteroaryl; the 5-10 membered heteroaryl is optionally further substituted by $R_6$, wherein $R_6$ is selected from H, CN, halogen, $C_{1-6}$ alkyl, $C_{1-6}$ haloalkyl, $SF_5$, $OR_a$ or 5-6 membered heteroaryl, and the 5-6 membered heteroaryl is optionally substituted by 1, 2 or 3 $R_z$;
alternatively, $R_3$, $R_4$ and the nitrogen atom to which they are attached together form a 4-10 membered heterocyclic group, the 4-10 membered heterocyclic group comprising 0 or 1 additional heteroatom selected from oxygen, nitrogen or sulfur, and being optionally substituted with 1, 2, 3 or 4 $R_x$;
$R_x$ is selected from $C_{1-6}$ alkyl, $C_{1-6}$ haloalkyl, phenyl or 5-10 membered heteroaryl, wherein the phenyl or 5-10 membered heteroaryl is optionally substituted with 1, 2 or 3 $R_y$;
$R_y$ is selected from halogen, $C_{1-6}$ alkyl, $C_{1-6}$ haloalkyl, $SF_5$, CN, $OR_a$, $C_{1-6}$ alkoxy, $-NR_{z1}R_{z2}$, $NR_{z1}R_{z2}$-$C_{1-6}$ alkyl-, $C_{3-6}$ cycloalkyl, 4-12 membered heterocyclyl or 5-10 membered heteroaryl, wherein the 4-12 membered heterocyclyl or 5-10 membered heteroaryl is optionally substituted by 1, 2 or 3 $R_z$;
$R_z$ is selected from $C_{1-6}$ alkyl, halogen, CN, $C_{1-6}$ haloalkyl or $C_{1-6}$ alkoxy;
$R_a$ is selected from H, $C_{1-6}$ alkyl or $C_{1-6}$ haloalkyl;
$R_{z1}$ and $R_{z2}$ are each independently selected from H or $C_{1-6}$ alkyl; and
*n is 0,* 1 or 2.

**2.** A compound of claim 1, or a pharmaceutically acceptable salt, isotopic variant, tautomer or stereoisomer thereof, which is a compound of formula (IIa) or formula (IIb):

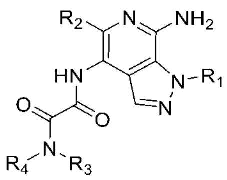

(IIa) or

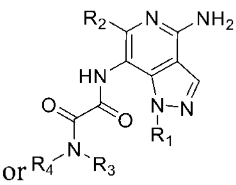

(IIb)

wherein,

$R_1$ is selected from H, $C_{1-6}$ alkyl, deuterated $C_{1-6}$ alkyl, $C_{3-6}$ cycloalkyl or $C_{1-6}$ haloalkyl;
$R_2$ is selected from H, $C_{1-6}$ alkyl or halogen;
$R_3$ and $R_4$ are independently selected from H, $C_{1-6}$ alkyl, $C_{3-6}$ cycloalkyl or 4-10 membered heterocyclyl, wherein the $C_{1-6}$ alkyl, $C_{3-6}$ cycloalkyl or 4-10 membered heterocyclyl in $R_3$ or $R_4$ is each optionally substituted by 1 or 2 $R_5$, wherein $R_5$ is selected from hydrogen, deuterium, halogen, $C_{1-6}$ alkyl, $C_{1-6}$ alkoxy, $C_{1-6}$ haloalkyl, $C_{1-6}$ haloalkoxy, CN, $SF_5$, $C_{3-6}$ cycloalkyl, $-S(O)_2CF_3$, $-OCF_3$, $-SCF_3$, $-SCH_3$ or 5-10 membered heteroaryl; the 5-10 membered heteroaryl is optionally further substituted by $R_6$, wherein $R_6$ is selected from H, CN, halogen, $C_{1-6}$ alkyl, $C_{1-6}$ haloalkyl, $SF_5$, $OR_a$ or 5-6 membered heteroaryl, the 5-6 membered heteroaryl being optionally substituted by 1, 2 or 3 $R_z$;
alternatively, $R_3$, $R_4$ and the nitrogen atom to which they are attached together form a 4-10 membered heterocyclic group, the 4-10 membered heterocyclic group comprising 0 or 1 additional heteroatom selected from oxygen, nitrogen or sulfur, and being optionally substituted with 1, 2, 3 or 4 $R_x$;
$R_x$ is selected from $C_{1-6}$ alkyl, $C_{1-6}$ haloalkyl, phenyl or 5-10 membered heteroaryl, wherein the phenyl or 5-10

membered heteroaryl is optionally substituted with 1, 2 or 3 $R_y$;
$R_y$ is selected from halogen, $C_{1-6}$ alkyl, $C_{1-6}$ haloalkyl, $SF_5$, CN, $OR_a$, $C_{1-6}$ alkoxy, $-NR_{z1}R_{z2}$, $NR_{z1}R_{z2}$-$C_{1-6}$ alkyl-, $C_{3-6}$ cycloalkyl, 4-12 membered heterocyclyl or 5-10 membered heteroaryl, wherein the 4-12 membered heterocyclyl or 5-10 membered heteroaryl is optionally substituted by 1, 2 or 3 $R_z$;
$R_z$ is selected from $C_{1-6}$ alkyl, halogen, CN, $C_{1-6}$ haloalkyl or $C_{1-6}$ alkoxy;
$R_a$ is selected from H, $C_{1-6}$ alkyl or $C_{1-6}$ haloalkyl; and
$R_{z1}$ and $R_{z2}$ are each independently selected from H or $C_{1-6}$ alkyl.

**3.** A compound of claim 1 or 2, or a pharmaceutically acceptable salt, isotopic variant, tautomer or stereoisomer thereof, having the following structure:

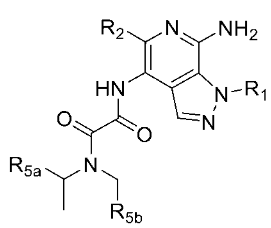
(III) or
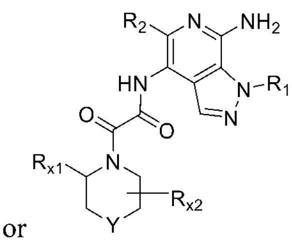
(IV)

wherein:

$R_1$ is selected from H, $C_{1-6}$ alkyl, deuterated $C_{1-6}$ alkyl, $C_{3-6}$ cycloalkyl or $C_{1-6}$ haloalkyl;
$R_2$ is selected from H, methyl, Cl or F;
$R_{5a}$ or $R_{5b}$ are each independently selected from 5-10 membered heteroaryl; the 5-10 membered heteroaryl is optionally substituted by $R_6$, $R_6$ being selected from H, CN, halogen, $C_{1-6}$ alkyl, $C_{1-6}$ haloalkyl, $SF_5$, $OR_a$ or 5-6 membered heteroaryl; the 5-6 membered heteroaryl is optionally substituted by 1, 2 or 3 $R_z$;
$R_{x1}$ is selected from phenyl or 5-10 membered heteroaryl, wherein the phenyl or 5-10 membered heteroaryl is optionally substituted with 1, 2 or 3 $R_y$;
$R_y$ is selected from halogen, $C_{1-6}$ alkyl, $C_{1-6}$ haloalkyl, $SF_5$, CN, $OR_a$, $C_{1-6}$ alkoxy, $-NR_{z1}R_{z2}$, $NR_{z1}R_{z2}$-$C_{1-6}$ alkyl-, $C_{3-6}$ cycloalkyl, 4-12 membered heterocyclyl or 5-10 membered heteroaryl, wherein the 4-12 membered heterocyclyl or 5-10 membered heteroaryl is optionally substituted by 1, 2 or 3 $R_z$;
$R_z$ is selected from $C_{1-6}$ alkyl, halogen, CN, $C_{1-6}$ haloalkyl or $C_{1-6}$ alkoxy;
$R_a$ is selected from H, $C_{1-6}$ alkyl or $C_{1-6}$ haloalkyl;
$R_{x2}$ is selected from $C_{1-6}$ alkyl or $C_{1-6}$ haloalkyl;
$R_{z1}$ and $R_{z2}$ are each independently selected from H or $C_{1-6}$ alkyl; and
Y is selected from O, S or $CH_2$.

**4.** A compound of claim 1 or 2, or a pharmaceutically acceptable salt, isotopic variant, tautomer or stereoisomer thereof, having the following structure:

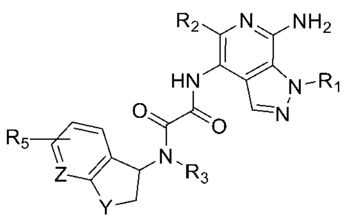
(VI) or
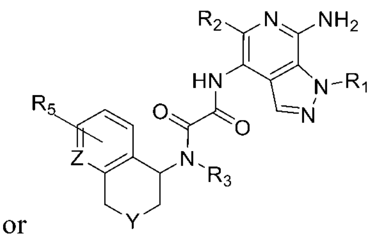
(VII)

wherein:

$R_1$ is selected from H, $C_{1-6}$ alkyl, deuterated $C_{1-6}$ alkyl, $C_{3-6}$ cycloalkyl or $C_{1-6}$ haloalkyl;
$R_2$ is selected from H, methyl, Cl or F;
$R_3$ is selected from H, $C_{1-6}$ alkyl, deuterated $C_{1-6}$ alkyl, $C_{3-6}$ cycloalkyl or 4-10 membered heterocyclyl; the $C_{1-6}$ alkyl or 4-10 membered heterocyclyl is optionally substituted by 1 or 2 $R_5$;
$R_5$ is selected from hydrogen, deuterium, halogen, $C_{1-6}$ alkyl, $C_{1-6}$ alkoxy, $C_{1-6}$ haloalkyl, $C_{1-6}$ haloalkoxy, CN, $SF_5$, $C_{3-6}$ cycloalkyl, $-S(O)_2CF_3$, $-OCF_3$, $-SCF_3$, $-SCH_3$ or 5-10 membered heteroaryl; the 5-10 membered

heteroaryl is optionally further substituted with $R_6$, $R_6$ being selected from H, CN, halogen, $C_{1-6}$ alkyl, $C_{1-6}$ haloalkyl, $SF_5$, $OR_a$ or 5-6 membered heteroaryl; the 5-6 membered heteroaryl is optionally substituted with 1, 2 or 3 $R_z$;

$R_z$ is selected from $C_{1-6}$ alkyl, halogen, CN, $C_{1-6}$ haloalkyl or $C_{1-6}$ alkoxy;

$R_a$ is selected from H, $C_{1-6}$ alkyl or $C_{1-6}$ haloalkyl;

Y is selected from NH, O, S or $CH_2$; and

Z is selected from N or CH.

**5.** The compound of any one of claims 1 to 3, or a pharmaceutically acceptable salt, isotopic variant, tautomer or stereoisomer thereof, having the following structure:

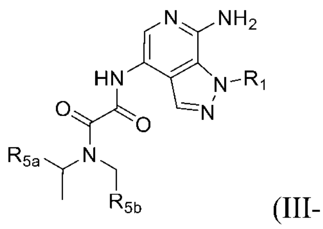

(III-1),

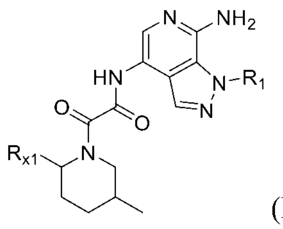

(IV-1) or

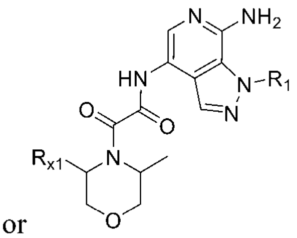

(V-1)

wherein,

$R_1$ is selected from $C_{1-6}$ alkyl, $C_{3-6}$ cycloalkyl or $C_{1-6}$ haloalkyl;

$R_{5a}$ or $R_{5b}$ are each independently selected from 5-10 membered heteroaryl; the 5-10 membered heteroaryl is optionally substituted by $R_6$, $R_6$ being selected from H, CN, halogen, $C_{1-6}$ alkyl, $C_{1-6}$ haloalkyl, $SF_5$, $OR_a$ or 5-6 membered heteroaryl; the 5-6 membered heteroaryl is optionally substituted by 1, 2 or 3 $R_z$;

$R_{x1}$ is selected from phenyl or 5-10 membered heteroaryl, wherein the phenyl or 5-10 membered heteroaryl is optionally substituted with 1, 2 or 3 $R_y$;

$R_y$ is selected from halogen, $C_{1-6}$ alkyl, $C_{1-6}$ haloalkyl, $SF_5$, CN, $OR_a$, $C_{1-6}$ alkoxy, $-NR_{z1}R_{z2}$, $NR_{z1}R_{z2}-C_{1-6}$ alkyl-, $C_{3-6}$ cycloalkyl, 4-12 membered heterocyclyl or 5-10 membered heteroaryl, wherein the 4-12 membered heterocyclyl or 5-10 membered heteroaryl is optionally substituted by 1, 2 or 3 $R_z$;

$R_z$ is selected from $C_{1-6}$ alkyl, halogen, CN, $C_{1-6}$ haloalkyl or $C_{1-6}$ alkoxy;

$R_a$ is selected from H, $C_{1-6}$ alkyl or $C_{1-6}$ haloalkyl; and

$R_{z1}$ and $R_{z2}$ are each independently selected from H or $C_{1-6}$ alkyl.

**6.** A compound of claim 5, or a pharmaceutically acceptable salt, isotopic variant, tautomer or stereoisomer thereof, which is a compound of formula (III-1):

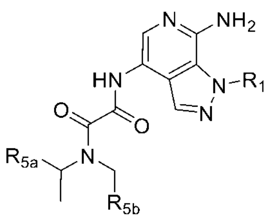

(III-1)

wherein,

$R_1$ is selected from methyl, $CD_3$, ethyl, or cyclopropyl;

$R_{5a}$ or $R_{5b}$ are each independently selected from 6-membered heteroaryl; the 6-membered heteroaryl is optionally further substituted by $R_6$, $R_6$ being selected from H, CN, halogen, $C_{1-6}$ alkyl, $C_{1-6}$ haloalkyl, $SF_5$, $OR_a$ or 5-6-membered heteroaryl; the 5-6-membered heteroaryl is optionally substituted by 1, 2 or 3 $R_z$;

$R_z$ is selected from $C_{1-6}$ alkyl, halogen, CN, $C_{1-6}$ haloalkyl or $C_{1-6}$ alkoxy; and

$R_a$ is selected from H, $C_{1-6}$ alkyl or $C_{1-6}$ haloalkyl.

**7.** A compound of claim 6, or a pharmaceutically acceptable salt, isotopic variant, tautomer or stereoisomer thereof, which is a compound of formula (III-2):

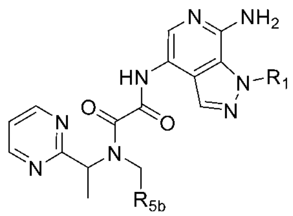

(III-2)

$R_1$ is selected from methyl, ethyl, or cyclopropyl;
$R_{5b}$ is

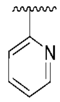

,

which is optionally further substituted by $R_6$, $R_6$ being selected from H, CN, halogen, $C_{1-6}$ alkyl, $C_{1-6}$ haloalkyl, $SF_5$, $OR_a$ or 5-6 membered heteroaryl; the 5-6 membered heteroaryl is optionally substituted by 1, 2 or 3 $R_z$;
$R_z$ is selected from $C_{1-6}$ alkyl, halogen, CN, $C_{1-6}$ haloalkyl or $C_{1-6}$ alkoxy; and
$R_a$ is selected from H, $C_{1-6}$ alkyl or $C_{1-6}$ haloalkyl.

**8.** A compound of claim 7, or a pharmaceutically acceptable salt, isotopic variant, tautomer or stereoisomer thereof, which is a compound of formula (III-3):

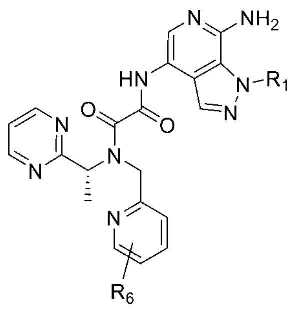

(III-3)

$R_1$ is selected from methyl, $CD_3$, ethyl or cyclopropyl;
$R_6$ is selected from H, CN, halogen, $C_{1-6}$ alkyl, $C_{1-6}$ haloalkyl, $SF_5$, $OR_a$ or 5-6 membered heteroaryl; the 5-6 membered heteroaryl is optionally substituted by 1, 2 or 3 $R_z$;
$R_z$ is selected from $C_{1-6}$ alkyl, halogen, CN, $C_{1-6}$ haloalkyl or $C_{1-6}$ alkoxy; and
$R_a$ is selected from H, $C_{1-6}$ alkyl or $C_{1-6}$ haloalkyl.

**9.** A compound of claim 5, or a pharmaceutically acceptable salt, isotopic variant, tautomer or stereoisomer thereof, which is a compound of formula (IV-1):

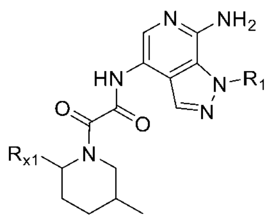

(IV-1),

wherein,

$R_1$ is selected from $C_{1-6}$ alkyl, deuterated $C_{1-6}$ alkyl, $C_{3-6}$ cycloalkyl or $C_{1-6}$ haloalkyl;
$R_{x1}$ is selected from phenyl or 5-10 membered heteroaryl, wherein the phenyl or 5-10 membered heteroaryl is optionally substituted with 1, 2 or 3 $R_y$;

$R_y$ is selected from halogen, $C_{1-6}$ alkyl, $C_{1-6}$ haloalkyl, $SF_5$, CN, $OR_a$, $C_{1-6}$ alkoxy, $C_{3-6}$ cycloalkyl,

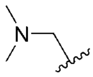, 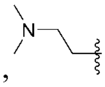,

4-12 membered heterocyclyl or 5-10 membered heteroaryl, wherein the 4-12 membered heterocyclyl or 5-10 membered heteroaryl is optionally substituted with 1, 2 or 3 $R_z$;
$R_z$ is selected from $C_{1-6}$ alkyl, halogen, CN, $C_{1-6}$ haloalkyl or $C_{1-6}$ alkoxy; and
$R_a$ is selected from H, $C_{1-6}$ alkyl or $C_{1-6}$ haloalkyl.

**10.** A compound of claim 9, or a pharmaceutically acceptable salt, isotopic variant, tautomer or stereoisomer thereof, which is a compound of formula (IV-2):

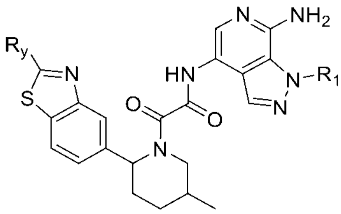

(IV-2),

wherein,

$R_1$ is selected from methyl, $CD_3$, ethyl or cyclopropyl;
$R_y$ is selected from $C_{1-6}$ alkyl, $C_{1-6}$ haloalkyl, CN, $OR_a$, $C_{1-6}$ alkoxy, $C_{3-6}$ cycloalkyl,

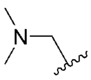, 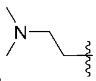,

4-12 membered heterocyclyl or 5-10 membered heteroaryl, wherein the 4-12 membered heterocyclyl or 5-10 membered heteroaryl is optionally substituted with 1, 2 or 3 $R_z$; and
$R_z$ is selected from $C_{1-6}$ alkyl, halogen, CN, $C_{1-6}$ haloalkyl or $C_{1-6}$ alkoxy.

**11.** A compound of claim 5, or a pharmaceutically acceptable salt, isotopic variant, tautomer or stereoisomer thereof, which is a compound of formula (V-1):

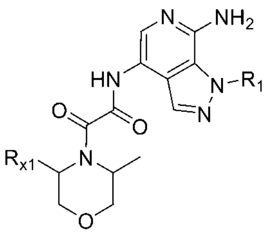

(V-1)

wherein,

$R_1$ is selected from $C_{1-6}$ alkyl, deuterated $C_{1-6}$ alkyl, $C_{3-6}$ cycloalkyl or $C_{1-6}$ haloalkyl;
$R_{x1}$ is a 5-10 membered heteroaryl group, which is optionally substituted by 1, 2 or 3 $R_y$;
$R_y$ is selected from halogen, $C_{1-6}$ alkyl, $C_{1-6}$ haloalkyl, $SF_5$, CN, $OR_a$, $C_{1-6}$ alkoxy, $C_{3-6}$ cycloalkyl, 4-12 membered heterocyclyl or 5-10 membered heteroaryl, wherein the 5-10 membered heteroaryl is optionally substituted with 1, 2 or 3 $R_z$;
$R_z$ is selected from $C_{1-6}$ alkyl, halogen, CN, $C_{1-6}$ haloalkyl or $C_{1-6}$ alkoxy; and
$R_a$ is selected from H, $C_{1-6}$ alkyl or $C_{1-6}$ haloalkyl.

**12.** A compound of claim 11, or a pharmaceutically acceptable salt, isotopic variant, tautomer or stereoisomer thereof,

which is a compound of formula (V-2):

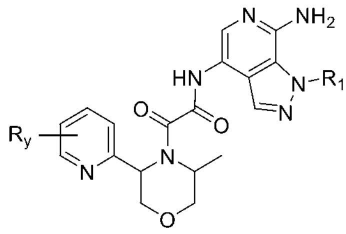

(V-2)

wherein,

$R_1$ is selected from methyl, $CD_3$, ethyl or cyclopropyl;
$R_y$ is selected from halogen, $C_{1-6}$ alkyl, $C_{1-6}$ haloalkyl, $SF_5$, CN, $OR_a$, $C_{1-6}$ alkoxy, $C_{3-6}$ cycloalkyl, 4-12 membered heterocyclyl or 5-10 membered heteroaryl, wherein the 5-10 membered heteroaryl is optionally substituted with 1, 2 or 3 $R_z$;
$R_z$ is selected from $C_{1-6}$ alkyl, halogen, CN, $C_{1-6}$ haloalkyl or $C_{1-6}$ alkoxy; and
$R_a$ is selected from H, $C_{1-6}$ alkyl or $C_{1-6}$ haloalkyl.

**13.** A compound of claim 12, or a pharmaceutically acceptable salt, isotopic variant, tautomer or stereoisomer thereof, which is a compound of formula (V-3):

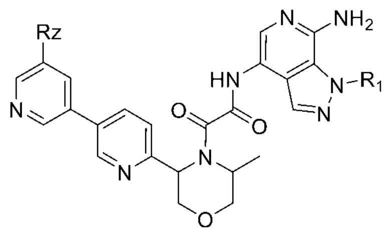

(V-3)

wherein,

$R_1$ is selected from methyl, $CD_3$, ethyl or cyclopropyl;
$R_z$ is selected from $C_{1-6}$ alkyl, halogen, CN, $C_{1-6}$ haloalkyl or $C_{1-6}$ alkoxy; and
$R_a$ is selected from H, $C_{1-6}$ alkyl or $C_{1-6}$ haloalkyl.

**14.** A compound of claim **4,** or a pharmaceutically acceptable salt, isotopic variant, tautomer or stereoisomer thereof, having the following structure:

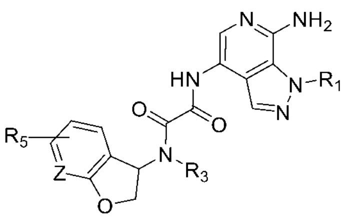

(VI-1) or

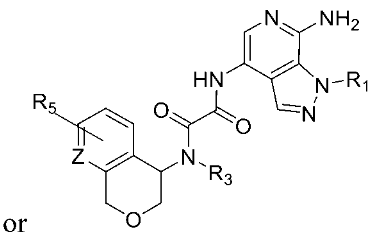

(VII-1)

wherein:

$R_1$ is selected from H, $C_{1-6}$ alkyl, deuterated $C_{1-6}$ alkyl, $C_{3-6}$ cycloalkyl or $C_{1-6}$ haloalkyl;
$R_3$ is selected from H, $C_{1-6}$ alkyl, deuterated $C_{1-6}$ alkyl, $C_{3-6}$ cycloalkyl or 4-10 membered heterocyclyl; the $C_{1-6}$ alkyl or 4-10 membered heterocyclyl is optionally substituted by 1 or 2 $R_5$;
$R_5$ is selected from hydrogen, deuterium, halogen, $C_{1-6}$ alkyl, $C_{1-6}$ alkoxy, $C_{1-6}$ haloalkyl, $C_{1-6}$ haloalkoxy, CN, $SF_5$, $C_{3-6}$ cycloalkyl, $-S(O)_2CF_3$, $-OCF_3$, $-SCF_3$, $-SCH_3$ or 5-10 membered heteroaryl; the 5-10 membered heteroaryl is optionally further substituted with $R_6$, $R_6$ being selected from H, CN, halogen, $C_{1-6}$ alkyl, $C_{1-6}$

haloalkyl, $SF_5$, $OR_a$ or 5-6 membered heteroaryl;
$R_a$ is selected from H, $C_{1}$-$_{6}$ alkyl or $C_{1}$-$_{6}$ haloalkyl; and
Z is selected from N or CH.

**15.** A compound of claim 14, or a pharmaceutically acceptable salt, isotopic variant, tautomer or stereoisomer thereof, having the following structure:

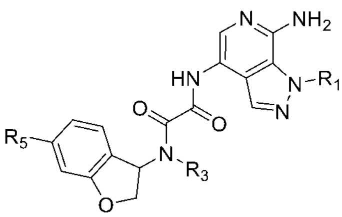 (VI-2) or 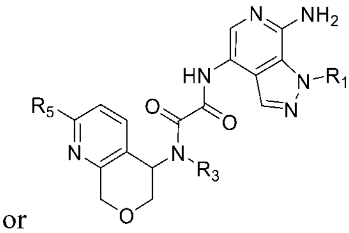 (VII-2)

wherein:

$R_1$ is selected from H, methyl, deuterated methyl, ethyl or cyclopropyl;
$R_3$ is selected from H, $C_{1}$-$_{6}$ alkyl, deuterated $C_{1}$-$_{6}$ alkyl, $C_{3}$-$_{6}$ cycloalkyl or 4-10 membered heterocyclyl; and
$R_5$ is selected from hydrogen, halogen, $C_{1}$-$_{6}$ alkyl, $C_{1}$-$_{6}$ alkoxy, $C_{1}$-$_{6}$ haloalkyl, $C_{1}$-$_{6}$ haloalkoxy, CN, $SF_5$, $C_{3}$-$_{6}$ cycloalkyl, $-S(O)_2CF_3$, $-OCF_3$, $-SCF_3$, $-SCH_3$ or 5-10 membered heteroaryl.

**16.** A compound of claim 15, or a pharmaceutically acceptable salt, isotopic variant, tautomer or stereoisomer thereof, wherein:

$R_1$ is selected from H, methyl, $CD_3$, ethyl or cyclopropyl;
$R_3$ is selected from H, methyl, $CD_3$, ethyl or cyclopropyl; and
$R_5$ is selected from hydrogen, F, Cl, Br, methyl, ethyl, methoxy, trifluoromethyl, difluoromethyl, CN, $SF_5$, cyclopropyl, $-S(O)_2CF_3$, $-OCF_3$, $-OCF_2Cl$, $-SCF_3$, $-SCH_3$ or pyridyl.

**17.** A compound, or a pharmaceutically acceptable salt, isotopic variant, tautomer or stereoisomer thereof, wherein the compound is selected from:

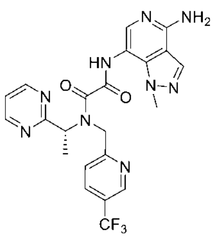 , 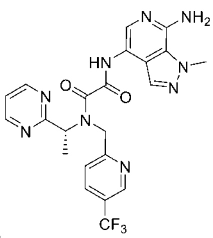 , 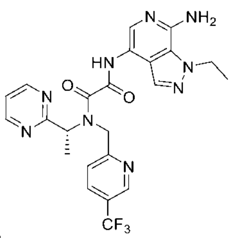 , 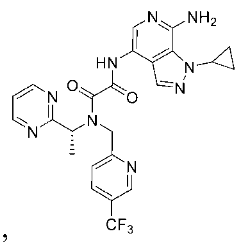 , 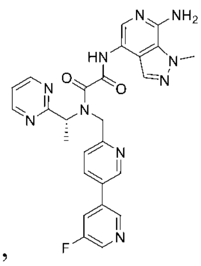 ,

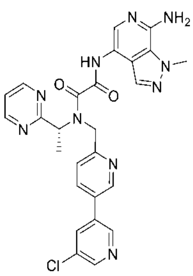 , 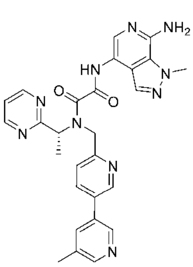 , 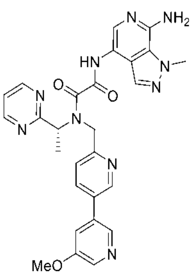 , 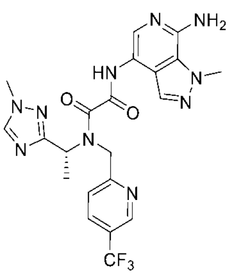 , 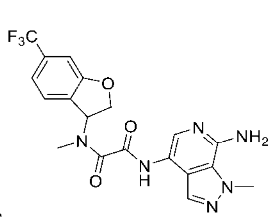 ,

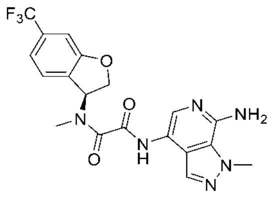,
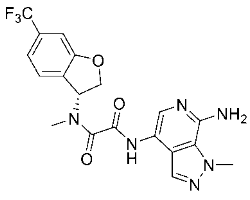,
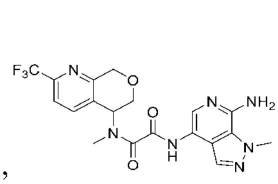,
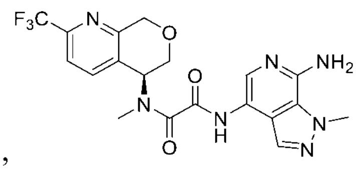,
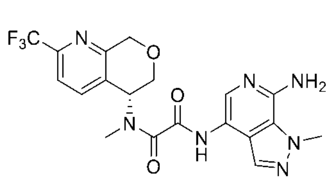,
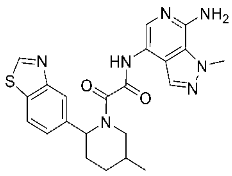,
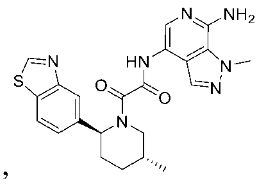,
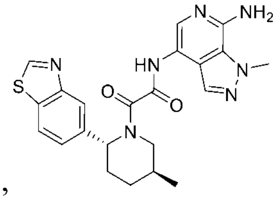,
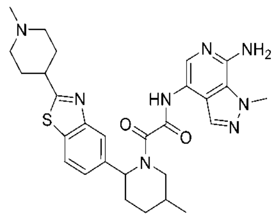,
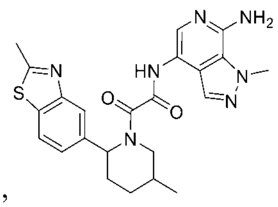,
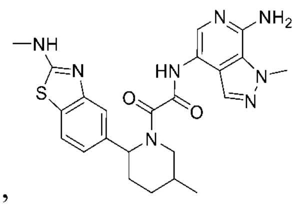,
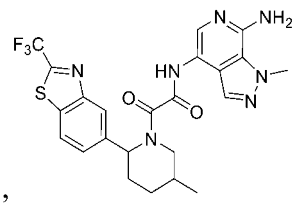,
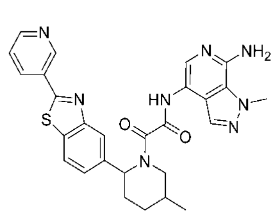,
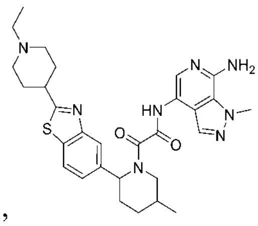,
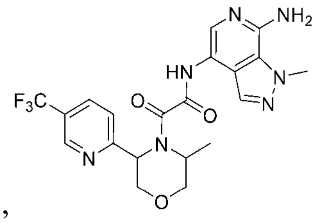,
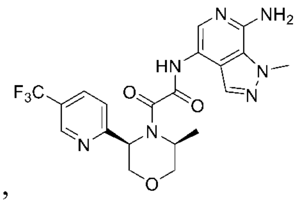,
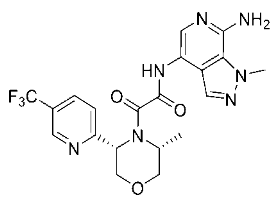,
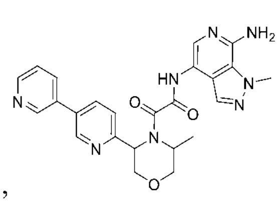,
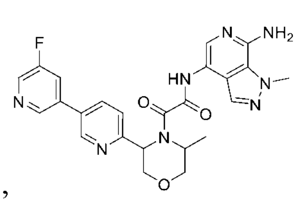,
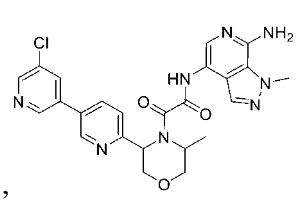,
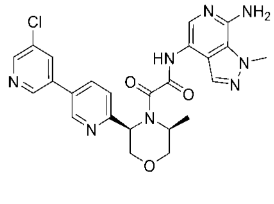,
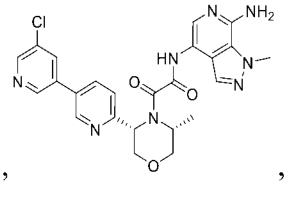,
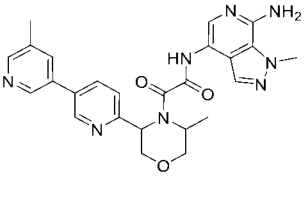,
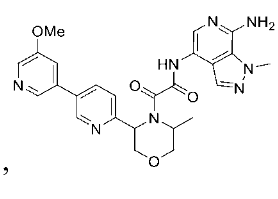,
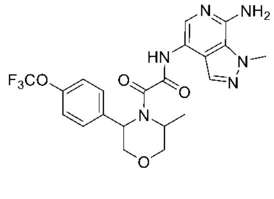,
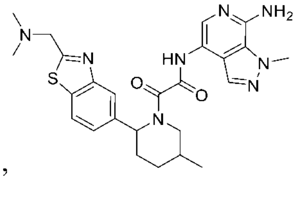,
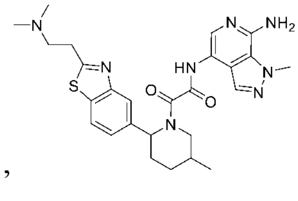,
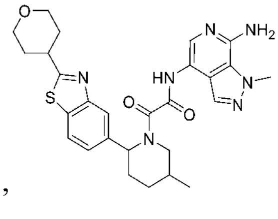,

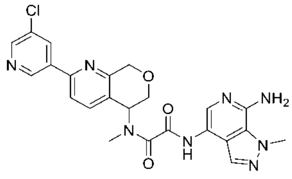,
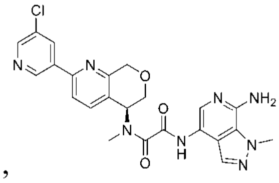,
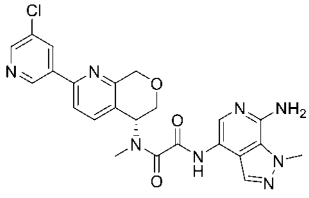,
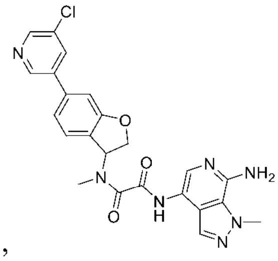,

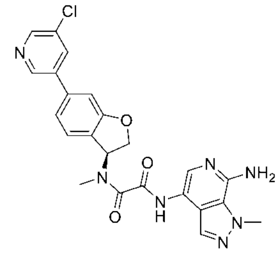,
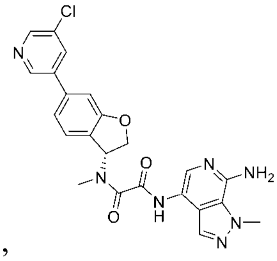,
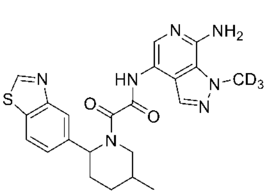,
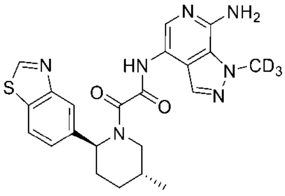,

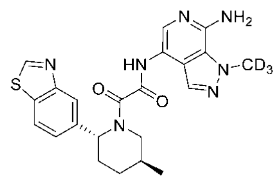,
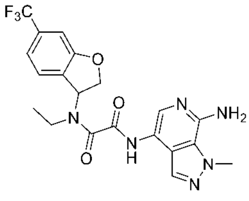,
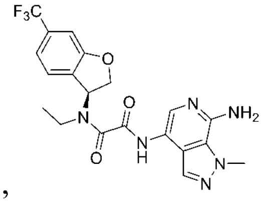,
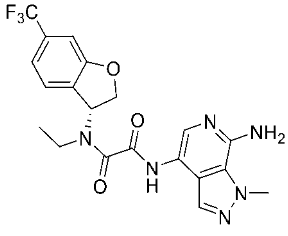,

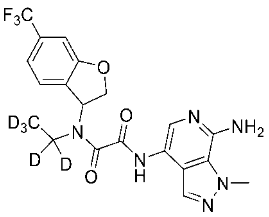,
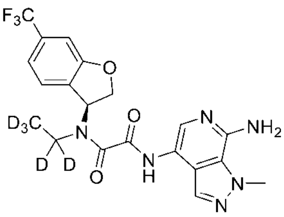,
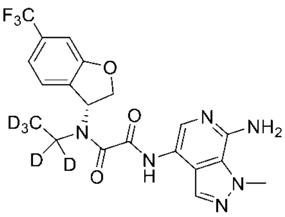,
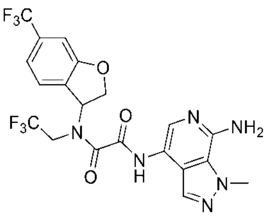,

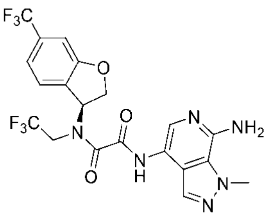,
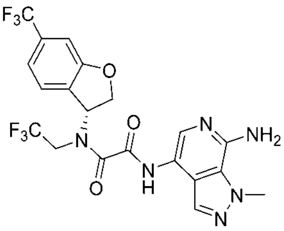,
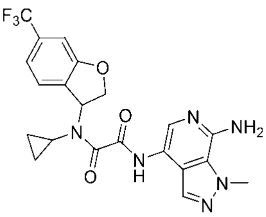,
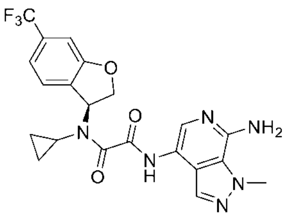,

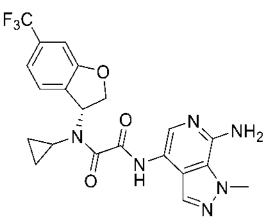,
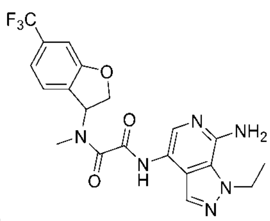,
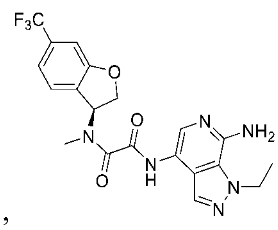,
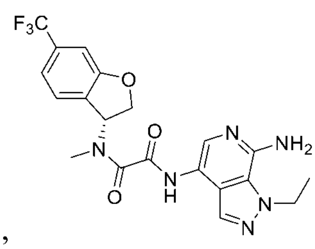,

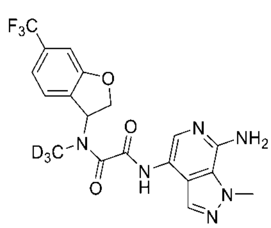 , 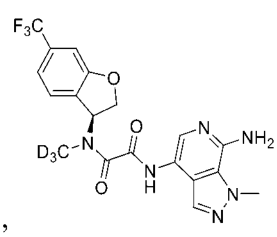 , 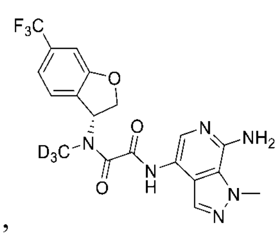 , 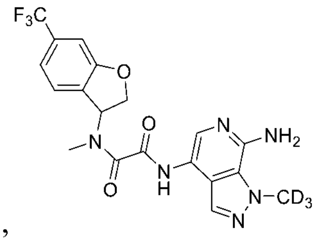 ,

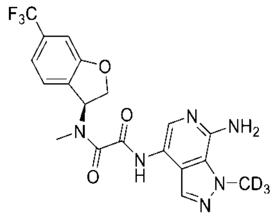 , 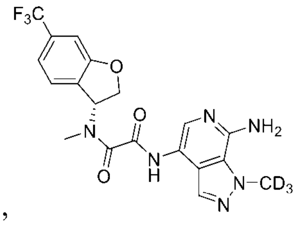 , 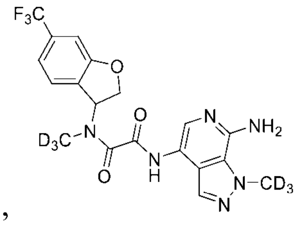 , 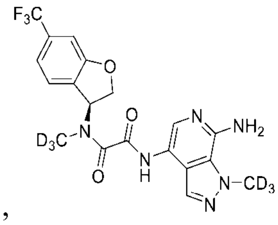 ,

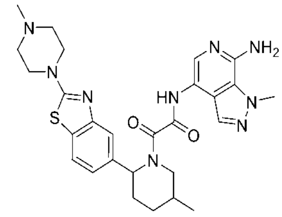 , 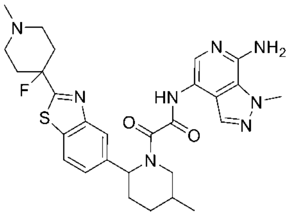 , 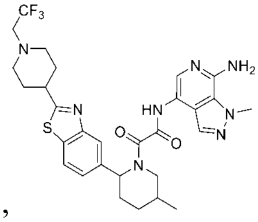 , 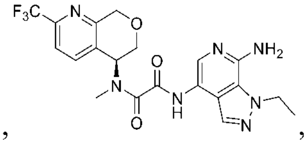 ,

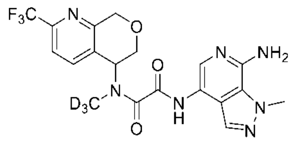 , 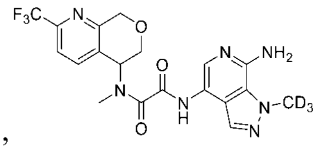 , 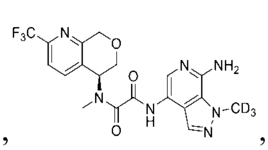 , 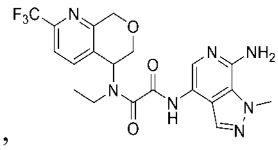 ,

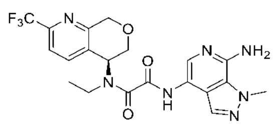 , 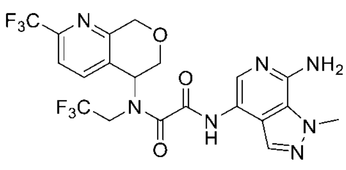 , 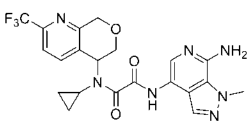 , 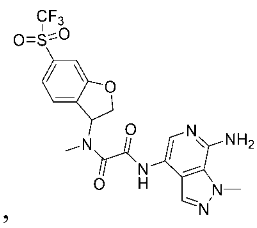 ,

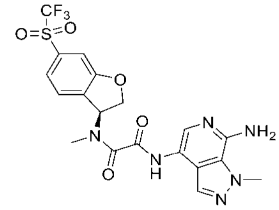 , 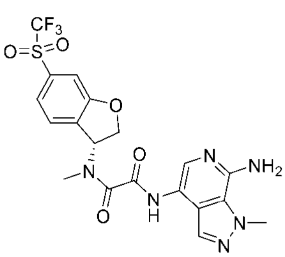 , 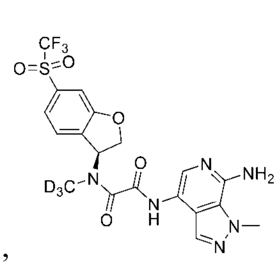 , 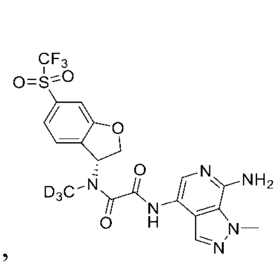 ,

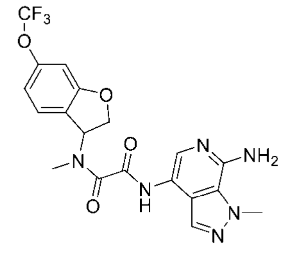,
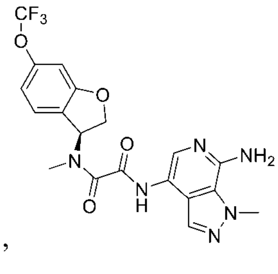,
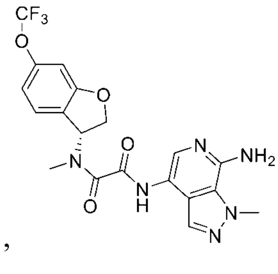,
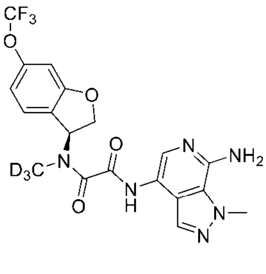,
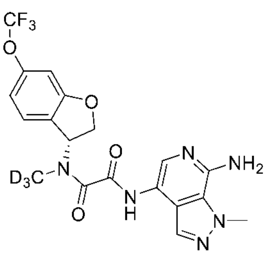,
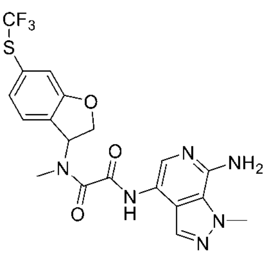,
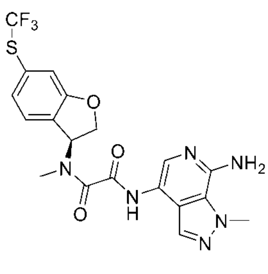,
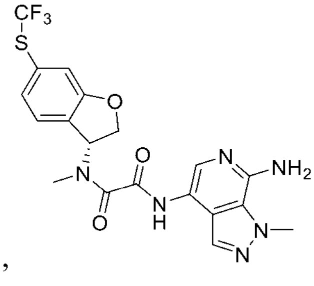,
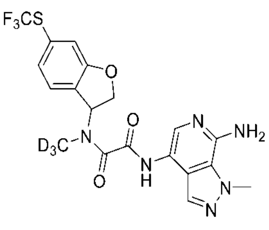,
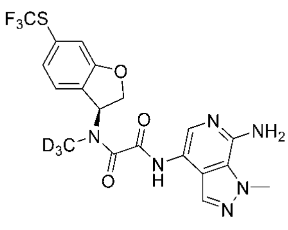,
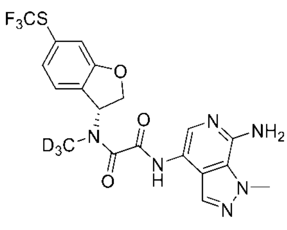,
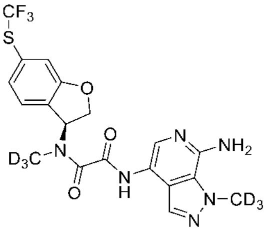,
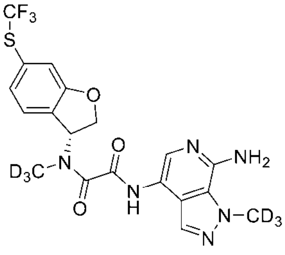,
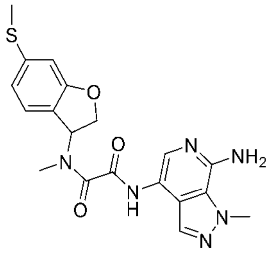,
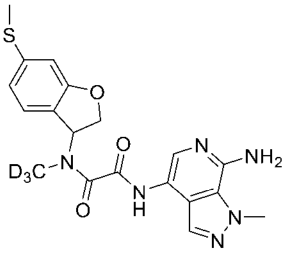,
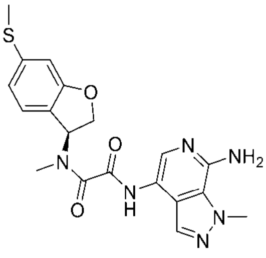,
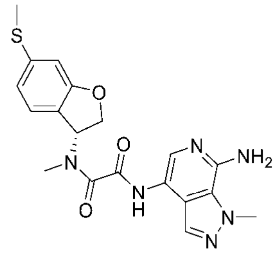,
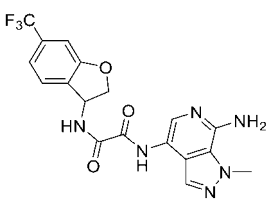,
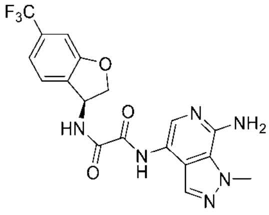,
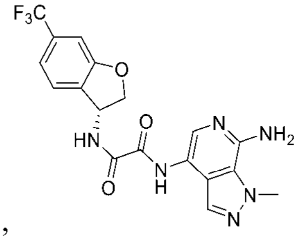,
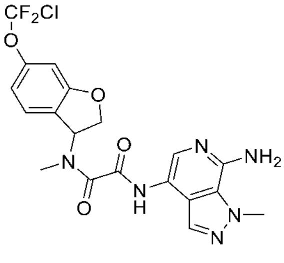
or
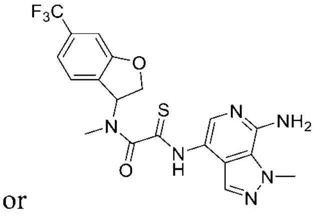.

**18.** A pharmaceutical composition comprising a compound of any one of claims 1 to 17, or a pharmaceutically acceptable salt, isotopic variant, tautomer or stereoisomer thereof, and a pharmaceutically acceptable excipient; preferably, it further comprises other therapeutic agents.

**19.** Use of a compound of any one of claims 1 to 17 or a pharmaceutically acceptable salt, isotopic variant, tautomer or stereoisomer thereof in the preparation of a medicament for treating and/or preventing a PRMT5 methyltransferase-mediated disease.

**20.** A method for treating and/or preventing a PRMT5 methyltransferase-mediated disease in a subject, the method comprising administering to the subject a compound of any one of claims 1 to 17 or a pharmaceutically acceptable salt, isotopic variant, tautomer or stereoisomer thereof, or a pharmaceutical composition of claim 18.

**21.** A compound of any one of claims 1 to 17 or a pharmaceutically acceptable salt, isotopic variant, tautomer or stereoisomer thereof or a pharmaceutical composition of claim 18 for use in treating and/or preventing a PRMT5 methyltransferase-mediated disease.

**22.** The use of claim 19 or the method of claim 20 or the use of a compound or composition of claim 17, wherein the PRMT5 methyltransferase-mediated disease is cancer, and the cancer is selected from the group consisting of: acoustic neuroma, adenocarcinoma, adrenal cancer, anal cancer, angiosarcoma (e.g., lymphangiosarcoma, lymphangioendothelial sarcoma, hemangioma), appendix cancer, benign monoclonal gammopathy, bile duct cancer, bladder cancer, brain cancer (e.g., meningioma, glioma, such as astrocytoma, oligodendroglioma, medulloblastoma), bronchogenic carcinoma, carcinoid tumor, cervical cancer (e.g. cervical adenocarcinoma), choriocarcinoma, chordoma, craniopharyngioma, colorectal cancer (e.g. colon cancer, rectal cancer, colorectal adenocarcinoma), epithelial cancer, ependymoma, endothelial sarcoma (e.g. Kaposi's sarcoma, multiple idiopathic hemorrhagic sarcoma), endometrial cancer (e.g. uterine cancer, uterine sarcoma), esophageal cancer (e.g. esophageal adenocarcinoma, Barrett's adenocarcinoma), Ewing's sarcoma, eye cancer (e.g. intraocular melanoma, retinoblastoma), hypereosinophilia, gallbladder cancer, gastric cancer (e.g., gastric adenocarcinoma), gastrointestinal stromal tumor (GIST), head and neck cancer (e.g., head and neck squamous cell carcinoma), oral cancer (e.g., oral squamous cell carcinoma), laryngeal cancer (e.g., laryngeal cancer, pharyngeal cancer, nasopharyngeal cancer, oropharyngeal cancer)), hematopoietic system cancer (e.g., leukemias, such as acute lymphoblastic leukemia (ALL) (e.g., B-cell ALL, T-cell ALL), acute myeloid leukemia (AML) (e.g., B-cell AML, T-cell AML), chronic myeloid leukemia (CML) (e.g., B-cell CML, T-cell CML), chronic lymphocytic leukemia (CLL) (e.g., B-cell CLL, T-cell CLL), follicular lymphoma, chronic lymphocytic leukemia/small lymphocytic lymphoma (CLL/SLL), marginal zone B-cell lymphoma (e.g., mucosa-associated lymphoid tissue (MALT) lymphoma, lymph node marginal zone B-cell lymphoma, splenic marginal zone mediastinal B-cell lymphoma), primary mediastinal B-cell lymphoma, Burkitt's lymphoma, lymphoplasmacytic lymphoma, hairy cell leukemia (HCL), immunoblastic large cell lymphoma, precursor B-lymphoblastic lymphoma, and primary central nervous system (CNS) lymphoma; and T-cell non-Hodgkin lymphoma, such as precursor T-lymphoblastic lymphoma/leukemia, peripheral T-cell lymphoma (e.g., cutaneous T-cell lymphoma (e.g., mycosis fungoides, Sezary syndrome), angioimmunoblastic T-cell lymphoma, extranodal natural killer T-cell lymphoma, enteropathy-associated T-cell lymphoma, subcutaneous panniculitis-like T-cell lymphoma, anaplastic large cell lymphoma); a mixture of one or more of the above leukemias/lymphomas; multiple myeloma (MM), heavy chain disease (e.g., $\alpha$-chain disease, $\gamma$-chain disease, $\mu$-chain disease), hemangioblastoma, inflammatory myofibroblastic tumor, immunocytic amyloidosis, renal cancer (e.g., Wilms tumor, renal cell carcinoma), liver cancer (e.g., hepatocellular carcinoma, malignant hepatocellular carcinoma), lung cancer (e.g., bronchogenic carcinoma, small cell lung cancer (SCLC), non-small cell lung cancer (NSCLC), lung adenocarcinoma, leiomyosarcoma (LMS), mastocytosis (e.g., systemic mastocytosis), myelodysplastic syndrome (MDS), mesothelioma, myeloproliferative disorders (MPDs) (e.g., polycythemia vera (PV), essential thrombocythemia (ET), agnogenic myeloid metaplasia (AMM), chronic idiopathic myelofibrosis, chronic myelocytic leukemia (CML), chronic neutrophilic leukemia (CNL), hypereosinophilic syndrome (HES), neuroblastoma, neurofibromas (e.g., neurofibromatosis type 1 or type 2, schwannomatosis), neuroendocrine cancer (e.g., gastroenteropancreatic neuroendocrine tumor (GEP-NET), carcinoid tumor), osteosarcoma, ovarian cancer (e.g., cystadenocarcinoma, ovarian embryonal carcinoma, ovarian adenocarcinoma), papillary adenocarcinoma, or penile cancer.

## INTERNATIONAL SEARCH REPORT

International application No.
**PCT/CN2024/071738**

### A. CLASSIFICATION OF SUBJECT MATTER

C07D471/04(2006.01)i; C07D491/044(2006.01)i; C07D401/12(2006.01)i; C07D239/95(2006.01)i; C07D213/75(2006.01)i; A61P35/00(2006.01)i; A61K31/437(2006.01)i; A61K31/4427(2006.01)i

According to International Patent Classification (IPC) or to both national classification and IPC

### B. FIELDS SEARCHED

Minimum documentation searched (classification system followed by classification symbols)

C07D,A61P,A61K

Documentation searched other than minimum documentation to the extent that such documents are included in the fields searched

Electronic data base consulted during the international search (name of data base and, where practicable, search terms used)

CNKI, CNABS, CNTXT, VCN, VEN, ENTXT, ENTXTC, DWPI, STN(REG, CAPLUS, MARPAT): 抑制剂, PRMT, MTA, 癌, 瘤, 乙酰胺, inhibitor, cancer, tumor, acetamide, 结构式, structural formula

### C. DOCUMENTS CONSIDERED TO BE RELEVANT

| Category* | Citation of document, with indication, where appropriate, of the relevant passages | Relevant to claim No. |
|---|---|---|
| PX | WO 2023146987 A1 (TANGO THERAPEUTICS, INC.) 03 August 2023 (2023-08-03) claims 1-9 and 77-88, and see description, pages 7 and 15 | 1-19, 21-22 (in part) |
| PX | WO 2023146989 A1 (TANGO THERAPEUTICS, INC.) 03 August 2023 (2023-08-03) claims 1-10 | 1-19, 21-22 (in part) |
| PX | WO 2023146990 A1 (TANGO THERAPEUTICS, INC.) 03 August 2023 (2023-08-03) claims 1-83 | 1-19, 21-22 (in part) |
| PX | WO 2023146991 A1 (TANGO THERAPEUTICS, INC.) 03 August 2023 (2023-08-03) claims 1-100 | 1-19, 21-22 (in part) |
| E | WO 2024022433 A1 (BEIJING EARTHWISE TECH CO., LTD.) 01 February 2024 (2024-02-01) description, pages 40-110, in particular pages 40-105, a plurality of compounds | 1-19, 21-22 (in part) |
| E | WO 2024037608 A1 (BEIJING EARTHWISE TECH CO., LTD.) 22 February 2024 (2024-02-22) description, pages 37-82, in particular compound 24, etc. | 1-19, 21-22 (in part) |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 April 2024** | **23 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/071738**

**C. DOCUMENTS CONSIDERED TO BE RELEVANT**

| Category* | Citation of document, with indication, where appropriate, of the relevant passages | Relevant to claim No. |
|---|---|---|
| E | WO 2024037607 A1 (BEIJING EARTHWISE TECH CO., LTD.) 22 February 2024 (2024-02-22)<br>description, pages 49-62, in particular pages 49-58, a plurality of compounds | 1-19, 21-22 (in part) |
| A | WO 2021004547 A1 (GUANGDONG NEWOPP BIOPHARMACEUTICALS CO., LTD.) 14 January 2021 (2021-01-14)<br>entire document | 1-19, 21-22 (in part) |
| A | WO 2022026892 A1 (TANGO THERAPEUTICS, INC.) 03 February 2022 (2022-02-03)<br>entire document, in particular claims 1-113, and table 1 | 1-19, 21-22 (in part) |
| A | WO 2022256806 A1 (IDEAYA BIOSCIENCES INC.) 08 December 2022 (2022-12-08)<br>entire document | 1-19, 21-22 (in part) |
| A | US 2012122834 A1 (SODROSKI JOSEPH; DANA FARBER CANCER INST INC. et al.) 17 May 2012 (2012-05-17)<br>entire document | 1-19, 21-22 (in part) |

Form PCT/ISA/210 (second sheet) (July 2022)

**INTERNATIONAL SEARCH REPORT**

International application No.
**PCT/CN2024/071738**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **20, 22 (in part)**
   because they relate to subject matter not required to be searched by this Authority, namely:

   Claims 20 and 22 (in part) relate to a treatment method implemented on the human/animal body (PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/071738**

| Patent document cited in search report | Publication date (day/month/year) | Patent family member(s) | Publication date (day/month/year) |
|---|---|---|---|
| WO 2023146987 A1 | 03 August 2023 | TW 202339736 A | 16 October 2023 |
| WO 2023146989 A1 | 03 August 2023 | None | |
| WO 2023146990 A1 | 03 August 2023 | TW 202339737 A | 16 October 2023 |
| WO 2023146991 A1 | 03 August 2023 | None | |
| WO 2024022433 A1 | 01 February 2024 | None | |
| WO 2024037608 A1 | 22 February 2024 | None | |
| WO 2024037607 A1 | 22 February 2024 | None | |
| WO 2021004547 A1 | 14 January 2021 | None | |
| WO 2022026892 A1 | 03 February 2022 | None | |
| WO 2022256806 A1 | 08 December 2022 | CA 3220160 A1 | 08 December 2022 |
| | | KR 20240019172 A | 14 February 2024 |
| | | AU 2022287057 A1 | 14 December 2023 |
| | | IL 308853 A | 01 January 2024 |
| US 2012122834 A1 | 17 May 2012 | US 9776963 B2 | 03 October 2017 |
| | | WO 2010053583 A2 | 14 May 2010 |
| | | WO 2010053583 A3 | 16 September 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5376645 A

### Non-patent literature cited in the description


- *Nat Rev Mol Cell Biol.*, October 2019, vol. 20 (10), 642-657
- *Nat Rev Drug Discov.*, July 2021, vol. 20 (7), 509-530
- *Cell Stress*, August 2020, vol. 4 (8), 199-215
- *Cancer Gene Ther.*, March 2022, vol. 29 (3-4), 264-276
- *Bioorg Med Chem Lett.*, 01 June 2019, vol. 29 (11), 1264-1269
- *Expert Opin Ther Pat.*, February 2019, vol. 29 (2), 97-114
- *Annals of Oncology*, 2020, vol. 31 (4), S462-S504
- *Annals of Oncology*, 2019, vol. 30 (5), v159-v193
- *Science*, 11 March 2016, vol. 351 (6278), 1214-8
- *Nat Rev Drug Discov.*, January 2020, vol. 19 (1), 23-38
- *Cell Rep.*, 19 April 2016, vol. 15 (3), 574-587
- *J Med Chem.*, 10 February 2022, vol. 65 (3), 1749-1766
- Remington's Pharmaceutical Sciences. Mack Publishing Company, 1985